# EUROPEAN PATENT APPLICATION

(11) **EP 4 748 841 A1**
(43) Date of publication of application: **27.05.2026**
(21) Application number: 24841953.3
(22) Date of filing: 18.03.2024
(51) Int. Cl.: C07D 487/14, C07D 471/14, A61K 31/519, A61P 25/28

(54) **BICYCLO[5,6] IMIDAZOLE PYRIMIDONE DERIVATIVE AND PREPARATION METHOD THEREFOR AND USE THEREOF**

(30) Priority: 17.07.2023 CN 202310873197; 15.08.2023 CN 202311023142; 20.11.2023 CN 202311545385
(71) Applicant: Shujing Biopharma Co., Ltd, Shanghai 201208 (CN); Jiangsu NHWA Pharmaceutical Co., Ltd, Xuzhou, Jiangsu 221000 (CN)
(72) Inventor: WAN, Zehong, Shanghai 201208 (CN)
(74) Representative: Lavoix
(86) International application number: PCT/CN2024/082107
(87) International publication number: WO 2025/015951

(57) **Abstract**

The present invention relates to the field of pharmaceutical chemistry, and in particular to a bicyclo[5,6] imidazole pyrimidone derivative and a preparation method therefor and a use thereof. In particular, the present invention relates to a compound represented by general formula (IA), a stereoisomer thereof, a pharmaceutically acceptable salt thereof or a prodrug thereof, a preparation method therefor, an intermediate thereof, a pharmaceutical composition containing the compound, and a use of the compound or the pharmaceutical composition thereof as an Lp-PLA2 inhibitor in preparation of a drug for preventing and/or treating related diseases mediated by Lp-PLA2.

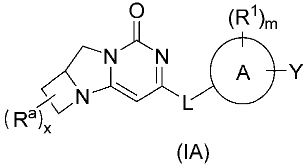

## Description

The present application claims priority to Chinese Patent Application No. 2023108731979 filed on July 17, 2023, Chinese Patent Application No. 2023110231425 filed on August 15, 2023, and Chinese Patent Application No. 2023115453855 filed on November 20, 2023, which are incorporated herein by reference in their entirety.

### TECHNICAL FIELD

The present disclosure pertains to the field of pharmaceutical chemistry, and particularly relates to a bicyclo[5,6] imidazole pyrimidone derivative, a preparation method therefor, and use thereof. More specifically, the present disclosure relates to a bicyclo[5,6] imidazole pyrimidone derivative, a preparation method therefor, an intermediate thereof, a pharmaceutical composition comprising the bicyclo[5,6] imidazole pyrimidone derivative, and use of the bicyclo[5,6] imidazole pyrimidone derivative or the pharmaceutical composition thereof as an Lp-PLA2 inhibitor in the preparation of a medicament for preventing and/or treating related diseases mediated by Lp-PLA2.

### BACKGROUND

Lipoprotein-associated phospholipase A2 (Lp-PLA2), also known as platelet-activating factor acetylhydrolase (PAF-AH), is involved in the hydrolysis of lipoprotein lipids or phospholipids. Lp-PLA2 travels with low-density lipoprotein (LDL) and hydrolyzes the sn-2 ester of oxidized phosphatidylcholine, producing lipid mediators: lysophosphatidylcholine (LysoPC) and oxidized non-esterified fatty acids (NEFAs). Studies have shown that LysoPC and NEFAs can trigger inflammatory responses (Zalewski A, et al., Arterioscler. Thromb. Vase. Biol., 2005, 25, 923-31). The products generated by the action of Lp-PLA2 enzyme (LysoPC and NEFAs) can promote atherosclerosis (Macphee CH, et al., Biochem J, 1999, 338, 479-87; Macphee CH et al., Current Opinion in Pharmacology, 2006, 6, 154-161; Zalewski A, et al., Arterioscler Thromb Vase Biol 2005, 25, 923-31). Studies indicate that LysoPC promotes the progression of atherosclerotic plaques, ultimately leading to the formation of necrotic cores (Wilensky et al., Current Opinion in Lipidology, 2009, 20, 415-420). Furthermore, the products generated by the action of Lp-PLA2 enzyme are associated with plaque vulnerability and pathology (Kolodgie FD, et al., Arteriosclerosis Thrombosis and Vascular Biology, 2006, 26, 2523-9; Ferguson, J. F., et al., Journal of the American College of Cardiology, 2012, 59, 764-772). The effects of Lp-PLA2 inhibitors on atherosclerotic plaque composition have also been demonstrated in diabetic and hypercholesterolemic porcine models of accelerated coronary atherosclerosis (Wilensky et al., Nature Medicine, 2008, 10, 1015-1016). Clinical studies have found that Lp-PLA2 inhibitors can stabilize atherosclerotic plaques in patients with such plaques, preventing further plaque progression and rupture (The STABILITY Investigators et al., N Engl J Med, 2014, 370, 1702-1711; O'Donoghue ML, et al., JAMA, 2014, 312, 1006-1015; Serruys PW, et al., Circulation, 2008,118,1172-1182; Mohler ER III, et al., Journal of the American College of Cardiology, 2008, 51, 1632-1641).

Other studies suggest that high Lp-PLA2 activity is associated with an increased risk of dementia, including Alzheimer's disease (AD) (Van Oijen, et al., Annals of Neurology, 2006, 59,139). Elevated levels of oxidized LDL have also been observed in AD patients (Kassner et al., Current Alzheimer Research, 2008, 5, 358-366; Sinem et al., Current Alzheimer Research, 2010, 7, 463-469). Additionally, studies indicate the presence of neuroinflammation and upregulated cytotoxic inflammatory cytokines in AD patients (Colangelo et al., Journal of Neuroscience Research, 2002, 70, 462-473). Studies also indicate that LysoPC is a pro-inflammatory factor that can induce the release of various toxic inflammatory cytokines (Shi et al., Atherosclerosis, 2007, 191, 54-62). Thus, these findings suggest that Lp-PLA2 inhibitors can reduce the production of LysoPC by inhibiting Lp-PLA2 activity, thereby offering therapeutic potential for AD.

Beyond its inflammatory effects, LysoPC is also involved in leukocyte activation, induction of apoptosis, and modulation of endothelial dysfunction (Wilensky et al., Current Opinion in Lipidology, 2009, 20, 415-420). Thus, Lp-PLA2 inhibitors can be used to treat tissue damage associated with diabetes by reducing the production of LysoPC. Given the inflammatory role of Lp-PLA2 and the link between local inflammatory processes and diabetic retinopathy, it can be hypothesized that Lp-PLA2 inhibitors can be used to treat diabetic eye diseases. Clinical studies suggest that Lp-PLA2 inhibitors can be used to treat diabetic macular edema (DME) (Staurenghi G et al., Ophthalmology, 2015, 122, 990).

Numerous Lp-PLA2 inhibitors and/or use thereof have been described in prior literature (including WO96/13484, WO96/19451, WO97/02242, WO97/12963, WO97/21675, WO97/21676, WO97/41098, WO97/41099, WO99/2442, WO00/10980, WO00/66566, WO00/66567, WO00/68208, WO01/60805, WO02/30904, WO02/30911, WO03/015786, WO03/016287, WO03/041712, WO03/042179, WO03/042206, WO03/042218, WO03/086400, WO03/87088, WO08/04886, US2008/0103156, US2008/0090851, US2008/0090852, WO08/048866, WO05/003118, WO06/063811, WO06/063813, WO08/141176, CN103827118, CN103827116, WO11/146494, WO12/037782, WO12/075917, WO12/076435, CN104478812, WO13/013503, WO13/014185, WO14/114248, WO14/114249, CN104968665, WO14/114694, WO16/011930, WO16/011931, WO16/101927, JP200188847, US2008/0279846, US2010/0239565, US2008/0280829, WO16/012916, CN112778331, WO21/063145, WO21/228159, WO21/089032, CN113666930, WO22/001881, WO22/007810, WO22/233302, CN115925721, WO23/109471, CN116120329, etc.). However, there is still a strong need in the art for new and differentiated Lp-PLA2 inhibitors.

### SUMMARY

The technical problem to be solved by the present disclosure is to provide an Lp-PLA2 inhibitor with a brand-new structure, and specifically to provide a bicyclo[5,6] imidazole pyrimidone derivative, a preparation method therefor, an intermediate thereof, a pharmaceutical composition thereof, and use thereof. The compound has good inhibitory activity against Lp-PLA2 and/or better metabolic stability, such as a lower clearance rate and/or a longer half-life.

The present disclosure aims to provide a compound represented by general formula (IA), a stereoisomer thereof, a pharmaceutically acceptable salt thereof, or a prodrug thereof: 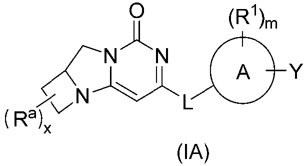 wherein:
L is -O(CH₂)ₙ₁-, -S(CH₂)ₙ₁-, -(CH₂)ₙ₁-, or -NRₐₐ(CH₂)ₙ₁-, wherein the (CH₂)ₙ₁- end is linked to ring A;
Rₐₐ is hydrogen, deuterium, C₁₋₆ alkyl, C₁₋₆ deuterated alkyl, or C₃₋₆ cycloalkyl, preferably hydrogen, deuterium, C₁₋₃ alkyl, or C₁₋₃ deuterated alkyl;
n1 is an integer of 1-4;
Y is H or 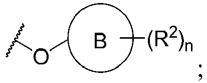
ring A and ring B are each independently C₆₋₁₄ aryl or 5- to 14-membered heteroaryl containing 1-4 heteroatoms selected from N, O, and S; when Y is not H, ring A is a group that is at least divalent;
R¹ and R² are each independently hydrogen, deuterium, halogen, hydroxyl, cyano, amino, oxo, C₁₋₆ alkyl, C₁₋₆ deuterated alkyl, C₁₋₆ haloalkyl, C₁₋₆ alkoxy, C₁₋₆ alkylthio, C₁₋₆ alkylamino, C₂₋₆ alkenyl, C₂₋₆ alkynyl, -S(O)-C₁₋₆ alkyl, -S(O)₂-C₁₋₆ alkyl, C₃₋₆ cycloalkyl, or -S(O)₂-C₃₋₆ cycloalkyl;
m and n are each independently an integer of 0-6;
R^{a} is hydrogen, deuterium, halogen, hydroxyl, cyano, amino, oxo, C₁₋₆ alkyl, C₁₋₆ deuterated alkyl, C₁₋₆ haloalkyl, C₁₋₆ alkoxy, C₁₋₆ alkylthio, C₁₋₆ alkylamino, or C₃₋₆ cycloalkyl, preferably hydrogen, deuterium, halogen, hydroxyl, cyano, amino, oxo, C₁₋₃ alkyl, C₁₋₃ deuterated alkyl, C₁₋₃ haloalkyl, C₁₋₃ alkoxy, C₁₋₃ alkylthio, or C₁₋₃ alkylamino;
x is an integer of 0-5.

In a further preferred embodiment of the present disclosure, general formula (IA) is further represented by general formula (I): 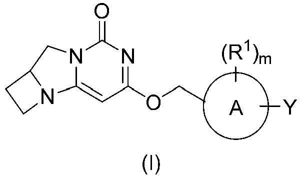 wherein:
Y is H or 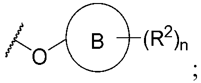
ring A and ring B are each independently C₆₋₁₄ aryl or 5- to 14-membered heteroaryl containing 1-4 heteroatoms selected from N, O, and S;
R¹ and R² are each independently hydrogen, deuterium, halogen, hydroxyl, cyano, amino, C₁₋₆ alkyl, C₁₋₆ deuterated alkyl, C₁₋₆ haloalkyl, C₁₋₆ alkoxy, C₁₋₆ alkylthio, or C₁₋₆ alkylamino;
m and n are each independently 0, 1, 2, 3, or 4.
In a further preferred embodiment of the present disclosure, ring A and ring B are each independently C₆₋₁₂ aryl or 5- to 10-membered heteroaryl containing 1-4 heteroatoms selected from N, O, and S, preferably C₆₋₁₀ aryl or 5- to 6-membered heteroaryl containing 1-3 heteroatoms selected from N, O, and S, preferably phenyl, naphthyl, pyridinyl, pyrimidinyl, or pyrazolyl, and more preferably 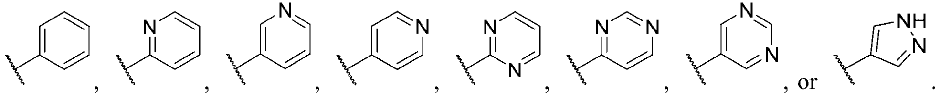 It should be noted that, in the case that the basic valence bond theory is satisfied, when Y is not H, ring A depicted herein is actually a group that is divalent or above, depending on the number of R¹ substituents on ring A. The same applies hereinafter.

In a further preferred embodiment of the present disclosure, ring A is

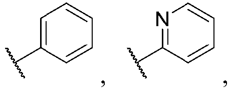

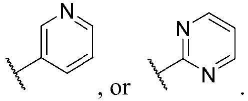

In a further preferred embodiment of the present disclosure, when Y is H, ring A is

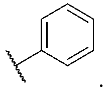

In a further preferred embodiment of the present disclosure, when Y is 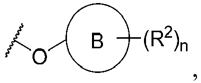 ring A is

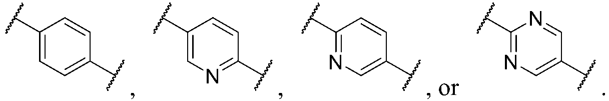

In a further preferred embodiment of the present disclosure, ring B is

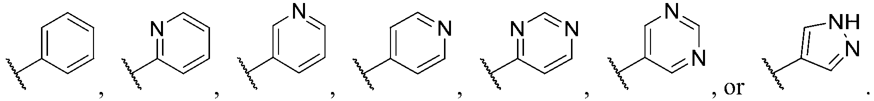

In a further preferred embodiment of the present disclosure, R¹ and R² are each independently hydrogen, deuterium, halogen, cyano, C₁₋₆ alkyl, C₁₋₆ deuterated alkyl, or C₁₋₆ haloalkyl, preferably hydrogen, deuterium, halogen, cyano, C₁₋₃ alkyl, C₁₋₃ deuterated alkyl, or C₁₋₃ haloalkyl, and more preferably hydrogen, deuterium, fluorine, chlorine, bromine, cyano, methyl, ethyl, n-propyl, isopropyl, or trifluoromethyl.

In a further preferred embodiment of the present disclosure, R¹ is hydrogen, fluorine, chlorine, cyano, methyl, or trifluoromethyl.

In a further preferred embodiment of the present disclosure, R² is hydrogen, fluorine, chlorine, cyano, methyl, ethyl, isopropyl, or trifluoromethyl.

In a further preferred embodiment of the present disclosure, m and n are each independently 0, 1, 2, or 3.

In a further preferred embodiment of the present disclosure, m is 0, 1, 2, or 3.

In a further preferred embodiment of the present disclosure, n is 0, 1, or 2.

In a further preferred embodiment of the present disclosure, the compound represented by general formula (I) is: 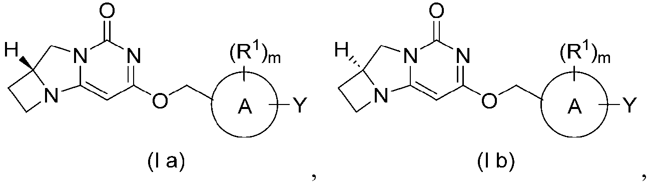 or a mixture thereof.

In a further preferred embodiment of the present disclosure, 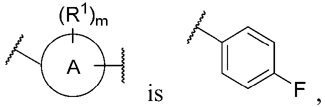 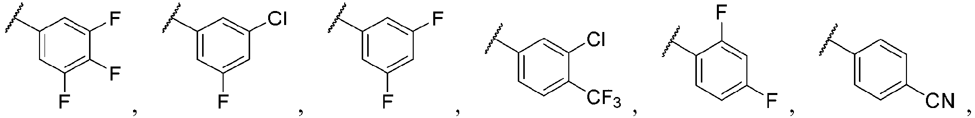 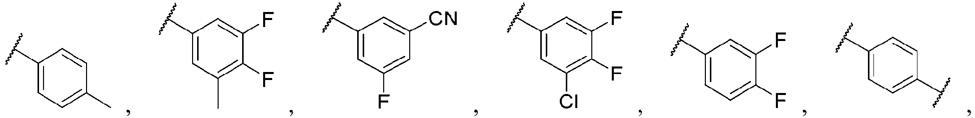 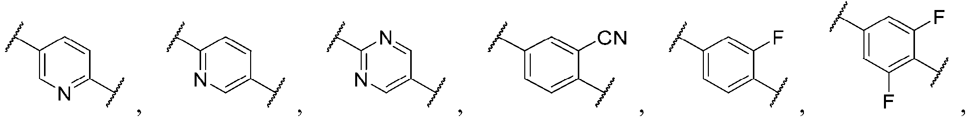 or

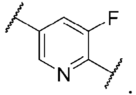

In a further preferred embodiment of the present disclosure,

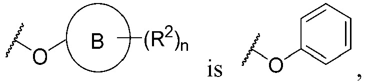

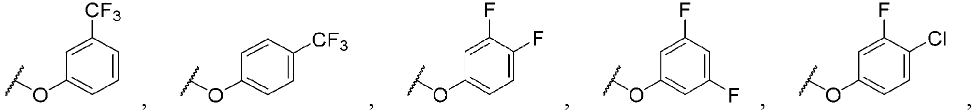

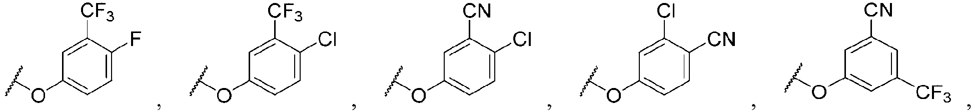

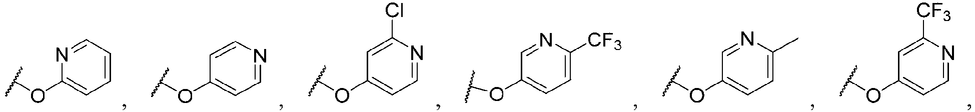

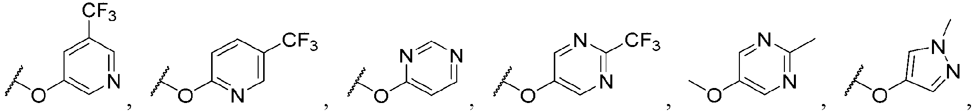

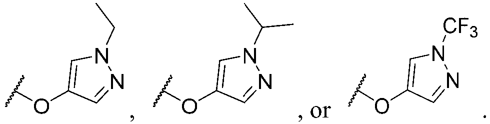

In a further preferred embodiment of the present disclosure, 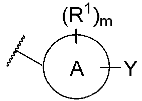 is 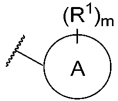 or 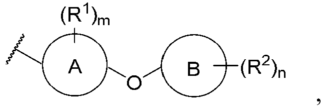 preferably

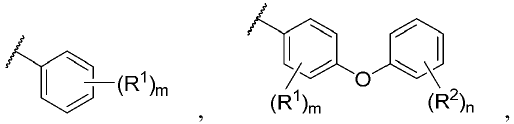

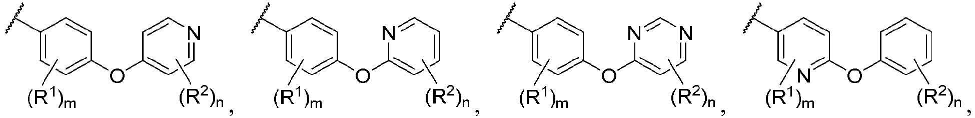

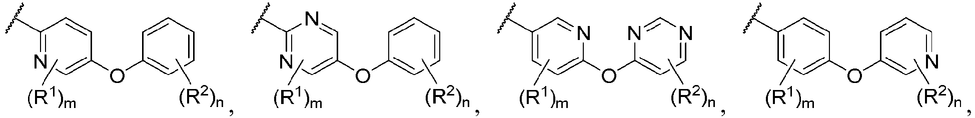

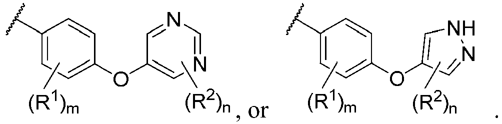

In a further preferred embodiment of the present disclosure, 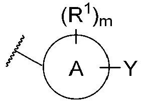 is

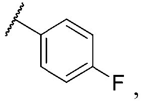

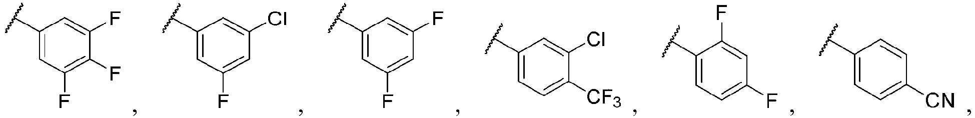

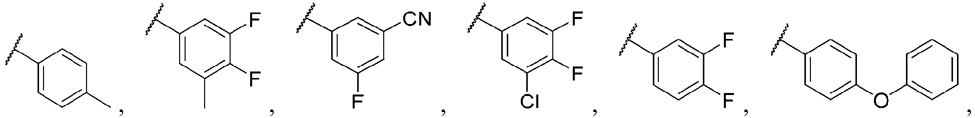

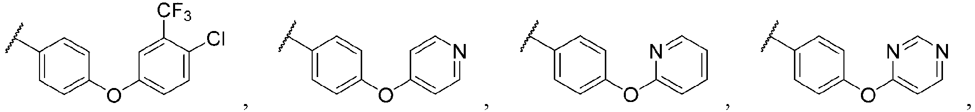

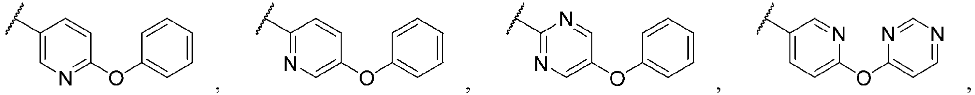

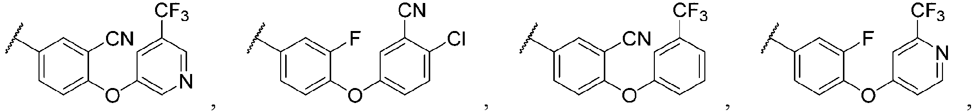

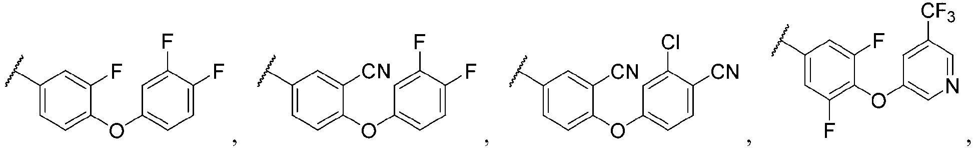

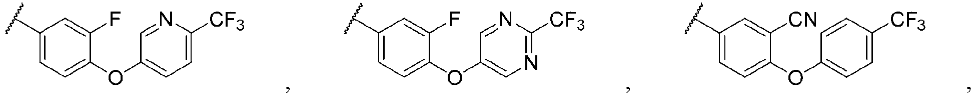

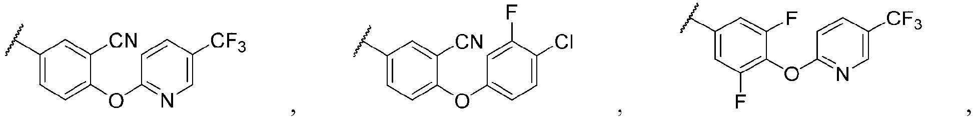

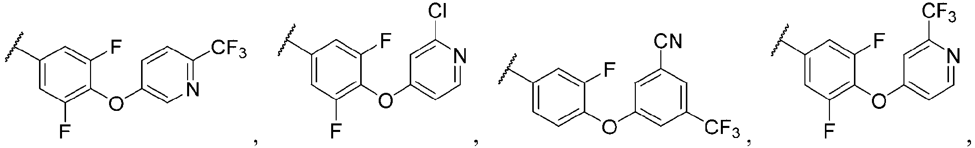

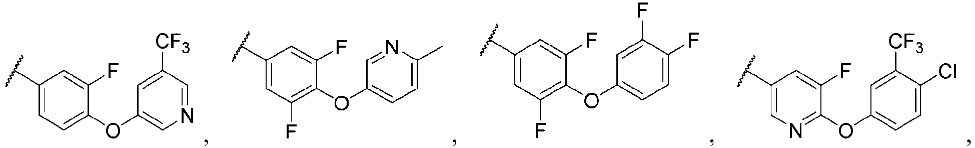

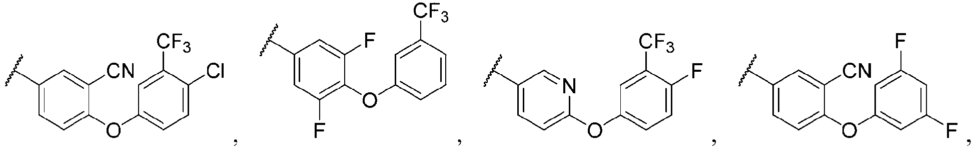

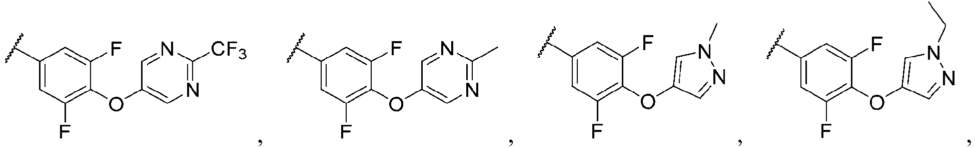

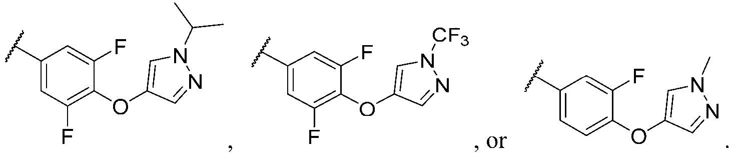

In a further preferred embodiment of the present disclosure, general formula (I) is further represented by general formula (II):

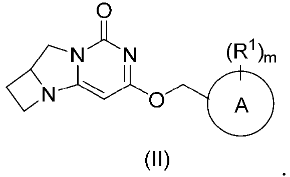

In a further preferred embodiment of the present disclosure, general formula (II) is:

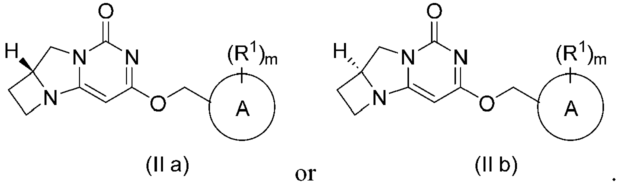

In a further preferred embodiment of the present disclosure, general formula (I) is further represented by general formula (III):

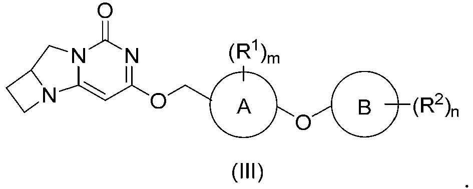

In a further preferred embodiment of the present disclosure, general formula (III) is:

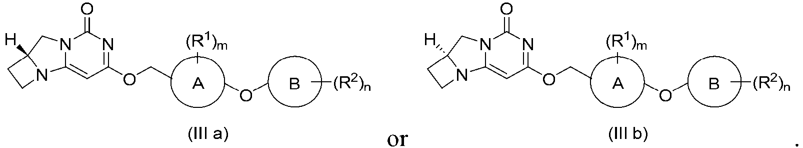

In a further preferred embodiment of the present disclosure, general formula (I) is further a compound as follows:

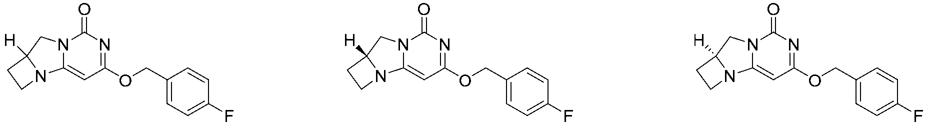

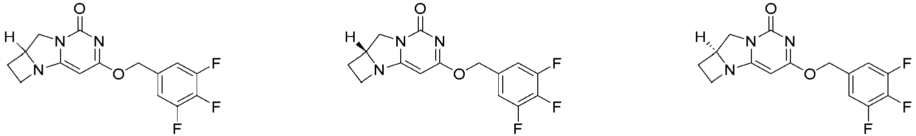

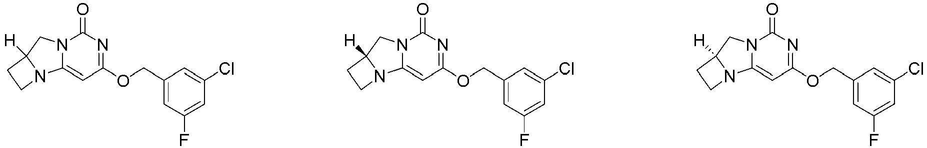

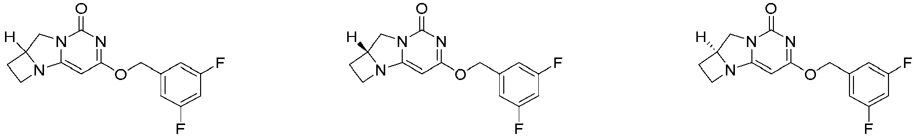

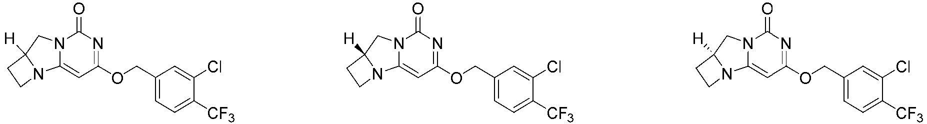

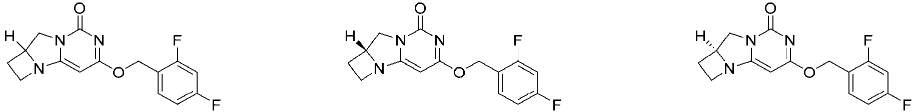

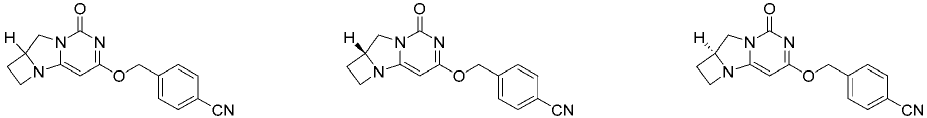

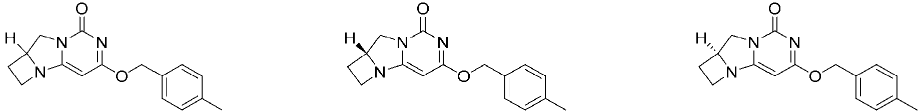

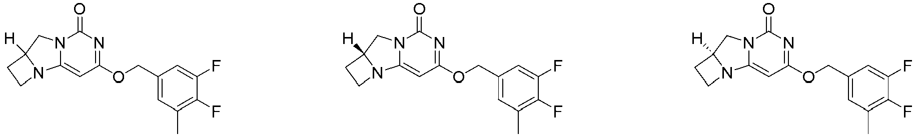

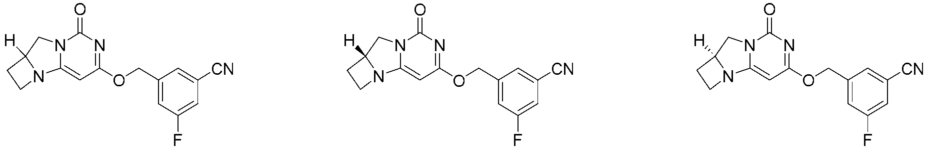

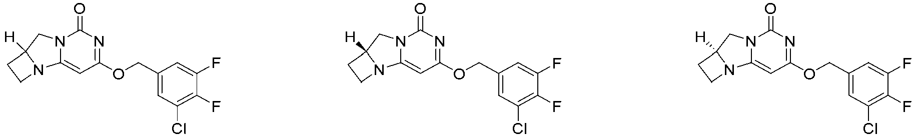

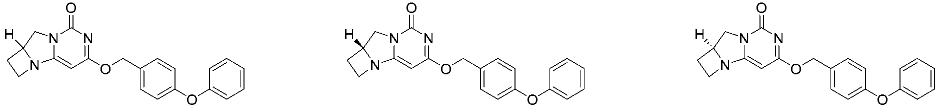

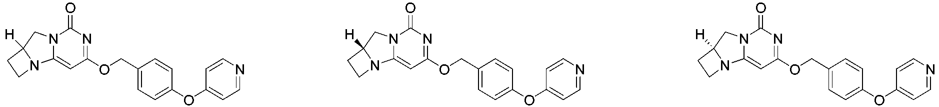

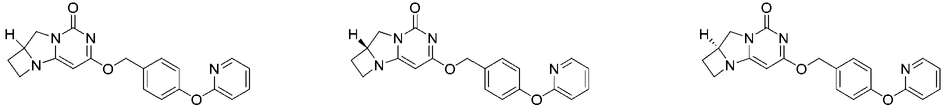

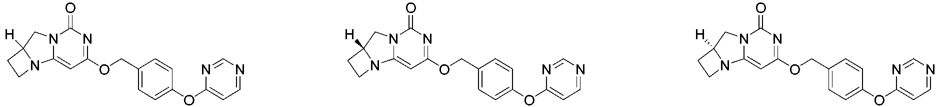

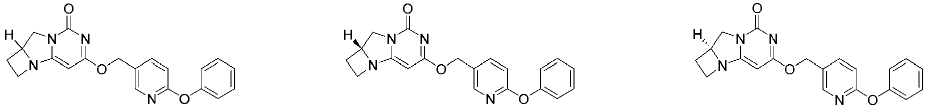

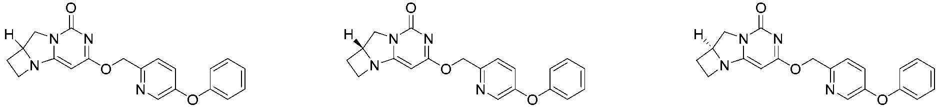

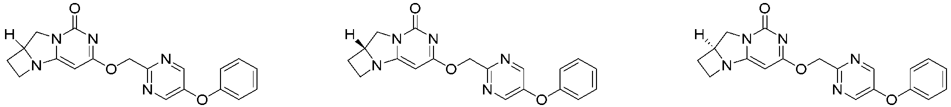

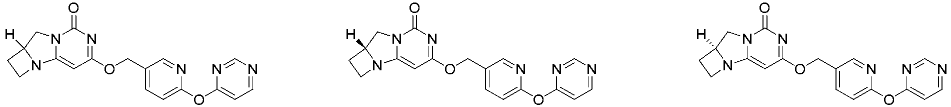

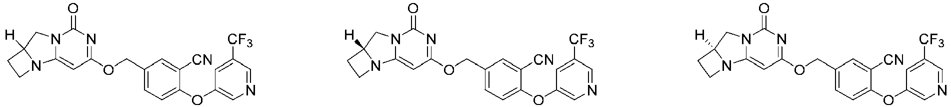

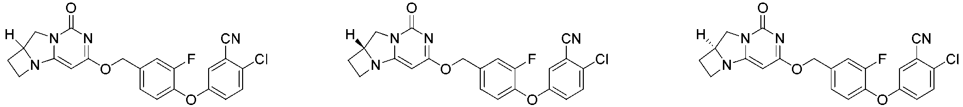

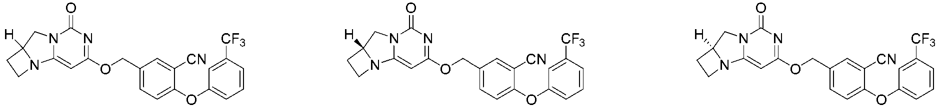

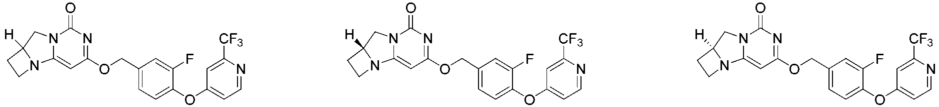

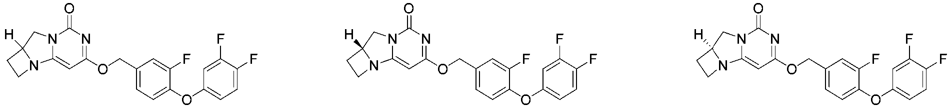

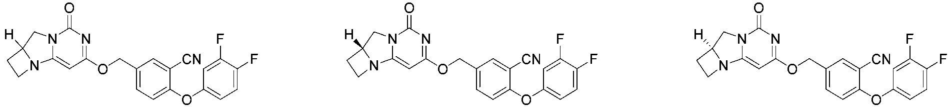

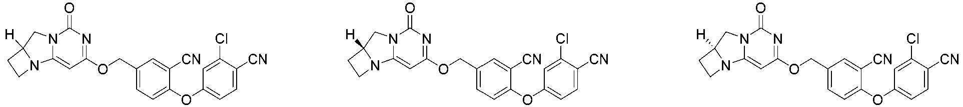

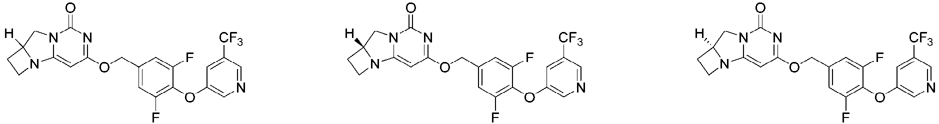

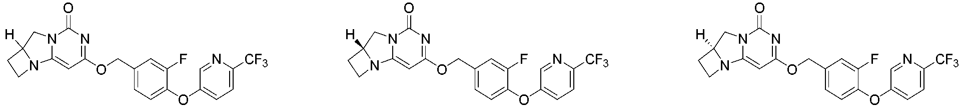

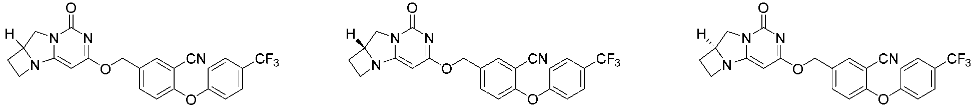

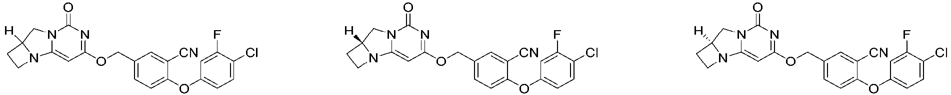

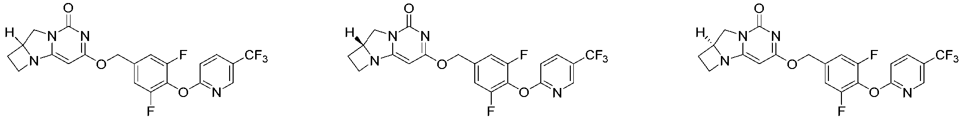

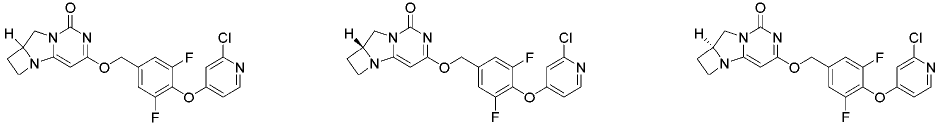

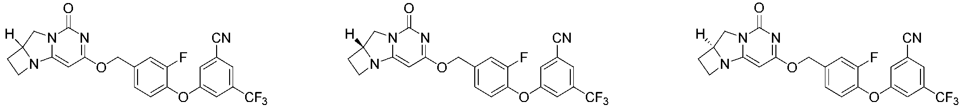

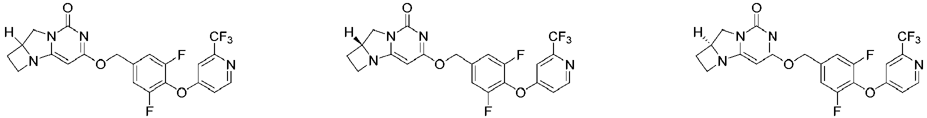

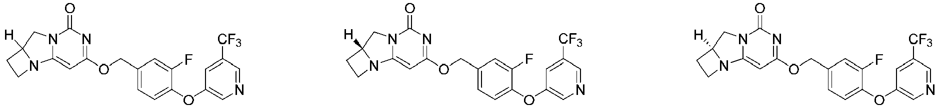

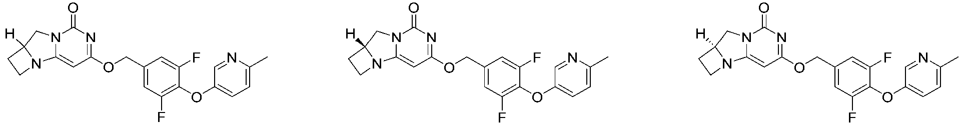

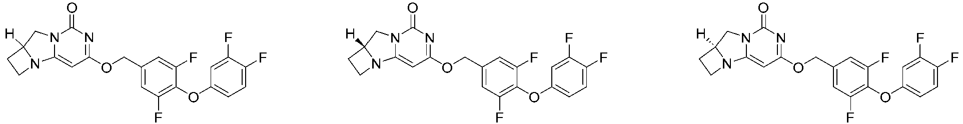

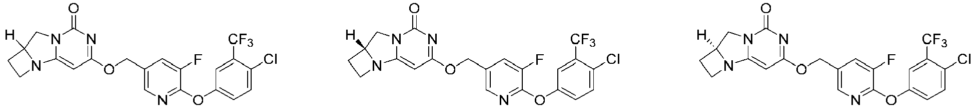

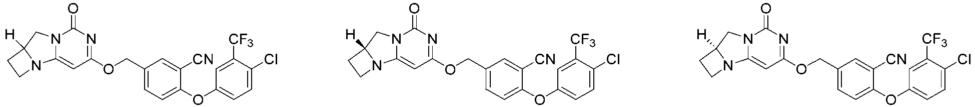

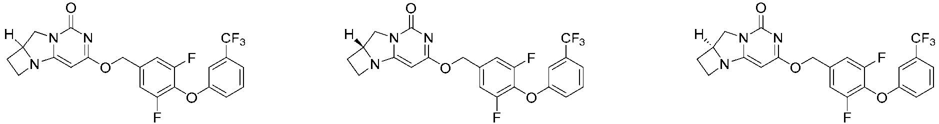

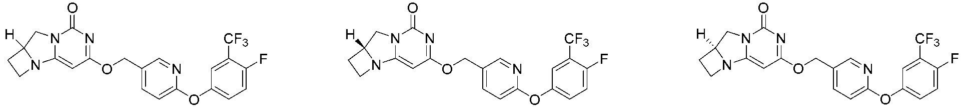

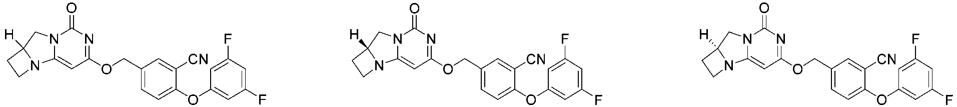

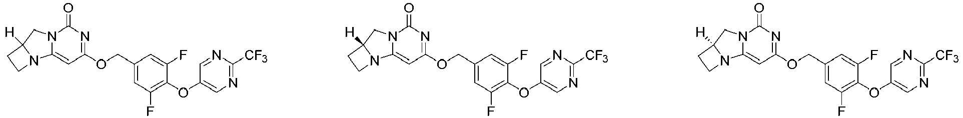

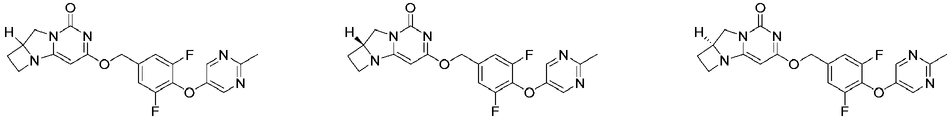

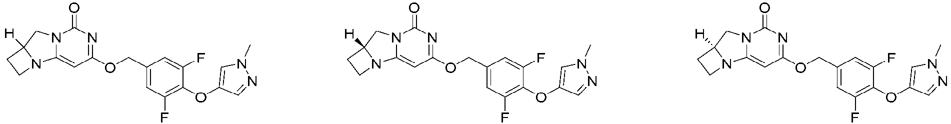

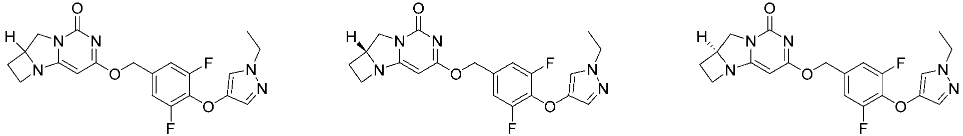

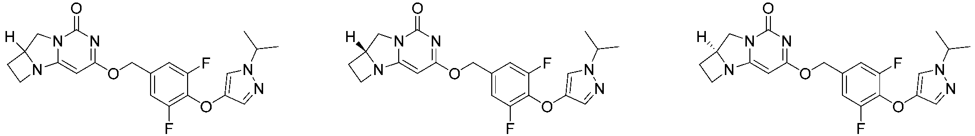

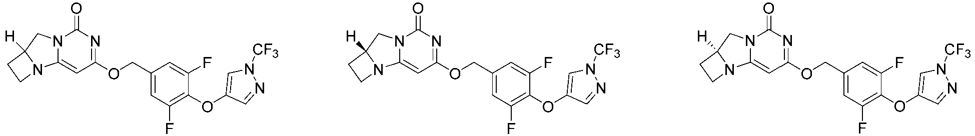

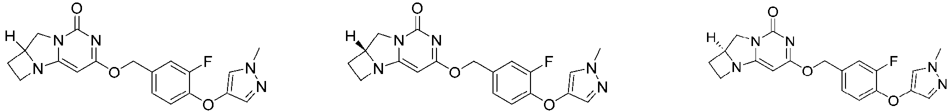

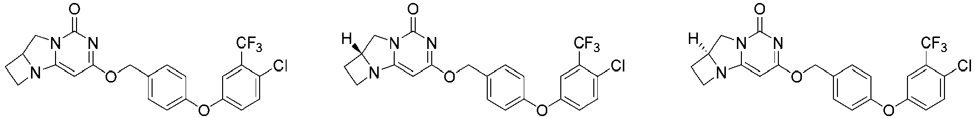

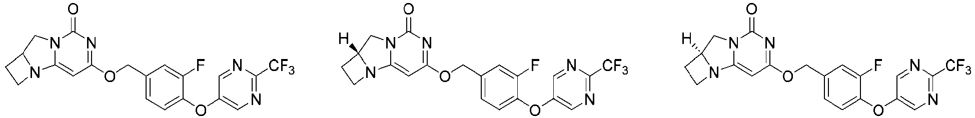

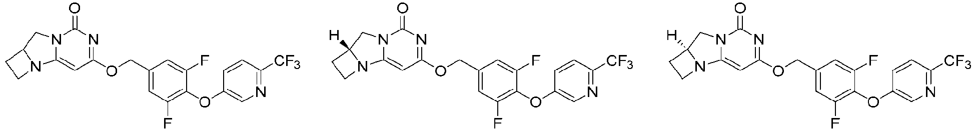

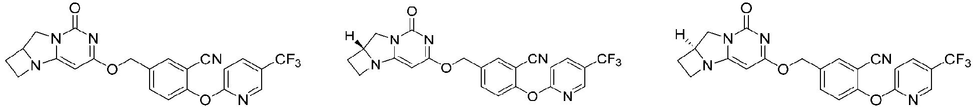

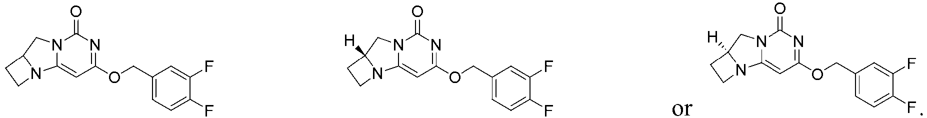

The present disclosure also provides a compound represented by formula (IA-1) or (IA-2), a stereoisomer thereof, or a salt thereof: 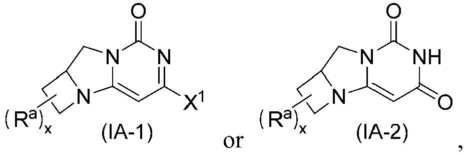 wherein X¹ is halogen, and R^{a} and x are as described above.

Further, (IA-1) or (IA-2) is preferably (I-1) or (I-2), respectively: 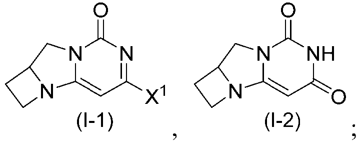 preferably, (I-1) is 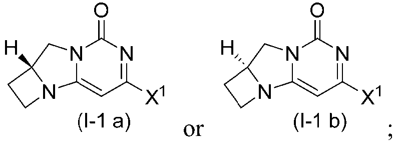 (I-2) is 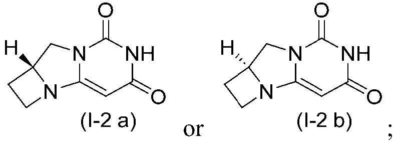 wherein X¹ is halogen, preferably fluorine, chlorine, bromine, or iodine, and more preferably chlorine. The preferred conditions described above may be combined arbitrarily to obtain preferred embodiments of the present disclosure on the basis of conventional knowledge in the art.

The present disclosure further provides a method for preparing a compound represented by general formula (IA), a stereoisomer thereof, or a pharmaceutically acceptable salt thereof. A commercially available starting material may be used in the preparation method, which is synthesized according to a known method.

In a certain embodiment, the preparation method preferably comprises the following step:
in a solvent, reacting a compound represented by general formula (IA-1) with a compound represented by general formula (IA-3) to give a compound represented by general formula (IA); 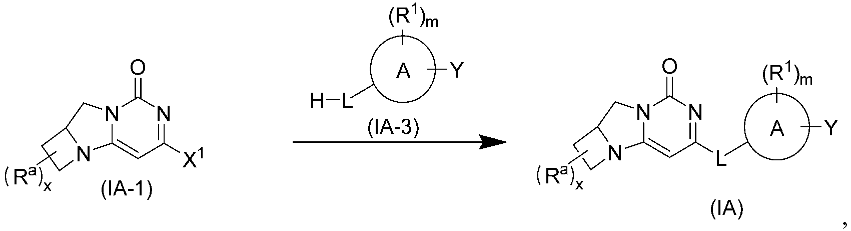
wherein:
   X¹ is halogen, preferably fluorine, chlorine, bromine, or iodine;
   L, R^{a}, x, ring A, R¹, Y, and m are as described above.

In a certain embodiment, the conditions of the reaction may be conventional conditions in the art for such reactions.

In a certain embodiment, the solvent may be a conventional solvent in the art for such reactions, such as water or an organic solvent, preferably one or more of water, methanol, ethanol, ethylene glycol, propylene glycol, n-propanol, isopropanol, n-butanol, isobutanol, tert-butanol, glacial acetic acid, acetone, butanone, 3-pentanone, n-hexane, cyclohexane, n-heptane, isopropyl ether, methyl tert-butyl ether, petroleum ether, N-methylpyrrolidone, N,N-dimethylformamide, N,N-dimethylacetamide, dimethyl sulfoxide, dichloromethane, trichloromethane, 1,2-dichloroethane, ethyl formate, methyl acetate, ethyl acetate, isopropyl acetate, acetonitrile, tetrahydrofuran, 1,4-dioxane, 1,2-dioxane, benzene, or toluene.

The present disclosure further provides a method for preparing a compound represented by general formula (I), a stereoisomer thereof, or a pharmaceutically acceptable salt thereof. A commercially available starting material may be used in the preparation method, which is synthesized according to a known method.

In a certain embodiment, the preparation method preferably comprises the following step:
in a solvent, reacting a compound represented by general formula (I-1) with a compound represented by general formula (I-3) to give a compound represented by general formula (I); 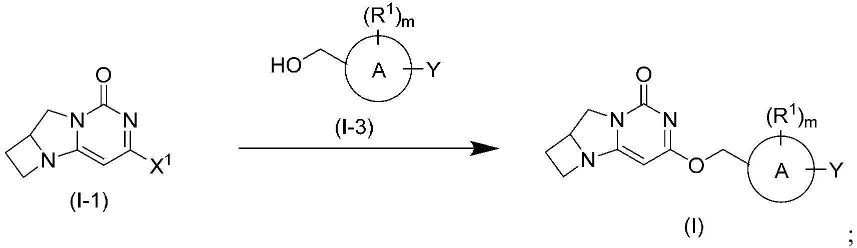
or in a solvent, reacting a compound represented by general formula (I-2) with a compound represented by general formula (I-4) to give a compound represented by general formula (I); 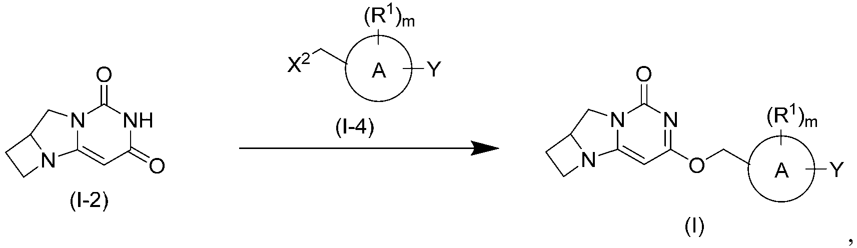
wherein:
   X¹ and X² are each independently halogen, preferably fluorine, chlorine, bromine, or iodine;
   ring A, R¹, Y, and m are as described above.

In a certain embodiment, the conditions of the reaction may be conventional conditions in the art for such reactions.

In a certain embodiment, the solvent may be a conventional solvent in the art for such reactions, such as water or an organic solvent, preferably one or more of water, methanol, ethanol, ethylene glycol, propylene glycol, n-propanol, isopropanol, n-butanol, isobutanol, tert-butanol, glacial acetic acid, acetone, butanone, 3-pentanone, n-hexane, cyclohexane, n-heptane, isopropyl ether, methyl tert-butyl ether, petroleum ether, N-methylpyrrolidone, N,N-dimethylformamide, N,N-dimethylacetamide, dimethyl sulfoxide, dichloromethane, trichloromethane, 1,2-dichloroethane, ethyl formate, methyl acetate, ethyl acetate, isopropyl acetate, acetonitrile, tetrahydrofuran, 1,4-dioxane, 1,2-dioxane, benzene, or toluene.

The present disclosure further relates to a pharmaceutical composition comprising a therapeutically effective dose of any one of the compounds, the stereoisomers thereof, the pharmaceutically acceptable salts thereof, or the prodrugs thereof, and one or more pharmaceutically acceptable carriers. In some embodiments of the present disclosure, the pharmaceutical composition described above may be formulated in a conventional manner using one or more pharmaceutically acceptable carriers. The carriers refer to carriers conventional in the pharmaceutical field, for example: diluents such as water; binders such as cellulose derivatives, gelatin, and polyvinylpyrrolidone; fillers such as starch; disintegrants such as calcium carbonate and sodium bicarbonate; lubricants such as calcium stearate or magnesium stearate. In addition, other auxiliary agents such as sweetening agents, fragrances, or colorants may also be added to the composition.

In some embodiments of the present disclosure, the pharmaceutical composition may be administered in any of the following ways: oral administration, spray inhalation, rectal administration, nasal administration, buccal administration, topical administration, parenteral administration such as subcutaneous, intravenous, intramuscular, intraperitoneal, intrathecal, intraventricular, intrasternal, or intracranial injection or infusion, or administration by means of an implantable reservoir. Among them, oral administration is the preferred way.

When being administered orally, the compound of the present application may be prepared into any orally acceptable formulation forms, including, but not limited to, tablets, capsules, aqueous solutions, aqueous suspensions, etc.

The present disclosure further relates to use of any one of the compounds, the stereoisomers thereof, or the pharmaceutically acceptable salts thereof, or the pharmaceutical composition thereof in the preparation of a medicament, and the medicament is an Lp-PLA2 inhibitor medicament.

The present disclosure further relates to use of any one of the compounds, the stereoisomers thereof, or the pharmaceutically acceptable salts thereof, or the pharmaceutical composition thereof in the preparation of a medicament, and the medicament is a medicament for a related disease mediated by Lp-PLA2.

The present disclosure also relates to a method for preventing and/or treating a related disease mediated by Lp-PLA2, which comprises administering to a mammal a therapeutically effective dose of any one of the compounds, the stereoisomers thereof, or the pharmaceutically acceptable salts, esters, prodrugs, solvates, hydrates, or derivatives thereof, or the pharmaceutical composition thereof. The present disclosure also provides a method for treating a disease condition, including but not limited to conditions associated with Lp-PLA2 dysfunction, by using the compound or pharmaceutical composition of the present disclosure.

In a further preferred embodiment of the present disclosure, the disease is one or more of a neurodegenerative disease, a cardiovascular and cerebrovascular disease, and a diabetic complication. In a further preferred embodiment of the present disclosure, the neurodegenerative disease is dementia, amyotrophic lateral sclerosis, or Parkinson's disease.

In a further preferred embodiment of the present disclosure, the dementia is Alzheimer's disease, vascular dementia, or multi-infarct dementia.

In a further preferred embodiment of the present disclosure, the cardiovascular and cerebrovascular disease is coronary heart disease, cerebral stroke, or atherosclerosis.

In a further preferred embodiment of the present disclosure, the diabetic complication is diabetic retinopathy, diabetic macular edema, diabetic nephropathy, diabetic neuropathy, diabetic peripheral neuropathic pain, or diabetic foot.

### DETAILED DESCRIPTION OF THE PRESENT DISCLOSURE

Unless otherwise defined, all technical and scientific terms used herein have the same meaning as that commonly understood by those of ordinary skill in the art to which the present disclosure pertains. In the event of a contradiction, the definition provided in the present application shall prevail. When a trade name appears herein, it is intended to refer to its corresponding commercial product or its active ingredient. All patents, published patent applications, and publications cited herein are incorporated herein by reference.

The term "alkyl" refers to a saturated linear or branched aliphatic hydrocarbon group, which has 1 to 20 (e.g., 1, 2, 3, 4, 5, 6, 7, 8, 9, 10, 11, 12, 13, 14, 15, 16, 17, 18, 19, or 20) carbon atoms, i.e., "C₁₋₂₀ alkyl". The alkyl is preferably an alkyl group having 1 to 12 carbon atoms (i.e., C₁₋₁₂ alkyl), more preferably an alkyl group having 1 to 8 carbon atoms (i.e., C₁₋₈ alkyl), further preferably an alkyl group having 1 to 6 carbon atoms (i.e., C₁₋₆ alkyl), and most preferably an alkyl group having 1 to 3 carbon atoms (i.e., C₁₋₃ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, and the like.

The term "alkylene" refers to a divalent alkyl group, wherein the alkyl is as defined above and has 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). The alkylene is preferably an alkylene group having 1 to 12 carbon atoms (i.e., C₁₋₁₂ alkylene), more preferably an alkylene group having 1 to 8 carbon atoms (i.e., C₁₋₈ alkylene), further preferably an alkylene group having 1 to 6 carbon atoms (i.e., C₁₋₆ alkylene), and most preferably an alkylene group having 1 to 3 carbon atoms (i.e., C₁₋₃ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, -CH₂CH₂CH₂CH₂-, and the like.

The term "alkenyl" refers to an alkyl group containing at least one carbon-carbon double bond in the molecule, wherein the alkyl is as defined above and has 2 to 12 (e.g., 2, 3, 4, 5, 6, 7, 8, 9, 10, 11, or 12) carbon atoms (i.e., C₂₋₁₂ alkenyl). The alkenyl is preferably an alkenyl group having 2 to 6 carbon atoms (i.e., C₂₋₆ alkenyl). Non-limiting examples include: ethenyl, 1-propenyl, 2-propenyl, 1-, 2-, or 3-butenyl, and the like.

The term "alkynyl" refers to an alkyl group containing at least one carbon-carbon triple bond in the molecule, wherein the alkyl is as defined above and has 2 to 12 (e.g., 2, 3, 4, 5, 6, 7, 8, 9, 10, 11, or 12) carbon atoms (i.e., C₂₋₁₂ alkynyl). The alkynyl is preferably an alkynyl group having 2 to 6 carbon atoms (i.e., C₂₋₆ alkynyl). Non-limiting examples include: ethynyl, propynyl, butynyl, pentynyl, hexynyl, and the like.

The term "cycloalkyl" refers to a saturated or partially unsaturated monocyclic cyclic hydrocarbon substituent (i.e., monocyclic cycloalkyl) or polycyclic cyclic hydrocarbon substituent (i.e., polycyclic cycloalkyl), which has 3 to 20 (e.g., 3, 4, 5, 6, 7, 8, 9, 10, 11, 12, 13, 14, 15, 16, 17, 18, 19, or 20) carbon atoms, i.e., C₃₋₂₀ cycloalkyl. The cycloalkyl is preferably a cycloalkyl group having 3 to 12 carbon atoms (i.e., C₃₋₁₂ cycloalkyl), more preferably a cycloalkyl group having 3 to 8 carbon atoms (i.e., C₃₋₈ cycloalkyl), further preferably a cycloalkyl group having 3 to 6 carbon atoms (i.e., C₃₋₆ cycloalkyl), most preferably a cycloalkyl group having 3 to 5 carbon atoms (i.e., C₃₋₅ cycloalkyl), or a cycloalkyl group having 5 to 6 carbon atoms (i.e., C₅₋₆ cycloalkyl). Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Non-limiting examples of the polycyclic cycloalkyl include: spiro cycloalkyl, fused cycloalkyl, and bridged cycloalkyl. The cycloalkyl may be optionally substituted or unsubstituted. When it is substituted, the substituent may be substituted at any available linking site, and the substituent is preferably one or more of the following groups independently selected from deuterium, halogen, hydroxyl, amino, cyano, oxo, alkyl, deuterated alkyl, haloalkyl, alkoxy, alkylthio, and alkylamino.

The term "heterocyclyl" refers to a saturated or partially unsaturated monocyclic heterocyclic hydrocarbon substituent (i.e., monocyclic heterocyclyl) or a polycyclic heterocyclic hydrocarbon substituent (i.e., polycyclic heterocyclyl), which has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl), wherein one or more (e.g., 1, 2, 3, or 4) ring atoms are heteroatoms selected from nitrogen, oxygen, P(O)ₘ, and S(O)ₙ (where m and n are integers of 0-2), excluding a ring moiety of -O-O-, -O-S-, or -S-S-, and the remaining ring atoms are carbon atoms. Preferably, the heterocyclyl has 3 to 12 ring atoms (i.e., 3- to 12-membered heterocyclyl), of which 1-4 heteroatoms are selected from N, O, and S atoms; more preferably, the heterocyclyl has 3 to 8 ring atoms (i.e., 3- to 8-membered heterocyclyl), of which 1-4, 1-3, or 1-2 heteroatoms are selected from N, O, and S atoms; further preferably, the heterocyclyl has 3 to 6 ring atoms (i.e., 3- to 6-membered heterocyclyl), of which 1-4, 1-3, or 1-2 heteroatoms are selected from N, O, and S atoms; most preferably, the heterocyclyl has 5 to 6 ring atoms (i.e., 5- to 6-membered heterocyclyl), of which 1-4, 1-3, or 1-2 heteroatoms are selected from N, O, and S atoms. Non-limiting examples of the monocyclic heterocyclyl include: azetidinyl, oxetanyl, thietanyl, pyrrolidinyl, imidazolidinyl, tetrahydrofuranyl, tetrahydrothienyl, tetrahydropyranyl, dihydroimidazolyl, dihydrofuranyl, dihydropyrazolyl, piperidinyl, piperazinyl, morpholinyl, 1,3-dioxolanyl, 2,2-difluoro-1,3-dioxolanyl, cyclopentanonyl, 2,2-difluorocyclopentanonyl, azepinyl, oxolanyl, azolidinyl, or the like. Non-limiting examples of the polycyclic heterocyclyl include: spiro heterocyclyl, fused heterocyclyl, and bridged heterocyclyl. The heterocyclyl may be optionally substituted or unsubstituted. When it is substituted, the substituent may be substituted at any available linking site, and the substituent is preferably one or more of the following groups independently selected from deuterium, halogen, hydroxyl, amino, cyano, oxo, alkyl, deuterated alkyl, haloalkyl, alkoxy, alkylthio, and alkylamino.

The term "aryl" refers to an all-carbon monocyclic group (i.e., monocyclic aryl) or a fused polycyclic group (i.e., polycyclic aryl) having a conjugated π-electron system, which has 6 to 14 (e.g., 6, 7, 8, 9, 10, 11, 12, 13, or 14) carbon atoms (i.e., C₆₋₁₄ aryl). The aryl is preferably an aryl group having 6 to 12 carbon atoms (i.e., C₆₋₁₂ aryl), more preferably an aryl group having 6 to 10 carbon atoms (i.e., C₆₋₁₀ aryl), further preferably phenyl or naphthyl, and most preferably phenyl. The monocyclic aryl is, for example, phenyl. Non-limiting examples of the polycyclic aryl include: naphthyl, anthryl, phenanthryl, and the like. The aryl may be optionally substituted or unsubstituted. When it is substituted, the substituent may be substituted at any available linking site, and the substituent is preferably one or more of the following groups independently selected from deuterium, halogen, hydroxyl, amino, cyano, oxo, alkyl, deuterated alkyl, haloalkyl, alkoxy, alkylthio, and alkylamino.

The term "heteroaryl" refers to a monocyclic heteroaryl group (i.e., monocyclic heteroaryl) or a fused polycyclic heteroaryl group (i.e., polycyclic heteroaryl) having a conjugated π-electron system, which has 5 to 14 (e.g., 5, 6, 7, 8, 9, 10, 11, 12, 13, or 14) ring atoms (i.e., 5- to 14-membered heteroaryl), wherein one or more (e.g., 1, 2, 3, or 4) ring atoms are heteroatoms selected from nitrogen, oxygen, P(O)ₘ, and S(O)ₙ (where m and n are integers of 0-2), preferably heteroatoms selected from nitrogen, oxygen, and sulfur, excluding a ring moiety of -O-O-, -O-S-, or -S-S-, and the remaining ring atoms are carbon atoms. The heteroaryl is preferably 5- to 10-membered heteroaryl containing 1-4 heteroatoms selected from N, O, and S. The monocyclic heteroaryl is preferably 5- to 6-membered heteroaryl containing 1-3 heteroatoms selected from N, O, and S, and non-limiting examples include: furanyl, pyranyl, thienyl, thiazolyl, isothiazolyl, oxazolyl, isoxazolyl, oxadiazolyl, thiadiazolyl, imidazolyl, pyrazolyl, triazolyl, tetrazolyl, pyrrolyl, pyridinyl, pyrimidinyl, pyridonyl, pyrazinyl, pyridazinyl, and the like. The polycyclic heteroaryl is preferably 5- to 6-membered heteroaryl containing 1-3 heteroatoms selected from N, O, and S fused to 5- to 6-membered heteroaryl containing 1-3 heteroatoms selected from N, O, and S, 5- to 10-membered heteroaryl containing 1-4 heteroatoms selected from N, O, and S fused to C₆₋₁₀ aryl, or C₆₋₁₀ aryl fused to 5- to 10-membered heteroaryl containing 1-4 heteroatoms selected from N, O, and S, further preferably 5- to 6-membered heteroaryl containing 1-3 heteroatoms selected from N, O, and S fused to 5- to 6-membered heteroaryl containing 1-3 heteroatoms selected from N, O, and S, 5- to 6-membered heteroaryl containing 1-3 heteroatoms selected from N, O, and S fused to phenyl, or phenyl fused to 5- to 6-membered heteroaryl containing 1-3 heteroatoms selected from N, O, and S; non-limiting examples include: indolyl, indazolyl, quinolinyl, isoquinolinyl, quinoxalinyl, phthalazinyl, benzimidazolyl, benzothienyl, thienophenyl, quinazolinyl, benzothiazolyl, carbazolyl, thienopyridinyl, pyridothienyl, pyridopyrrolyl, and the like. The heteroaryl may be optionally substituted or unsubstituted. When it is substituted, the substituent may be substituted at any available linking site, and the substituent is preferably one or more of the following groups independently selected from deuterium, halogen, hydroxyl, amino, cyano, oxo, alkyl, deuterated alkyl, haloalkyl, alkoxy, alkylthio, and alkylamino. The term "alkoxy" refers to -O-(alkyl) or -O-(unsubstituted cycloalkyl), wherein the alkyl and cycloalkyl are as defined above and have 1 to 10 (e.g., 1, 2, 3, 4, 5, 6, 7, 8, 9, or 10) carbon atoms (i.e., C₁₋₁₀ alkoxy). The alkoxy is preferably an alkoxy group having 1 to 8 carbon atoms (i.e., C₁₋₈ alkoxy), more preferably an alkoxy group having 1 to 6 carbon atoms (i.e., C₁₋₆ alkoxy), and most preferably an alkoxy group having 1 to 3 carbon atoms (i.e., C₁₋₃ alkoxy). Non-limiting examples include: methoxy, ethoxy, propoxy, butoxy, cyclopropyloxy, cyclobutyloxy, cyclopentyloxy, cyclohexyloxy, and the like.

The term "alkylthio" refers to -S-(alkyl) or -S-(unsubstituted cycloalkyl), wherein the alkyl and cycloalkyl are as defined above and have 1 to 10 (e.g., 1, 2, 3, 4, 5, 6, 7, 8, 9, or 10) carbon atoms (i.e., C₁₋₁₀ alkylthio). The alkylthio is preferably an alkylthio group having 1 to 8 carbon atoms (i.e., C₁₋₈ alkylthio), more preferably an alkylthio group having 1 to 6 carbon atoms (i.e., C₁₋₆ alkylthio), and most preferably an alkylthio group having 1 to 3 carbon atoms (i.e., C₁₋₃ alkylthio). Non-limiting examples include: methylthio, ethylthio, propylthio, butylthio, cyclopropylthio, cyclobutylthio, cyclopentylthio, cyclohexylthio, and the like.

The term "alkylamino" refers to -NH-(alkyl or unsubstituted cycloalkyl) or -N-(alkyl or unsubstituted cycloalkyl)-(alkyl or unsubstituted cycloalkyl), wherein the alkyl and cycloalkyl are as defined above. Non-limiting examples of the alkylamino include: methylamino, ethylamino, propylamino, butylamino, cyclopropylamino, cyclobutylamino, cyclopentylamino, cyclohexylamino, and the like. The term "halo", "halogen", or "halogenated" should be understood to refer to a fluorine (F), chlorine (Cl), bromine (Br), or iodine (I) atom, preferably a fluorine, chlorine, or bromine atom.

The term "haloalkyl" refers to alkyl substituted with one or more halogens, wherein the alkyl is as defined above. Non-limiting examples include: fluoromethyl, chloromethyl, bromomethyl, iodomethyl, difluoromethyl, chlorofluoromethyl, dichloromethyl, bromofluoromethyl, trifluoromethyl, chlorodifluoromethyl, dichlorofluoromethyl, trichloromethyl, bromodifluoromethyl, bromochlorofluoromethyl, dibromofluoromethyl, 2-fluoroethyl, 2-chloroethyl, 2-bromoethyl, 2,2-difluoroethyl, 2-chloro-2-fluoroethyl, 2,2-dichloroethyl, 2-bromo-2-fluoroethyl, 2,2,2-trifluoroethyl, 2-chloro-2,2-difluoroethyl, 2,2-dichloro-2-fluoroethyl, 2,2,2-trichloroethyl, 2-bromo-2,2-difluoroethyl, 2-bromo-2-chloro-2-fluoroethyl, 2-bromo-2,2-dichloroethyl, 1,1,2,2-tetrafluoroethyl, pentafluoroethyl, 1-chloro-1,2,2,2-tetrafluoroethyl, 2-chloro-1,1,2,2-tetrafluoroethyl, 1,2-dichloro-1,2,2-trifluoroethyl, 2-bromo-1,1,2,2-tetrafluoroethyl, and the like, preferably fluoromethyl, difluoromethyl, trifluoromethyl, 2-fluoroethyl, 2-chloroethyl, 2-bromoethyl, and 2,2-difluoroethyl. The term "deuterated alkyl" refers to alkyl substituted with one or more deuterium, wherein the alkyl is as defined above.

The term "hydroxyl" refers to -OH.

The term "amino" refers to -NH₂.

The term "cyano" refers to -CN.

The term "oxo" refers to =O.

"n-BuOH" refers to n-butanol. "MeOH" refers to methanol. "PPh₃" refers to triphenylphosphine. "DBAD" refers to di-tert-butyl azodicarboxylate. "ACN" refers to acetonitrile. "DMF" refers to N,N-dimethylformamide. "DMSO" refers to dimethyl sulfoxide. "DCM" refers to dichloromethane. "DIEA" refers to N,N-diisopropylethylamine. "EA" refers to ethyl acetate. "PE" refers to petroleum ether. "THF" refers to tetrahydrofuran. "TEA" refers to triethylamine.

The terms "comprise", "include", "have", "contain", or "involve" and other variations thereof herein are inclusive or open-ended and do not exclude additional unrecited elements or method steps. It should be understood by those skilled in the art that the terms described above such as "comprise" encompass the meaning of "consist of".

The term "one or more" or the similar expression "at least one" may indicate, for example, 1, 2, 3, 4, 5, 6, 7, 8, 9, 10, or more.

When the lower and upper limits of a range of numerical values are disclosed, any numerical value falling within the range and any included range are specifically disclosed. In particular, each range of values disclosed herein is to be understood as indicating each numerical value and range encompassed within a relatively broad range.

As used herein, "Z" and "-Z-" both refer to the same particular group, which can be used interchangeably.

The expression "m-n" as used herein refers to the range of m to n as well as to sub-ranges consisting of point values therein and the point values. For example, the expression "C₂-C₈" or "C₂₋₈" encompasses a range of 2 to 8 carbon atoms and should be understood as also encompassing any sub-range and each point value therein, e.g., C₂-C₅, C₃-C₄, C₂-C₆, C₃-C₆, C₄-C₆, C₄-C₇, C₄-C₈, and the like, as well as C₂, C₃, C₄, C₅, C₆, C₇, C₈, and the like. For example, the expression "C₃-C₁₀" or "C₃₋₁₀" should also be understood in a similar manner, for example, it can encompass any sub-range and point value therein, e.g., C₃-C₉, C₆-C₉, C₆-C₈, C₆-C₇, C₇-C₁₀, C₇-C₉, C₇-C₈, C₈-C₉, and the like, as well as C₃, C₄, C₅, C₆, C₇, C₈, C₉, C₁₀, and the like. For another example, the expression "C₁-C₆" or "C₁₋₆" encompasses a range of 1 to 6 carbon atoms and should be understood as also encompassing any sub-range and each point value therein, e.g., C₂-C₅, C₃-C₄, C₁-C₂, C₁-C₃, C₁-C₄, C₁-C₅, C₁-C₆, and the like, as well as C₁, C₂, C₃, C₄, C₅, C₆, and the like. For another example, the expression "three- to ten-membered" should be understood as encompassing any sub-range and each point value therein, e.g., three- to five-membered, three- to six-membered, three- to seven-membered, three- to eight-membered, four- to five-membered, four- to six-membered, four- to seven-membered, four- to eight-membered, five- to seven-membered, five- to eight-membered, six- to seven-membered, six- to eight-membered, nine- to ten-membered, and the like, as well as three-membered, four-membered, five-membered, six-membered, seven-membered, eight-membered, nine-membered, ten-membered, and the like. Other similar expressions herein should also be understood in a similar manner.

The expressions "X is selected from A, B, or C", "X is selected from A, B, and C", "X is A, B, or C", "X is A, B, and C", and the like as used herein all carry the same meaning, i.e., X may be any one or more of A, B, and C.

The term "optional" or "optionally" refers to that the subsequently described event or circumstance may occur or may not occur. The description includes instances where the event or circumstance occurs and instances where the event or circumstance does not occur. For example, "cycloalkyl optionally substituted with alkyl" means that alkyl may, but does not necessarily, exist. The description includes instances where the cycloalkyl is substituted with alkyl and instances where the cycloalkyl is not substituted with alkyl.

The terms "substitution" and "substituted" mean that one or more (e.g., one, two, three, or four) hydrogens on a specified atom are replaced by a choice from the designated groups, with the proviso that the normal valency of the specified atom in the present case is not exceeded and that the replacement results in a stable compound. A combination of substituents and/or variables is permissible only if the combination results in a stable compound. When it is stated that a certain substituent is absent, it should be understood that the substituent may be one or more hydrogen atoms, provided that the structure enables the compound to reach a stable state. When it is stated that each carbon atom in a group may optionally be replaced by a heteroatom, the proviso is that the normal valency of all atoms in the group in the present case is not exceeded, and that a stable compound is formed.

If a substituent is described as "optionally substituted", the substituent may be unsubstituted or may be substituted. If an atom or group is described as optionally substituted with one or more substituents in the list of substituents, one or more hydrogens on the atom or group can be replaced by an independently selected and optional substituent. When the substituent is oxo (namely =O), it means that two hydrogen atoms are replaced. When a substituent is hydrogen, this may also mean that the corresponding group is "unsubstituted" or "not substituted". Unless otherwise specified, as used herein, the attachment site of a substituent may be from any suitable position of the substituted group. When a bond of a substituent is shown as passing through a bond connecting two atoms in a ring, the substituent may be bonded to any one of the ring-forming atoms in the substitutable ring.

When any variable (e.g., R), as well as labeled variables (e.g., R₁, R₂, R₃, R₄, R₅, R₆, R₇, etc.), occurs more than once in a composition or structure of a compound, the variable is independently defined in each case at each occurrence. For example, if a group is substituted with 0, 1, 2, 3, or 4 R substituents, the group may optionally be substituted with up to four R substituents, and the options for each R substituent in each case are independent of each other.

The compound of the present disclosure may exist in the form of a specific geometric isomer or stereoisomer. All such compounds in the present disclosure include cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in the substituents of the compounds of the present disclosure. All these isomers and mixtures thereof are encompassed within the scope of the present disclosure. In certain embodiments, preferred compounds are those isomer compounds that exhibit superior biological activity. Purified or partially purified isomers and stereoisomers, or racemic or diastereomeric mixtures of the compounds of the present disclosure are also encompassed within the scope of the present disclosure. Purification and separation of such substances can be accomplished by standard techniques known in the art.

The compounds of the present disclosure include all suitable isotopic derivatives of the compounds. The term "isotopic derivative" refers to a compound in which at least one atom is replaced by an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (tritium, T), ¹¹C, ¹³C, ¹⁴C, ¹⁵N, ¹⁷O, ¹⁸O, ³²P, ³³P, ³³S, ³⁴S, ³⁵S, ³⁶S, ¹⁸F , ³⁶Cl, ⁸²Br, ¹²³I, ¹²⁴I, ¹²⁵I, ¹²⁹I, and ¹³¹I, preferably deuterium.

All variations in the isotopic composition of the compounds of the present disclosure, whether radioactive or not, are encompassed within the scope of the present disclosure. Each available hydrogen atom attached to a carbon atom may be independently replaced by a deuterium atom, wherein the replacement of deuterium may be partial or complete, and the replacement of partial deuterium refers to the replacement of at least one hydrogen atom by at least one deuterium atom. In the compounds of the present disclosure, when a position is specifically assigned as deuterium D, the position should be understood as the abundance of deuterium being at least 1000 times greater than the natural abundance (which is 0.015%) (i.e., at least 15% deuterium incorporation). In some embodiments, the abundance of deuterium of each of the assigned deuterium atoms is at least 1000 times greater than the natural abundance of deuterium (i.e., at least 15% deuterium incorporation). In some embodiments, the abundance of deuterium of each of the assigned deuterium atoms is at least 2000 times greater than the natural abundance of deuterium (i.e., at least 30% deuterium incorporation). In some embodiments, the abundance of deuterium of each of the assigned deuterium atoms is at least 3000 times greater than the natural abundance of deuterium (i.e., at least 45% deuterium incorporation). In some embodiments, the abundance of deuterium of each of the assigned deuterium atoms is at least 3340 times greater than the natural abundance of deuterium (i.e., at least 50.1% deuterium incorporation). In some embodiments, the abundance of deuterium of each of the assigned deuterium atoms is at least 3500 times greater than the natural abundance of deuterium (i.e., at least 52.5% deuterium incorporation). In some embodiments, the abundance of deuterium of each of the assigned deuterium atoms is at least 4000 times greater than the natural abundance of deuterium (i.e., at least 60% deuterium incorporation). In some embodiments, the abundance of deuterium of each of the assigned deuterium atoms is at least 4500 times greater than the natural abundance of deuterium (i.e., at least 67.5% deuterium incorporation). In some embodiments, the abundance of deuterium of each of the assigned deuterium atoms is at least 5000 times greater than the natural abundance of deuterium (i.e., at least 75% deuterium incorporation). In some embodiments, the abundance of deuterium of each of the assigned deuterium atoms is at least 5500 times greater than the natural abundance of deuterium (i.e., at least 82.5% deuterium incorporation). In some embodiments, the abundance of deuterium of each of the assigned deuterium atoms is at least 6000 times greater than the natural abundance of deuterium (i.e., at least 90% deuterium incorporation). In some embodiments, the abundance of deuterium of each of the assigned deuterium atoms is at least 6333.3 times greater than the natural abundance of deuterium (i.e., at least 95% deuterium incorporation). In some embodiments, the abundance of deuterium of each of the assigned deuterium atoms is at least 6466.7 times greater than the natural abundance of deuterium (i.e., at least 97% deuterium incorporation). In some embodiments, the abundance of deuterium of each of the assigned deuterium atoms is at least 6600 times greater than the natural abundance of deuterium (i.e., at least 99% deuterium incorporation). In some embodiments, the abundance of deuterium of each of the assigned deuterium atoms is at least 6633.3 times greater than the natural abundance of deuterium (i.e., at least 99.5% deuterium incorporation).

The term "pharmaceutically acceptable" substance refers to those substances which are, within the scope of sound medical judgment, suitable for use in contact with the tissues of patients without undue toxicity, irritation, allergic response, and other problems, commensurate with a reasonable benefit/risk ratio, and effective for their intended use.

The term "pharmaceutically acceptable salt" refers to salts of the compounds of the present disclosure, which are safe and effective and possess the desired biological activities when used in mammals.

The term "pharmaceutical composition" refers to a composition containing one or more of the compounds described in the present disclosure or physiologically/pharmaceutically acceptable salts or prodrugs thereof, and other components such as physiologically/pharmaceutically acceptable carriers. The pharmaceutical composition is intended to promote the administration to an organism, so as to facilitate the absorption of the active ingredient, thereby exerting its biological activity.

The term "pharmaceutically acceptable carrier" refers to those substances that do not have a significant irritating effect on organisms and do not impair the biological activity and properties of the active compound. The term "pharmaceutically acceptable carrier" includes, but is not limited to, a glidant, a sweetener, a diluent, a preservative, a dye/colorant, a flavoring agent, a surfactant, a wetting agent, a dispersant, a disintegrant, a stabilizer, a solvent, or an emulsifier.

The terms "administration" or "administering" and the like refer to methods that enable a compound or composition to be delivered to a desired site of biological action. These methods include, but are not limited to, oral administration or parenteral administration (including intraventricular, intravenous, subcutaneous, intraperitoneal, intramuscular, and intravascular injection or infusion), topical administration, rectal administration, etc., especially injection or oral administration.

As used herein, the term "treat", "treating", or "treatment" includes alleviating, relieving, or ameliorating a disease or symptom, preventing other symptoms, ameliorating or preventing underlying metabolic factors of a symptom, inhibiting a disease or symptom, such as arresting the development of a disease or symptom, relieving a disease or symptom, promoting remission of a disease or symptom, or halting signs of a disease or symptom, and extends to include prevention. The term "treat", "treating", or "treatment" also includes achieving a therapeutic benefit and/or a prophylactic benefit. The therapeutic benefit refers to eradication or amelioration of a condition being treated. In addition, the therapeutic benefit is achieved by eradicating or ameliorating one or more physiological signs associated with an underlying disease, such that amelioration of the underlying disease in the patient can be observed, although the patient may still be afflicted with the underlying disease. The prophylactic benefit refers to the use of a composition by a patient to prevent the risk of a certain disease, or the administration of a composition to a patient when the patient develops one or more physiological conditions of a disease, although the disease has not yet been diagnosed.

The term "active ingredient", "therapeutic agent", "active substance", or "active agent" refers to a chemical entity that is effective in treating or preventing a target disorder, disease, or condition. The term "neuropsychiatric disease" refers to a generic term for neurological diseases and psychiatric diseases, including neurological diseases and/or psychiatric diseases.

For a drug, drug unit, or active ingredient, the term "effective amount", "therapeutically effective amount", or "prophylactically effective amount" refers to an amount of a drug or pharmaceutical agent that is sufficient to provide the desired effect with acceptable side effects. The determination of the effective amount varies from person to person. It depends on the age and general condition of an individual, as well as the specific active substance used. The appropriate effective amount in a case may be determined by those skilled in the art in the light of routine tests.

As used herein, the term "individual" includes a human or non-human animal. An exemplary human individual includes a human individual (referred to as a patient) with a disease (e.g., a disease described herein) or a normal individual. In the present disclosure, the "non-human animal" includes all vertebrates, such as non-mammals (e.g., birds, amphibians, and reptiles) and mammals, such as non-human primates, livestock, and/or domesticated animals (e.g., sheep, dogs, cats, cows, pigs, etc.). The term "room temperature" refers to a temperature of 10-40 °C. In some embodiments, "room temperature" refers to a temperature of 15-30 °C; in other embodiments, "room temperature" refers to a temperature of 18-25 °C.

The term "equivalent" or its abbreviation "eq" refers to the equivalent amount of other starting materials required, based on the stoichiometric equivalent relationship of the chemical reaction and with the basic starting material used in each step as the reference (1 equivalent).

In the context of the present disclosure, "approximately" or "about", when used or whether used or not, refers to within 10% of the given value or range, appropriately within 5%, and particularly within 1%. Alternatively, for those of ordinary skill in the art, the term "approximately" or "about" indicates within the acceptable standard error range of the mean value. Whenever a number with a value of N is disclosed, any number within a value of N±1%, N±2%, N±3%, N±5%, N±7%, N±8%, or N±10% will be explicitly disclosed, where "±" refers to plus or minus.

The following detailed description of the present disclosure is intended to illustrate non-limiting embodiments and to enable others skilled in the art to more fully understand the technical solutions of the present disclosure, their principles, and their practical applications, so that others skilled in the art may modify and implement the present disclosure in various forms to allow it to be optimally adapted to the requirements of particular use.

### Beneficial Effects

The present disclosure relates to a class of novel structural compounds, which are inhibitors acting on Lp-PLA2 and have the effect of preventing and/or treating related diseases mediated by Lp-PLA2. In some embodiments, the compounds of the present disclosure have good affinity for Lp-PLA2 and are capable of effectively inhibiting Lp-PLA2 activity in human plasma. In some embodiments, the compounds of the present disclosure exhibit better metabolic stability (e.g., lower clearance rate and/or longer half-life) in liver microsomes of SD rats, beagle dogs, cynomolgus monkeys, humans, and the like. In some embodiments, the compounds of the present disclosure have superior druggability properties, including improved pharmacokinetic properties (e.g., good plasma concentration and area under the curve, improved bioavailability, suitable half-life, and duration of action), improved *in vivo* pharmacodynamic effects, improved safety (lower toxicity and/or fewer side effects), good patient compliance, and/or less susceptibility to developing tolerance. Specifically, the results of the inhibitory activity against Lp-PLA2 in human plasma in the present disclosure indicate that the compounds of the present disclosure are effective Lp-PLA2 inhibitors with good inhibitory activity. The results of the *in vitro* liver microsome stability experiment indicate that the compounds of the present disclosure have lower clearance rates and longer half-lives in liver microsomes of SD rats, beagle dogs, cynomolgus monkeys, and humans. Based on the pharmacodynamic activity and pharmacological mechanism of the series of compounds, a slow metabolic rate is beneficial for the compounds to exert the pharmacodynamic activity, so the compounds of the present disclosure exhibit a more excellent effect in the aspect of metabolic stability.

### DETAILED DESCRIPTION

The present disclosure will be further illustrated with reference to the following specific examples. It should be understood that these examples are merely intended to illustrate the present disclosure rather than limit the scope of the present disclosure. Furthermore, it should be understood that various changes or modifications of the present disclosure can be made by those skilled in the art after reading the teachings of the present disclosure, and these equivalents also fall within the scope of the appended claims of the present application.

### Examples

Embodiments of the present disclosure will be described in detail below with reference to examples, but those skilled in the art will appreciate that the following examples are only illustrative of the present disclosure and should not be construed as limiting the scope of the present disclosure. Experimental procedures without specified conditions in the examples were conducted according to conventional conditions or conditions recommended by the manufacturers. Reagents or instruments without specified manufacturers used herein are conventional products that are commercially available. The proportions or percentages used herein are calculated by weight, unless otherwise specified.

The compound structure of the present disclosure is determined by nuclear magnetic resonance (NMR) and/or liquid chromatography-mass spectrometry (LC-MS).

The NMR chemical shifts (δ) are given in parts per million (ppm). The NMR determination was conducted by using an AVANCE III600 nuclear magnetic resonance apparatus or Varian Mercury plus 400 MHz, with deuterated dimethyl sulfoxide (DMSO-*d*₆), deuterated methanol (CD₃OD), or deuterated chloroform (CDCl₃) as determination solvents, and tetramethylsilane (TMS) as an internal standard.

Liquid chromatography-mass spectrometry (LC-MS) analysis was performed using a Japan Shimadzu LCMS2020 mass spectrometer or an Agilent LC/MSD 1200 Series quadrupole mass spectrometer.

HPLC analysis was performed by using a Japan Shimadzu LC20A liquid chromatograph.

The Yantai Jiangyou silica gel plate was adopted as a thin layer chromatography silica gel plate. The specification adopted by the TLC was 0.2 mm ± 0.03 mm, and the specification adopted by the thin layer chromatography for product separation and purification was 0.4 mm-0.5 mm.

The intermediate 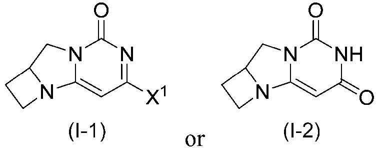 used in the examples is preferably one of the following structures: 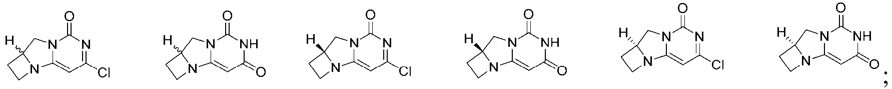 they can be synthesized according to schemes 1-2 below.

The alcohol 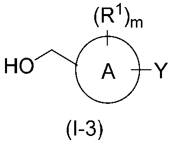 used in the examples is preferably 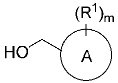 or 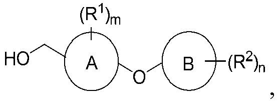 more preferably one of the following structures: 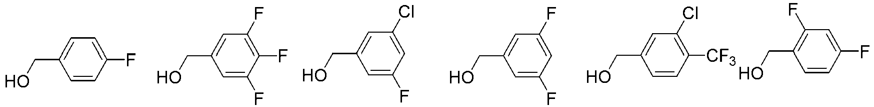 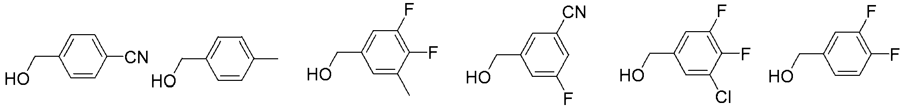 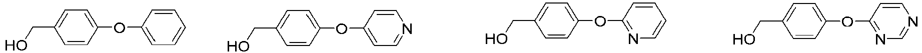 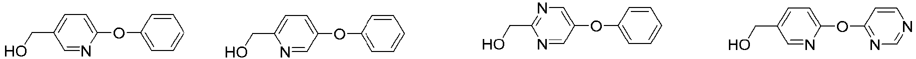 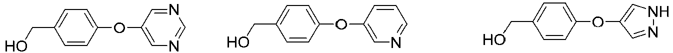 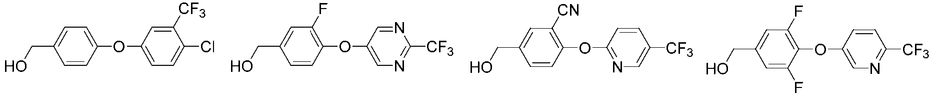 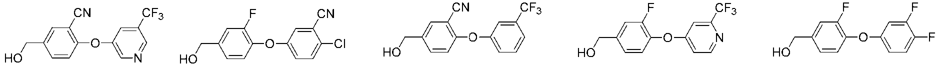 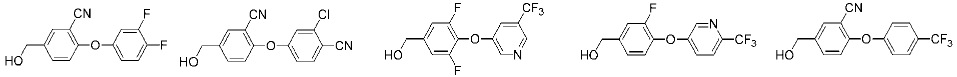 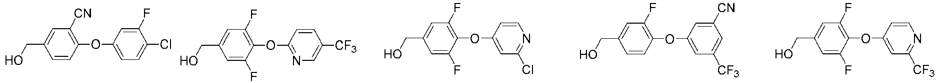 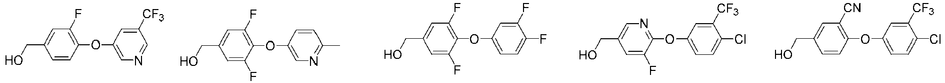 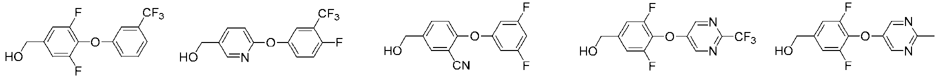 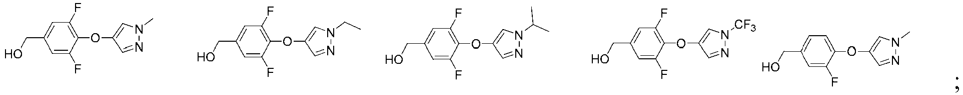 they can be purchased or synthesized according to scheme 3 below, or can be obtained by those skilled in the art through conventional synthesis methods based on the structure of the product according to the synthesis method of the alcohol compound.

The compound represented by general formula (I) described in the present disclosure can be synthesized using representative reactions of schemes 1-5.

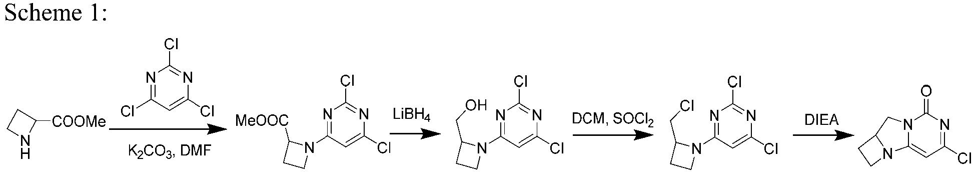

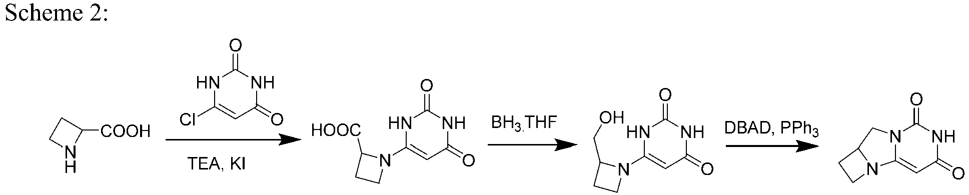

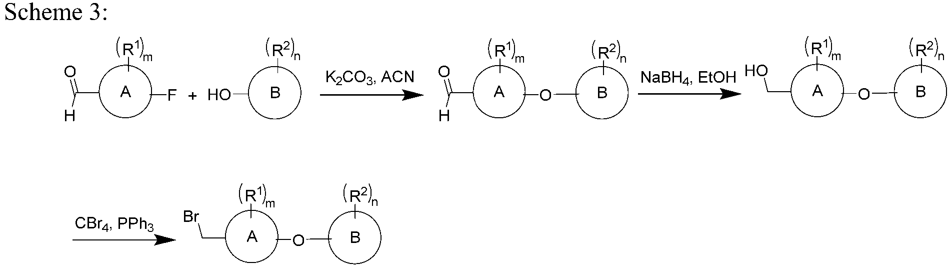

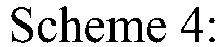

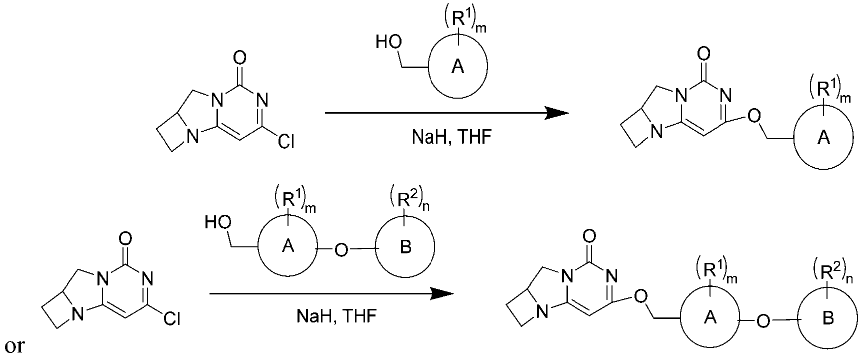

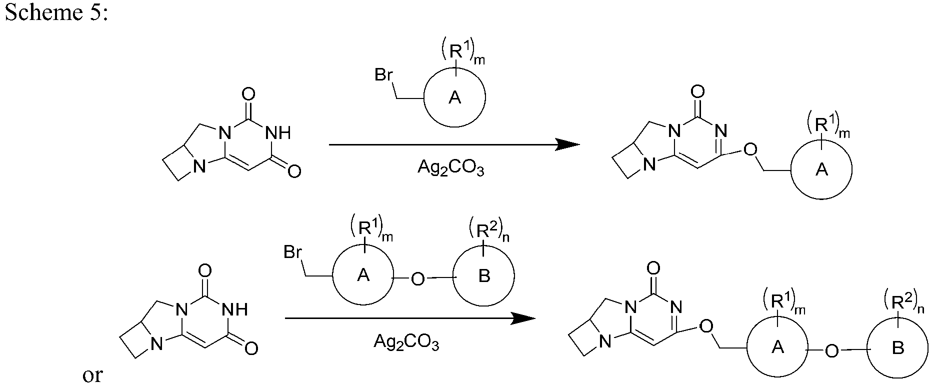

### Intermediate 1

### (R)-6,7,7a,8-Tetrahydro-1H-azeto[1',2':3,4]imidazo[1,2-c]pyrimidine-1,3(2H)-dione

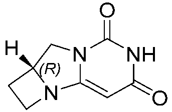

### Synthetic route:

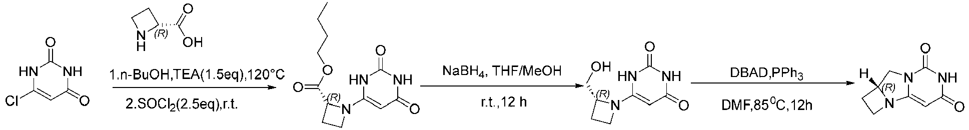

### Step 1: synthesis of butyl (R)-1-(2,6-dioxo-1,2,3,6-tetrahydropyrimidin-4-yl)azetidine-2-carboxylate

6-Chloro-1,3-dihydropyrimidine-2,4-dione (72.4 g, 0.494 mol) and triethylamine (75.0 g, 0.741 mol) were added to a solution of (R)-azetidine-2-carboxylic acid (50.0 g, 0.494 mol) in n-butanol (3 L), and the mixture was stirred at 120 °C for 12 h. The reaction liquid was cooled to 0 °C, and thionyl chloride (147.08 g, 1.236 mol) was added at 0 °C. The mixture was stirred at room temperature for 2 h. The reaction liquid was filtered and concentrated, and water (2 L) was added. The mixture was extracted with DCM (2 L × 3). The extract phases were combined and concentrated to give a crude product, which was separated and purified by column chromatography (DCM:MeOH = 30:1) to give the target product (80 g, yield: 54%).

LC-MS[M+H]⁺:268.1.

### Step 2: synthesis of (R)-6-(2-(hydroxymethyl)azetidin-1-yl)pyrimidine-2,4(1H,3H)-dione

NaBH₄ (33.9 g, 0.897 mol) was added to a solution of butyl (R)-1-(2,6-dioxo-1,2,3,6-tetrahydropyrimidin-4-yl)azetidine-2-carboxylate (80.0 g, 0.299 mol) in THF/MeOH (2000 mL/500 mL) at 0 °C, and the mixture was stirred at 25 °C for 12 h. MeOH (1 L) was added to the reaction liquid, and the mixture was filtered, concentrated, added to water (1 L), and extracted with DCM (1 L × 3). The extract phase was lyophilized to give the target product (150 g, crude). LC-MS[M+H]⁺:198.1.

### Step 3: synthesis of (R)-6,7,7a,8-tetrahydro-1H-azeto[1',2':3,4]imidazo[1,2-c]pyrimidine-1,3(2H)-dione

PPh₃ (266.0 g, 1.014 mol) and DBAD (233.5 g, 1.014 mol) were added to a solution of (R)-6-(2-(hydroxymethyl)azetidin-1-yl)pyrimidine-2,4(1H,3H)-dione (80.0 g, 0.405 mol) in DMF (2000 mL) under a nitrogen atmosphere, and the mixture was stirred at 85 °C for 12 h. The reaction liquid was filtered and concentrated, and the residue was separated and purified by column chromatography (DCM:MeOH = 10:1) to give the target product of intermediate 1 (20 g, yield: 24%).

LC-MS[M+H]⁺:180.1.

¹H NMR (400 MHz, DMSO-*d*₆): δ 10.71 (s, 1H), 4.81 (s, 1H), 4.79-4.72 (m, 1H), 4.38-4.31 (m, 1H), 3.97-3.8 (m, 2H), 3.62-3.56 (m, 1H), 2.84-2.75 (m, 1H), 2.52-2.44 (m, 1H).

### Intermediate 2

### 6,7,7a,8-Tetrahydro-1H-azeto[1',2':3,4]imidazo[1,2-c]pyrimidine-1,3(2H)-dione

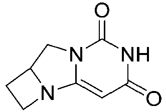

### Synthetic route:

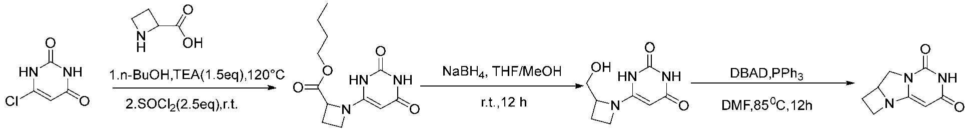

For the preparation method of intermediate 2, reference was made to the preparation method of intermediate 1, and (R)-azetidine-2-carboxylic acid in step 1 was replaced by azetidine-2-carboxylic acid.

LC-MS[M+H]⁺:180.1.

¹H NMR (400 MHz, DMSO-*d*₆): δ 10.71 (s, 1H), 4.81 (s, 1H), 4.79-4.72 (m, 1H), 4.38-4.31 (m, 1H), 3.97-3.8 (m, 2H), 3.62-3.56 (m, 1H), 2.84-2.75 (m, 1H), 2.52-2.44 (m, 1H).

### Intermediate 3

### (S)-6,7,7a,8-Tetrahydro-1H-azeto[1',2':3,4]imidazo[1,2-c]pyrimidine-1,3(2H)-dione

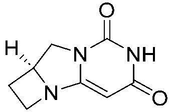

### Synthetic route:

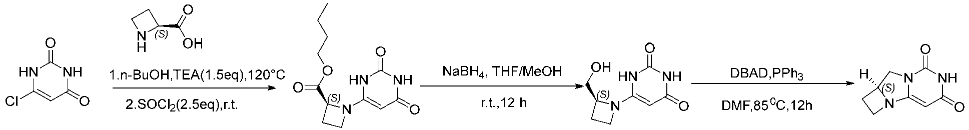

For the preparation method of intermediate 3, reference was made to the preparation method of intermediate 1, and (R)-azetidine-2-carboxylic acid in step 1 was replaced by (S)-azetidine-2-carboxylic acid.

LC-MS[M+H]⁺:180.1.

### Example 1

### 3-((3-Fluoro-4-((6-(trifluoromethyl)pyridin-3-yl)oxy)benzyl)oxy-6,7,7a,8-tetrahydro-1H-azeto[1',2':3,4]imidazo[1,2-c]pyrimidin-1-one

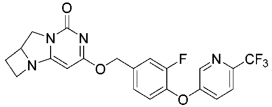

### Synthetic route:

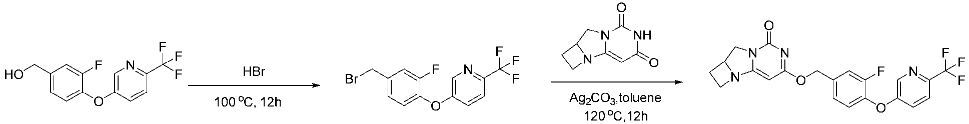

### Step 1: synthesis of 5-(4-(bromomethyl)-2-fluorophenoxy)-2-(trifluoromethyl)pyridine

An aqueous solution of (3-fluoro-4-((6-(trifluoromethyl)pyridin-3-yl)oxy)phenyl)methanol (250 mg, 0.870 mmol) in 48% HBr (5 mL) was stirred at 100 °C for 12 h. The reaction liquid was cooled, and saturated NaHCO₃ (aqueous solution) (50 mL) was added. The mixture was extracted with EA (50 mL × 2). The organic phases were combined, washed with brine, dried over Na₂SO₄, filtered, and concentrated to give a crude product (260 mg, yield: 76%), which was directly used in the next step without purification.

LC-MS[M+H]⁺:350.0.

### Step 2: synthesis of 3-((3-fluoro-4-((6-(trifluoromethyl)pyridin-3-yl)oxy)benzyl)oxy-6,7,7a,8-tetrahydro-1H-azeto[1',2':3,4]imidazo[1,2-c]pyrimidin-1-one

A solution of 6,7,7a,8-tetrahydro-1H-azeto[1',2':3,4]imidazo[1,2-c]pyrimidine-1,3(2H)-dione (100 mg, 0.558 mmol) and Ag₂CO₃ (384.7 mg, 1.395 mmol) in toluene (10 mL) was stirred at 110 °C for 1 h. The reaction liquid was cooled to 70 °C. 5-(4-(Bromomethyl)-2-fluorophenoxy)-2-(trifluoromethyl)pyridine (214.9 mg, 0.613 mmol) was added to the reaction liquid. The reaction liquid was cooled, filtered, and concentrated, and the residue was separated and purified by column chromatography (EA:MeOH = 10:1) to give a crude product (100 mg), which was purified by HPLC to give the target product (24.1 mg, yield: 9.6%).

LC-MS[M+H]⁺:449.1.

¹H NMR (400 MHz, DMSO-*d*₆) δ 8.61 (d, *J* = 2.7 Hz, 1H), 7.90 (d, *J* = 8.7 Hz, 1H), 7.55 (dd, *J* = 7.8, 5.2 Hz, 2H), 7.43 (t, *J* = 8.2 Hz, 1H), 7.36 (d, *J* = 8.5 Hz, 1H), 5.47 (s, 1H), 5.38-5.29 (m, 2H), 4.88-4.76 (m, 1H), 4.39 (dd, *J =* 17.0, 9.0 Hz, 1H), 4.05-3.95 (m, 2H), 3.65 (td, *J =* 9.5, 3.7 Hz, 1H), 2.87-2.74 (m, 1H), 2.47 (d, *J* = 7.2 Hz, 1H).

### Example 2

### 3-((3,4,5-Trifluorobenzyl)oxy)-6,7,7a,8-tetrahydro-1H-azeto[1',2':3,4]imidazo[1,2-c]pyrimidin-1-one

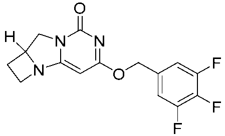

### Synthetic route:

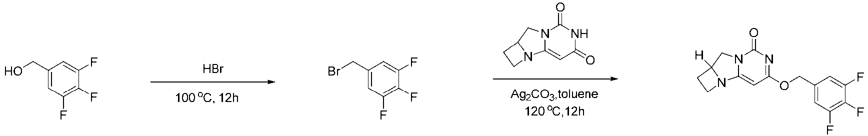

LC-MS[M+H]⁺:324.1.

### Example 3

### 3-((3-Chloro-5-fluorobenzyl)oxy)-6,7,7a,8-tetrahydro-1H-azeto[1',2':3,4]imidazo[1,2-c]pyrimidin-1-one

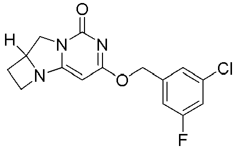

### Synthetic route:

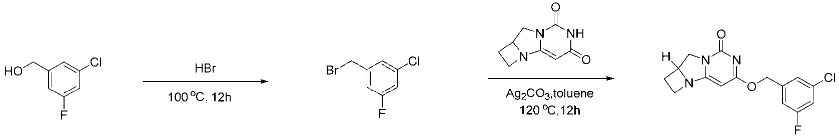

LC-MS[M+H]⁺:322.1.

### Example 4

### 3-((3,5-Difluorobenzyl)oxy)-6,7,7a,8-tetrahydro-1H-azeto[1',2':3,4]imidazo[1,2-c]pyrimidin-1-one

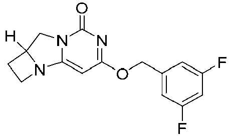

### Synthetic route:

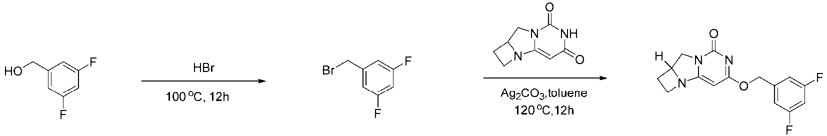

LC-MS[M+H]⁺:306.1.

### Example 5

### 3-((3-Chloro-4-(trifluoromethyl)benzyl)oxy)-6,7,7a,8-tetrahydro-1H-azeto[1',2':3,4]imidazo[1,2-c]pyrimidin-1-one

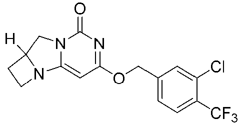

### Synthetic route:

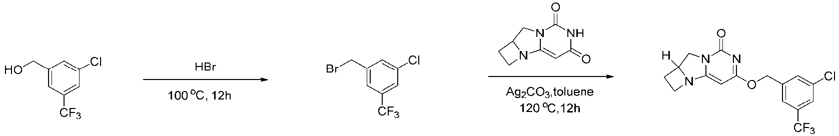

LC-MS[M+H]⁺:372.1.

### Example 6

### 3-((2,4-Difluorobenzyl)oxy)-6,7,7a,8-tetrahydro-1H-azeto[1',2':3,4]imidazo[1,2-c]pyrimidin-1-one

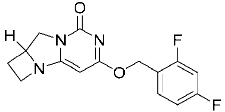

### Synthetic route:

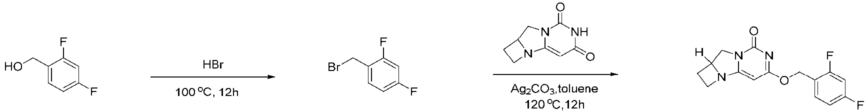

LC-MS[M+H]⁺:306.1.

### Example 7

### 3-((4-Fluorobenzyl)oxy)-6,7,7a,8-tetrahydro-1H-azeto[1',2':3,4]imidazo[1,2-c]pyrimidin-1-one

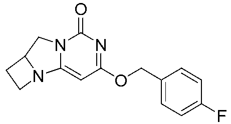

### Synthetic route:

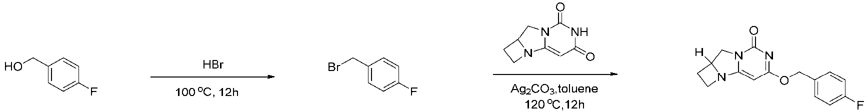

LC-MS[M+H]⁺:288.1.

¹H NMR (400 MHz, DMSO-*d*₆) δ 7.47 (dd, *J* = 8.6, 5.6 Hz, 2H), 7.26-7.16 (m, 2H), 5.42 (s, 1H), *5.27 (q, J* = 12.3 Hz, 2H), 4.82 (qd, *J =* 7.5, 4.7 Hz, 1H), 4.37 (dd, *J* = 16.9, 9.2 Hz, 1H), 4.02-3.94 (m, 2H), 3.63 (td, *J =* 9.5, 3.7 Hz, 1H), 2.79 (dq, *J* = 17.8, 9.6 Hz, 1H), 2.48-2.45 (m, *J* = 7.3, 3.9 Hz, 1H).

### Example 8

### 3-((4-Methylbenzyl)oxy)-6,7,7a,8-tetrahydro-1H-azeto[1',2':3,4]imidazo[1,2-c]pyrimidin-1-one

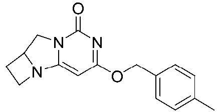

### Synthetic route:

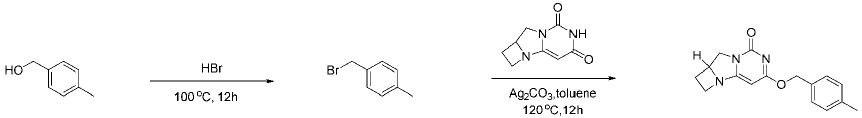

LC-MS[M+H]⁺:284.1.

¹H NMR (400 MHz, DMSO-*d*₆) δ 7.30 (d, *J* = 8.0 Hz, 2H), 7.18 (d, *J* = 7.8 Hz, 2H), 5.40 (s, 1H), 5.24 (q, *J* = 12.2 Hz, 2H), 4.82 (qd, *J* = 7.4, 4.5 Hz, 1H), 4.37 (dd, *J* = 16.9, 9.2 Hz, 1H), 4.09-3.91 (m, 2H), 3.63 (td, *J* = 9.5, 3.9 Hz, 1H), 2.79 (dq, *J* = 17.8, 9.5 Hz, 1H), 2.48-2.39 (m, 1H), 2.30 (s, 3H).

### Example 9

### 3-((3,4-Difluoro-5-methylbenzyl)oxy)-6,7,7a,8-tetrahydro-1H-azeto[1',2':3,4]imidazo[1,2-c]pyrimidin-1-one

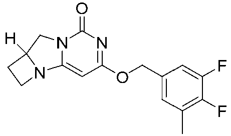

### Synthetic route:

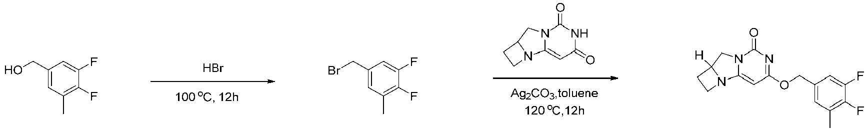

LC-MS[M+H]⁺:320.1.

### Example 10

### 3-((3-Cyano-5-fluorobenzyl)oxy)-6,7,7a,8-tetrahydro-1H-azeto[1',2':3,4]imidazo[1,2-c]pyrimidin-1-one

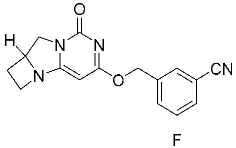

### Synthetic route:

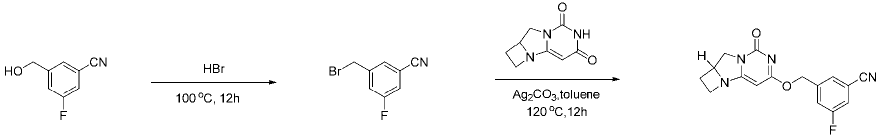

LC-MS[M+H]⁺:313.1.

### Example 11

### 3-((3,4-Difluoro-5-chlorobenzyl)oxy)-6,7,7a,8-tetrahydro-1H-azeto[1',2':3,4]imidazo[1,2-c]pyrimidin-1-one

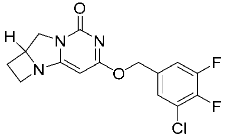

### Synthetic route:

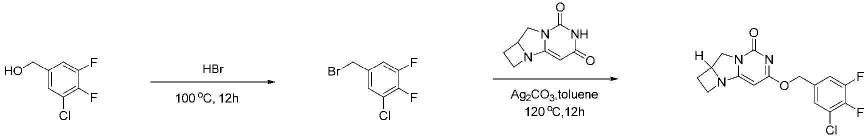

LC-MS[M+H]⁺:340.1.

### Example 12

### 3-((4-Phenoxybenzyl)oxy)-6,7,7a,8-tetrahydro-1H-azeto[1',2':3,4]imidazo[1,2-c]pyrimidin-1-one

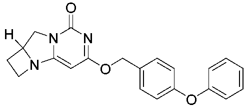

### Synthetic route:

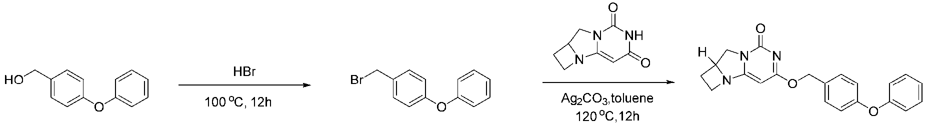

LC-MS[M+H]⁺:362.1.

¹H NMR (400 MHz, DMSO-*d*₆) δ 7.49-7.35 (m, 4H), 7.15 (t, *J* = 7.4 Hz, 1H), 7.06-6.97 (m, 4H), 5.41 (s, 1H), 5.27 (q, *J* = 12.2 Hz, 2H), 4.89-4.76 (m, 1H), 4.38 (dd, *J* = 17.0, 9.2 Hz, 1H), 4.07-3.92 (m, 2H), 3.63 (td, *J* = 9.5, 3.7 Hz, 1H), 2.88-2.72 (m, 1H), 2.47-2.41 (m, 1H).

### Example 13

### 3-((4-(Pyridin-4-yloxy)benzyl)oxy)-6,7,7a,8-tetrahydro-1H-azeto[1',2':3,4]imidazo[1,2-c]pyrimidin-1-one

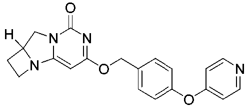

### Synthetic route:

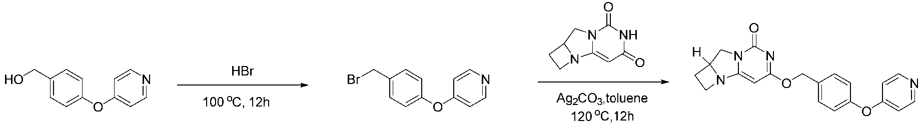

LC-MS[M+H]⁺:363.1.

### Example 14

### 3-((4-(Pyridin-2-yloxy)benzyl)oxy)-6,7,7a,8-tetrahydro-1H-azeto[1',2':3,4]imidazo[1,2-c]pyrimidin-1-one

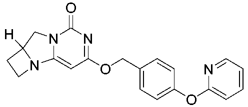

### Synthetic route:

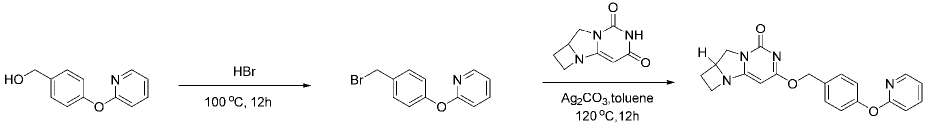

LC-MS[M+H]⁺:363.1.

### Example 15

### 3-((4-(Pyrimidin-4-yloxy)benzyl)oxy)-6,7,7a,8-tetrahydro-1H-azeto[1',2':3,4]imidazo[1,2-c]pyrimidin-1-one

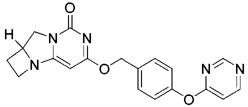

### Synthetic route:

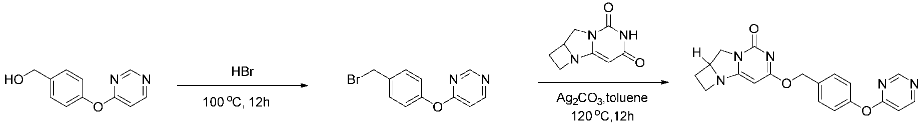

LC-MS[M+H]⁺:364.1.

### Example 16

### 3-((6-Phenoxypyridin-3-yl)methoxy)-6,7,7a,8-tetrahydro-1H-azeto[1',2':3,4]imidazo[1,2-c]pyrimidin-1-one

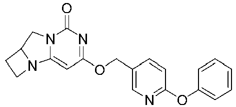

### Synthetic route:

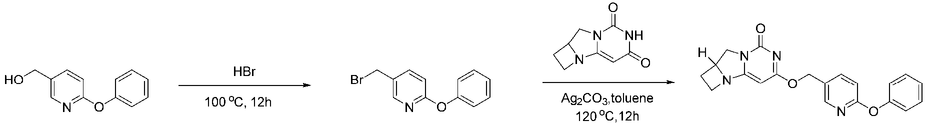

LC-MS[M+H]⁺:363.1.

¹H NMR (400 MHz, DMSO-*d*₆) δ 8.22 (d, *J* = 2.2 Hz, 1H), 7.92 (dd, *J* = 8.4, 2.4 Hz, 1H), 7.42 (t, *J* = 7.9 Hz, 2H), 7.22 (t, *J* = 7.4 Hz, 1H), 7.13 (d, *J =* 8.2 Hz, 2H), 7.04 (d, *J =* 8.4 Hz, 1H), 5.41 (s, 1H), 5.27 (q, *J* = 12.3 Hz, 2H), 4.82 (qd, *J* = 7.4, 4.6 Hz, 1H), 4.37 (dd, *J* = 16.9, 9.1 Hz, 1H), 4.07-3.93 (m, 2H), 3.63 (td, *J* =9.4, 3.6 Hz, 1H), 2.86-2.71 (m, 1H), 2.47-2.43 (m, 1H).

### Example 17

### 3-((5-Phenoxypyridin-2-yl)methoxy)-6,7,7a,8-tetrahydro-1H-azeto[1',2':3,4]imidazo[1,2-c]pyrimidin-1-one

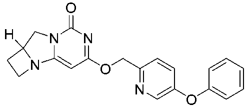

### Synthetic route:

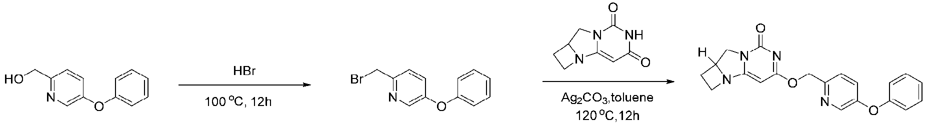

LC-MS[M+H]⁺:363.1.

### Example 18

### 3-((5-Phenoxypyrimidin-2-yl)methoxy)-6,7,7a,8-tetrahydro-1H-azeto[1',2':3,4]imidazo[1,2-c]pyrimidin-1-one

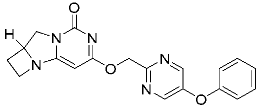

### Synthetic route:

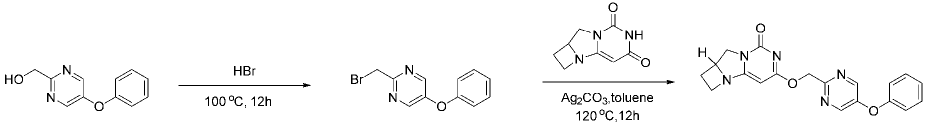

LC-MS[M+H]⁺:364.1.

### Example 19

### 3-((6-(Pyrimidin-4-yloxy)pyridin-3-yl)methoxy)-6,7,7a,8-tetrahydro-1H-azeto[1',2':3,4]imidazo[1,2-c]pyrimidin-1-one

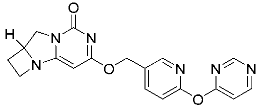

### Synthetic route:

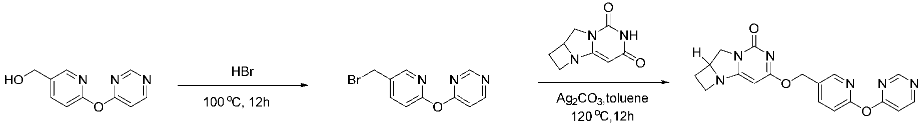

LC-MS[M+H]⁺:365.1.

### Example 20

### 5-(((1-Oxo-6,7,7a,8-tetrahydro-1H-azeto[1',2':3,4]imidazo[1,2-c]pyrimidin-3-yl)oxy)methyl)-2-((5-(trifluoromethyl)pyridin-3-yl)oxy)benzonitrile

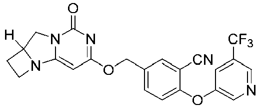

### Synthetic route:

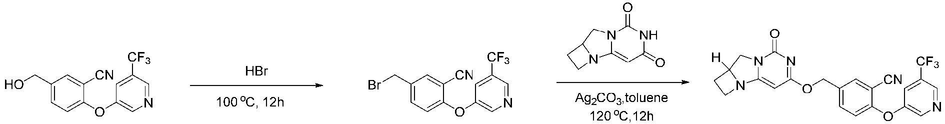

LC-MS[M+H]⁺:456.1.

### Example 21

### 5-(((1-Oxo-6,7,7a,8-tetrahydro-1H-azeto[1',2':3,4]imidazo[1,2-c]pyrimidin-3-yl)oxy)methyl)-2-((5-(trifluoromethyl)pyridin-2-yl)oxy)benzonitrile

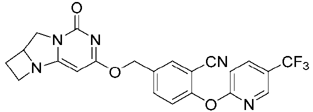

### Synthetic route:

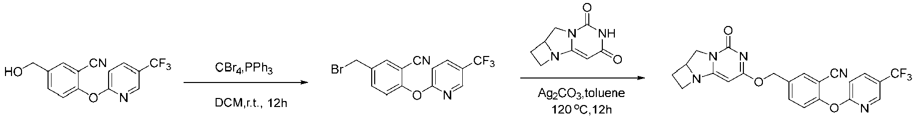

### Step 1: synthesis of 5-(bromomethyl)-2-((5-(trifluoromethyl)pyridin-2-yl)oxy)benzonitrile

CBr₄ (225.4 mg, 0.679 mmol) and PPh₃ (178.2 mg, 0.679 mmol) were added to a solution of 5-(hydroxymethyl)-2-((5-(trifluoromethyl)pyridin-2-yl)oxy)benzonitrile (200 mg, 0.679 mmol) in DCM (5 mL). The reaction liquid was stirred at 25 °C for 12 h, and the reaction was quenched with water (20 mL). The mixture was extracted with DCM (20 mL × 2). The combined organic phases were dried over Na₂SO₄, filtered, and concentrated, and the residue was separated and purified by column chromatography (PE:EA = 3:1) to give the target product (230 mg, yield: 95.04%). LC-MS[M+H]⁺:357.

### Step 2: synthesis of 5-(((1-oxo-6,7,7a,8-tetrahydro-1H-azeto[1',2':3,4]imidazo[1,2-c]pyrimidin-3-yl)oxy)methyl)-2-((5-(trifluoromethyl)pyridin-2-yl)oxy)benzonitrile

A solution of 6,7,7a,8-tetrahydro-1H-azeto[1',2':3,4]imidazo[1,2-c]pyrimidine-1,3(2H)-dione (90 mg, 0.502 mmol) and Ag₂CO₃ (346.2 mg, 1.255 mmol) in toluene (10 mL) was stirred at 110 °C for 1 h. The reaction liquid was cooled to 70 °C. 5-(Bromomethyl)-2-((5-(trifluoromethyl)pyridin-2-yl)oxy)benzonitrile (197.3 mg, 0.552 mmol) was added to the reaction liquid. The reaction liquid was stirred at 120 °C for another 12 h, cooled, filtered, and concentrated, and the residue was separated and purified by column chromatography (EA:MeOH = 10:1) to give a crude product (100 mg), which was purified by HPLC to give the target product (24.6 mg, yield: 10.6%).

LC-MS[M+H]⁺:456.1.

¹H NMR (400 MHz, DMSO-*d*₆) δ 8.49 (s, 1H), 8.10 (s, 1H), 7.94 (d, *J =* 8.4 Hz, 1H), 7.85 (dd, *J* = 9.7, 2.6 Hz, 1H), 7.78 (d, *J =* 8.2 Hz, 1H), 6.73 (d, *J* = 9.7 Hz, 1H), 5.50 (s, 1H), 5.48-5.36 (m, 2H), 4.89-4.77 (m, 1H), 4.39 (dd, *J* = 16.9, 9.1 Hz, 1H), 4.05-3.95 (m, 2H), 3.66 (td, *J =* 9.5, 3.7 Hz, 1H), 2.81 (dt, *J =* 18.8, 9.6 Hz, 1H), 2.50-2.46 (m, 1H).

### Example 22

### 5-(((1-Oxo-6,7,7a,8-tetrahydro-1H-azeto[1',2':3,4]imidazo[1,2-c]pyrimidin-3-yl)oxy)methyl)-2-(3-(trifluoromethyl)phenoxy)benzonitrile

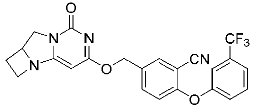

### Synthetic route:

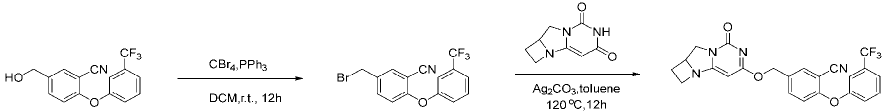

LC-MS[M+H]⁺:455.1.

¹H NMR (400 MHz, DMSO-*d*₆) δ 8.00 (d, *J* = 2.0 Hz, 1H), 7.81-7.67 (m, 2H), 7.64 (d, *J* = 7.9 Hz, 1H), 7.58 (s, 1H), 7.48 (d, *J* = 8.0 Hz, 1H), 7.11 (d, *J* = 8.7 Hz, 1H), 5.45 (s, 1H), 5.39-5.24 (m, 2H), 4.90-4.74 (m, 1H), 4.39 (dd, *J* = 16.9, 9.1 Hz, 1H), 4.07-3.92 (m, 2H), 3.64 (td, *J = 9.5,* 3.7 Hz, 1H), 2.80 (dt, *J =* 18.9, 9.6 Hz, 1H), 2.50-2.47 (m, 1H).

### Example 23

### 3-((3-Fluoro-4-((2-(trifluoromethyl)pyridin-4-yl)oxy)benzyl)oxy-6,7,7a,8-tetrahydro-1H-azeto[1',2':3,4]imidazo[1,2-c]pyrimidin-1-one

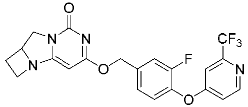

### Synthetic route:

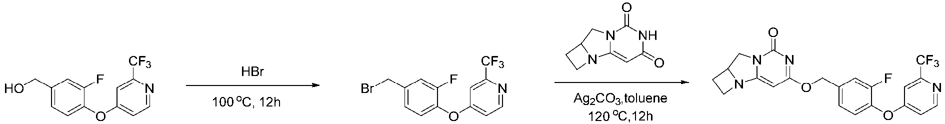

LC-MS[M+H]⁺:449.1.

¹H NMR (400 MHz, DMSO-*d*₆) δ 8.66 (d, *J* = 5.7 Hz, 1H), 7.64-7.44 (m, 3H), 7.40 (d, *J* = 8.4 Hz, 1H), 7.20 (dd, *J* = 5.6, 2.3 Hz, 1H), 5.48 (s, 1H), 5.41-5.29 (m, 2H), 4.93-4.75 (m, 1H), 4.40 (dd, *J* = 16.9, 9.1 Hz, 1H), 4.07-3.94 (m, 2H), 3.65 (td, *J* = 9.5, 3.7 Hz, 1H), 2.81 (dt, *J* = 19.0, 9.5 Hz, 1H), 2.48-2.44 (m, 1H).

### Example 24

### 3-((4-(3,4-Difluorophenoxy)-3-fluorobenzyl)oxy)-6,7,7a,8-tetrahydro-1H-azeto[1',2':3,4]imidazo[1,2-c]pyrimidin-1-one

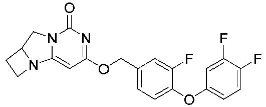

### Synthetic route:

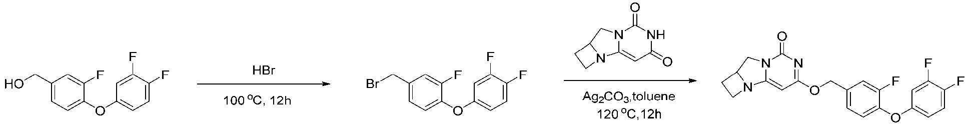

LC-MS[M+H]⁺:416.1.

¹H NMR (400 MHz, DMSO-*d*₆) δ 7.46 (ddd, *J* = 21.8, 13.0, 5.4 Hz, 2H), 7.24 (ddd, *J* = 13.5, 11.8, 5.8 Hz, 3H), 6.87-6.78 (m, 1H), 5.45 (s, 1H), 5.38-5.19 (m, 2H), 4.83 (tt, *J =* 12.1, 6.1 Hz, 1H), 4.39 (dd, *J* = 16.9, 9.1 Hz, 1H), 4.07-3.94 (m, 2H), 3.64 (td, *J* = 9.6, 3.8 Hz, 1H), 2.87-2.71 (m, 1H), 2.48-2.43 (m, 1H).

### Example 25

### 2-(3,4-Difluorophenoxy)-5-(((1-oxo-6,7,7a,8-tetrahydro-1H-azeto[1',2':3,4]imidazo[1,2-c]pyrimidin-3-yl)oxy)methyl)benzonitrile

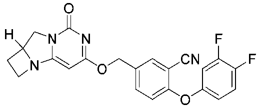

### Synthetic route:

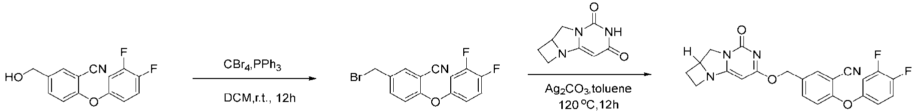

LC-MS[M+H]⁺:423.1.

### Example 26

### 2-Chloro-4-(2-cyano-4-(((1-oxo-6,7,7a,8-tetrahydro-1H-azeto[1',2':3,4]imidazo[1,2-c]pyrimidin-3-yl)oxy)methyl)phenoxy)benzonitrile

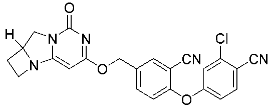

### Synthetic route:

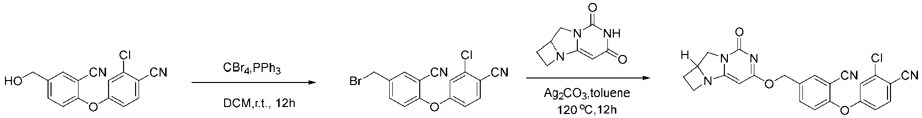

LC-MS[M+H]⁺:446.1.

### Example 27

### 3-((3,5-Difluoro-4-((5-(trifluoromethyl)pyridin-3-yl)oxy)benzyl)oxy-6,7,7a,8-tetrahydro-1H-azeto[1',2':3,4]imidazo[1,2-c]pyrimidin-1-one

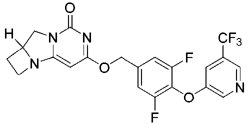

### Synthetic route:

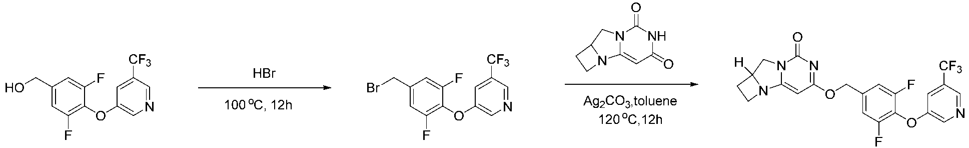

LC-MS[M+H]⁺:467.1.

### Example 28

### 3-((4-Cyanobenzyl)oxy)-6,7,7a,8-tetrahydro-1H-azeto[1',2':3,4]imidazo[1,2-c]pyrimidin-1-one

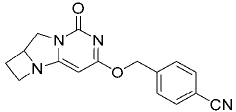

### Synthetic route:

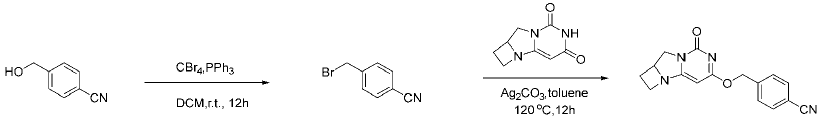

LC-MS[M+H]⁺: 295.1.

¹H NMR (400 MHz, DMSO-*d*₆) δ 7.86 (d, *J* = 8.2 Hz, 2H), 7.59 (d, *J* = 8.2 Hz, 2H), 5.48 (s, 1H), 5.46-5.32 (m, 2H), 4.83 (qd, *J* = 7.5, 4.8 Hz, 1H), 4.39 (dd, *J* = 16.9, 9.2 Hz, 1H), 4.05-3.93 (m, 2H), 3.65 (td, *J* = 9.5, 3.8 Hz, 1H), 2.88-2.72 (m, 1H), 2.48-2.42 (m, 1H).

### Example 29

### 5-(((1-Oxo-6,7,7a,8-tetrahydro-1H-azeto[1',2':3,4]imidazo[1,2-c]pyrimidin-3-yl)oxy)methyl)-2-(4-(trifluoromethyl)phenoxy)benzonitrile

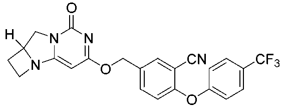

### Synthetic route:

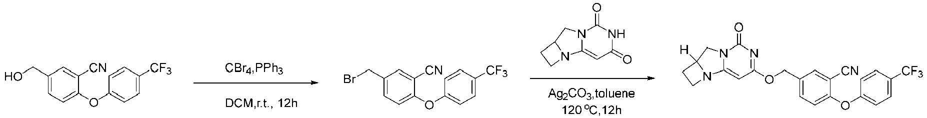

LC-MS[M+H]⁺: 455.1.

### Example 30

### 2-(4-Chloro-3-fluorophenoxy)-5-(((1-oxo-6,7,7a,8-tetrahydro-1H-azeto[1',2':3,4]imidazo[1,2-c]pyrimidin-3-yl)oxy)methyl)benzonitrile

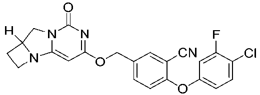

### Synthetic route:

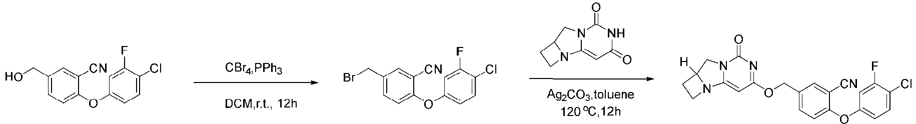

LC-MS[M+H]⁺:439.1.

### Example 31

### 3-((3,5-Difluoro-4-((5-(trifluoromethyl)pyridin-2-yl)oxy)benzyl)oxy-6,7,7a,8-tetrahydro-1H-azeto [1',2':3,4]imidazo [1,2-c]pyrimidin-1-one

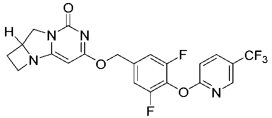

### Synthetic route:

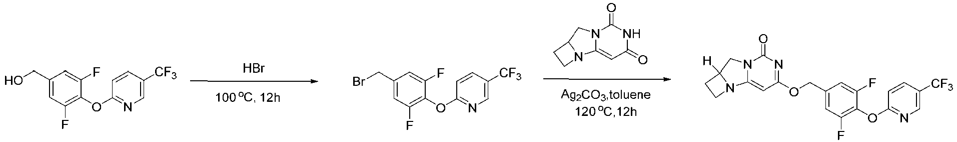

LC-MS[M+H]⁺:467.1.

### Example 32

### 3-((4-((2-Chloropyridin-4-yl)oxy)-3,5-difluorobenzyl)oxy-6,7,7a,8-tetrahydro-1H-azeto[1',2':3,4]imidazo[1,2-c]pyrimidin-1-one

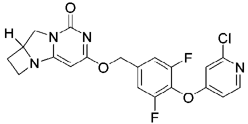

### Synthetic route:

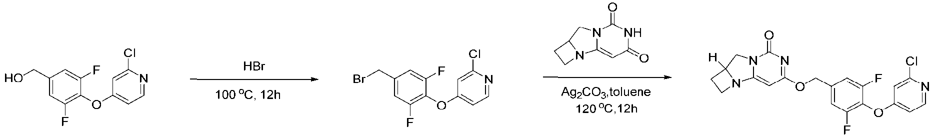

LC-MS[M+H]⁺:433.1.

### Example 33

### 3-(2-Fluoro-4-(((1-0x0-6,7,7a,8-tetrahydro-1H-azeto[1',2':3,4]imidazo[1,2-c]pyrimidin-3-yl)oxy)methyl)phenoxy)-5-(trifluoromethyl)benzonitrile

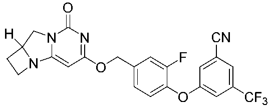

### Synthetic route:

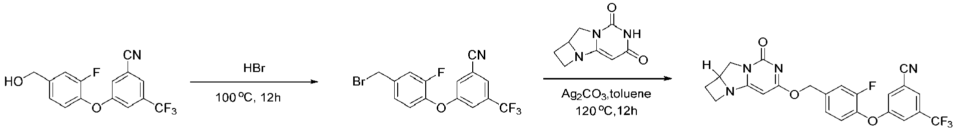

LC-MS[M+H]⁺:473.1.

### Example 34

### 3-((3,5-Difluoro-4-((2-(trifluoromethyl)pyridin-4-yl)oxy)benzyl)oxy-6,7,7a,8-tetrahydro-1H-azeto[1',2':3,4]imidazo[1,2-c]pyrimidin-1-one

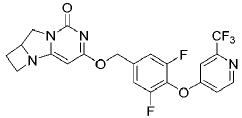

### Synthetic route:

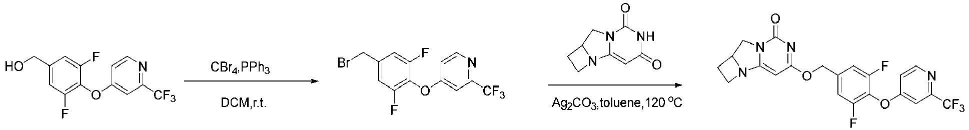

### Step 1: synthesis of 4-(4-(bromomethyl)-2,6-difluorophenoxy)-2-(trifluoromethyl)pyridine

CBr₄ (12.2 g, 0.038 mol) and PPh₃ (9.7 g, 0.033 mol) were added to a solution of (3,5-difluoro-4-((2-(trifluoromethyl)pyridin-4-yl)oxy)phenyl)methanol (11.3 g, 0.037 mol) in DCM (200 mL). The reaction liquid was stirred at 25 °C for 12 h, and the reaction was quenched by adding water (50 mL). The mixture was extracted with DCM (50 mL × 2). The combined organic phases were dried over Na₂SO₄, filtered, and concentrated. The residue was purified by flash column chromatography on silica gel (PE:EA = 33:1) to give the target product (6.5 g, yield: 42%).

LC-MS[M+H]⁺:368.0.

### Step 2: synthesis of 3-((3,5-difluoro-4-((2-(trifluoromethyl)pyridin-4-yl)oxy)benzyl)oxy-6,7,7a,8-tetrahydro-1H-azeto[1',2':3,4]imidazo[1,2-c]pyrimidin-1-one

A solution of 6,7,7a,8-tetrahydro-1H-azeto[1',2':3,4]imidazo[1,2-c]pyrimidine-1,3(2H)-dione (2.7 g, 0.015 mol) and Ag₂CO₃ (10.41 g, 0.038 mol) in toluene (150 mL) was stirred at 110 °C for 1 h. Then the reaction liquid was cooled to 70 °C, and 4-(4-(bromomethyl)-2,6-difluorophenoxy)-2-(trifluoromethyl)pyridine (6.1 g, 0.016 mol) was added to the reaction liquid. The reaction liquid was cooled to room temperature, filtered, and concentrated, and the residue was separated and purified by column chromatography (EA:MeOH = 10:1) to give a crude product (3 g), which was purified by HPLC to give the target product.

LC-MS[M+H]⁺:467.1.

¹H NMR (400 MHz, DMSO-*d*₆) δ 8.68 (d, *J* = 5.6 Hz, 1H), 7.67 (d, *J* = 2.3 Hz, 1H), 7.48 (d, *J* = 8.9 Hz, 2H), 7.32 (dd, *J =* 5.5, 2.2 Hz, 1H), 5.51 (s, 1H), 5.44-5.28 (m, 2H), 4.91-4.76 (m, 1H), 4.40 (dd, *J* = 16.9, 9.1 Hz, 1H), 4.09-3.94 (m, 2H), 3.66 (td, *J* = 9.4, 3.6 Hz, 1H), 2.81 (dt, *J* = 19.0, 9.7 Hz, 1H), 2.50-2.46 (m, 1H).

### Example 34-R

### (R)-3-((3,5-Difluoro-4-((2-(trifluoromethyl)pyridin-4-yl)oxy)benzyl)oxy-6,7,7a,8-tetrahydro-1H-azeto[1',2':3,4]imidazo[1,2-c]pyrimidin-1-one

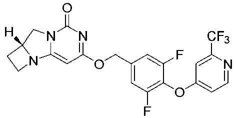

### Synthetic route:

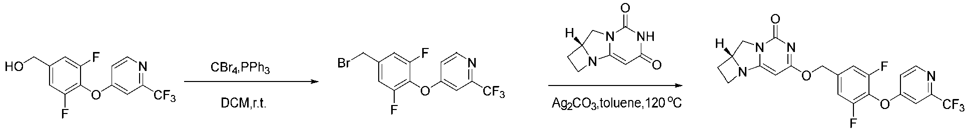

### Step 1: synthesis of 4-(4-(bromomethyl)-2,6-difluorophenoxy)-2-(trifluoromethyl)pyridine

CBr₄ (12.2 g, 0.038 mol) and PPh₃ (9.7 g, 0.033 mol) were added to a solution of (3,5-difluoro-4-((2-(trifluoromethyl)pyridin-4-yl)oxy)phenyl)methanol (11.3 g, 0.037 mol) in DCM (200 mL). The reaction liquid was stirred at 25 °C for 12 h, and the reaction was quenched by adding water (50 mL). The mixture was extracted with DCM (50 mL × 2). The combined organic phases were dried over Na₂SO₄, filtered, and concentrated. The residue was purified by flash column chromatography on silica gel (PE:EA = 33:1) to give the target product (6.5 g, yield: 42%).

LC-MS[M+H]⁺:368.0.

### Step 2: synthesis of (R)-3-((3,5-difluoro-4-((2-(trifluoromethyl)pyridin-4-yl)oxy)benzyl)oxy-6,7,7a,8-tetrahydro-1H-azeto[1',2':3,4]imidazo[1,2-c]pyrimidin-1-one

A solution of (R)-6,7,7a,8-tetrahydro-1H-azeto[1',2':3,4]imidazo[1,2-c]pyrimidine-1,3(2H)-dione (2.7 g, 0.015 mol) and Ag₂CO₃ (10.41 g, 0.038 mol) in toluene (150 mL) was stirred at 110 °C for 1 h. Then the reaction liquid was cooled to 70 °C, and 4-(4-(bromomethyl)-2,6-difluorophenoxy)-2-(trifluoromethyl)pyridine (6.1 g, 0.016 mol) was added to the reaction liquid. The reaction liquid was cooled to room temperature, filtered, and concentrated, and the residue was separated and purified by column chromatography (EA:MeOH = 10:1) to give a crude product (3 g), which was purified by HPLC to give the target product (2.02 g, yield: 28%).

LC-MS[M+H]⁺:467.1.

¹H NMR (400 MHz, DMSO-*d*₆) δ 8.68 (d, *J* = 5.6 Hz, 1H), 7.67 (d, *J* = 2.3 Hz, 1H), 7.48 (d, *J* = 8.9 Hz, 2H), 7.32 (dd, *J = 5.5,* 2.2 Hz, 1H), 5.51 (s, 1H), 5.44-5.28 (m, 2H), 4.91-4.76 (m, 1H), 4.40 (dd, *J* = 16.9, 9.1 Hz, 1H), 4.09-3.94 (m, 2H), 3.66 (td, *J* = 9.4, 3.6 Hz, 1H), 2.81 (dt, *J* = 19.0, 9.7 Hz, 1H), 2.50-2.46 (m, 1H).

### Example 34-S

### (S)-3-((3,5-Difluoro-4-((2-(trifluoromethyl)pyridin-4-yl)oxy)benzyl)oxy-6,7,7a,8-tetrahydro-1H-azeto[1',2':3,4]imidazo[1,2-c]pyrimidin-1-one

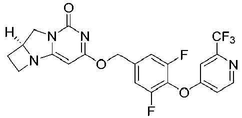

### Synthetic route:

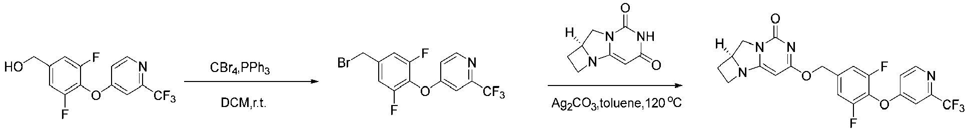

For the synthetic route, reference was made to Example 34-R, and (R)-6,7,7a,8-tetrahydro-1H-azeto[1',2':3,4]imidazo[1,2-c]pyrimidine-1,3(2H)-dione in step 2 was replaced by (S)-6,7,7a,8-tetrahydro-1H-azeto[1',2':3,4]imidazo[1,2-c]pyrimidine-1,3(2H)-dione.

LC-MS[M+H]⁺:467.1.

¹H NMR (400 MHz, CDCl₃): δ 8.60 (d, *J* = 5.6 Hz, 1H), 7.26 (s, 1H), 7.15 (d, *J* = 5.2 Hz, 2H), 6.98 (dd, *J* = 2 Hz, 5.6 Hz, 1H), 5.49-5.41 (m, 2H), 5.37 (s, 1H), 4.94-4.88 (s, 1H), 4.58-4.50 (m, 1H), 4.23-4.13 (m, 2H), 3.73-3.67 (m, 1H), 2.82-2.67(m, 2H).

### Example 35

### 3-((3-Fluoro-4-((5-(trifluoromethyl)pyridin-3-yl)oxy)benzyl)oxy-6,7,7a,8-tetrahydro-1H-azeto[1',2':3,4]imidazo[1,2-c]pyrimidin-1-one

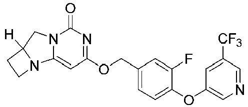

### Synthetic route:

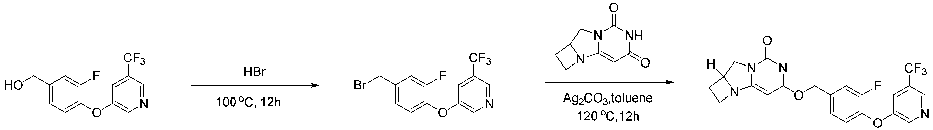

LC-MS[M+H]⁺:449.1.

### Example 36

### 3-((3,5-Difluoro-4-((6-methylpyridin-3-yl)oxy)benzyl)oxy-6,7,7a,8-tetrahydro-1H-azeto[1',2':3,4]imidazo[1,2-c]pyrimidin-1-one

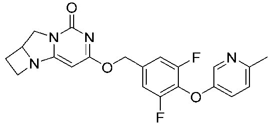

### Synthetic route:

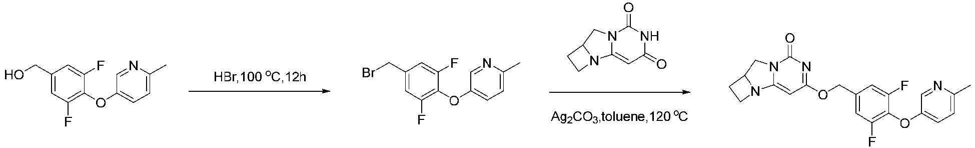

### Step 1: synthesis of 5-(4-(bromomethyl)-2,6-difluorophenoxy)-2-methylpyridine

(3,5-Difluoro-4-((6-methylpyridin-3-yl)oxy)phenyl)methanol (10.0 g, 0.039 mol) was added to a solution of HBr (100 mL), and the mixture was stirred at 100 °C for 12 h. The reaction liquid was cooled to room temperature, and a saturated aqueous NaHCO₃ solution (500 mL) was added. The mixture was extracted with EA (500 mL × 2), and the extract phase was washed with saturated brine, dried over anhydrous Na₂SO₄, filtered, and concentrated to give the target crude product (9.0 g, yield: 64%), which was directly used in the next step without further purification.

LC-MS[M+H]⁺:314.0.

### Step 2: synthesis of 3-((3,5-difluoro-4-((6-methylpyridin-3-yl)oxy)benzyl)oxy-6,7,7a,8-tetrahydro-1H-azeto[1',2':3,4]imidazo[1,2-c]pyrimidin-1-one

6,7,7a,8-Tetrahydro-1H-azeto[1',2':3,4]imidazo[1,2-c]pyrimidine-1,3(2H)-dione (4.0 g, 0.022 mol) and Ag₂CO₃ (15.3 g, 0.055 mol) were added to a solution of toluene (150 mL), and the mixture was stirred at 110 °C for 1 h. The reaction liquid was cooled to 70 °C, and 5-(4-(bromomethyl)-2,6-difluorophenoxy)-2-methylpyridine (7.7 g, 0.024 mol) was added. The resulting mixture was stirred at 120 °C for 12 h. The reaction liquid was cooled to room temperature, filtered, and concentrated, and the residue was separated and purified by column chromatography (EA:MeOH = 10:1) to give a crude product (2.5 g), which was purified by HPLC to give the target product.

LC-MS[M+H]⁺:413.1.

¹H NMR (400 MHz, DMSO-*d*₆) δ 8.26 (d, *J* = 2.9 Hz, 1H), 7.40 (d, *J* = 8.9 Hz, 2H), 7.29 (dd, *J* = 8.6, 2.9 Hz, 1H), 7.23 (d, *J* = 8.6 Hz, 1H), 5.49 (s, 1H), 5.39-5.25 (m, 2H), 4.88-4.76 (m, 1H), 4.40 (dd, *J* = 16.9, 9.1 Hz, 1H), 4.06-3.92 (m, 2H), 3.65 (td, *J* = 9.5, 3.7 Hz, 1H), 2.81 (dt, *J* = 19.0, 9.5 Hz, 1H), 2.50-2.47 (m, 1H), 2.43 (s, 3H).

### Example 36-R

### (R)-3-((3,5-Difluoro-4-((6-methylpyridin-3-yl)oxy)benzyl)oxy-6,7,7a,8-tetrahydro-1H-azeto[1',2':3,41imidazo[1,2-c]pyrimidin-1-one

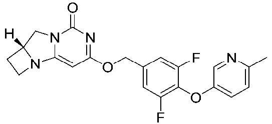

### Synthetic route:

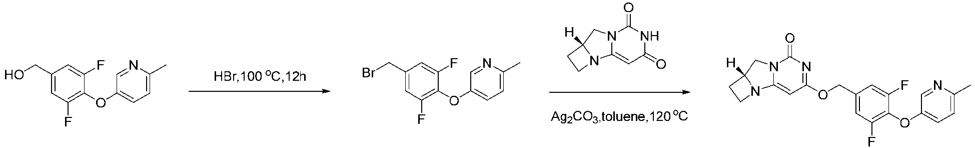

### Step 1: synthesis of 5-(4-(bromomethyl)-2,6-difluorophenoxy)-2-methylpyridine

(3,5-Difluoro-4-((6-methylpyridin-3-yl)oxy)phenyl)methanol (10.0 g, 0.039 mol) was added to a solution of HBr (100 mL), and the mixture was stirred at 100 °C for 12 h. The reaction liquid was cooled to room temperature, and a saturated aqueous NaHCO₃ solution (500 mL) was added. The mixture was extracted with EA (500 mL × 2), and the extract phase was washed with saturated brine, dried over anhydrous Na₂SO₄, filtered, and concentrated to give the target crude product (9.0 g, yield: 64%), which was directly used in the next step without further purification.

LC-MS[M+H]⁺:314.0.

### Step 2: synthesis of (R)-3-((3,5-difluoro-4-((6-methylpyridin-3-yl)oxy)benzyl)oxy-6,7,7a,8-tetrahydro-1H-azeto[1',2':3,4]imidazo[1,2-c]pyrimidin-1-one

(R)-6,7,7a,8-Tetrahydro-1H-azeto[1',2':3,4]imidazo[1,2-c]pyrimidine-1,3(2H)-dione (4.0 g, 0.022 mol) and Ag₂CO₃ (15.3 g, 0.055 mol) were added to a solution of toluene (150 mL), and the mixture was stirred at 110 °C for 1 h. The reaction liquid was cooled to 70 °C, and 5-(4-(bromomethyl)-2,6-difluorophenoxy)-2-methylpyridine (7.7 g, 0.024 mol) was added. The resulting mixture was stirred at 120 °C for 12 h. The reaction liquid was cooled to room temperature, filtered, and concentrated, and the residue was separated and purified by column chromatography (EA:MeOH = 10:1) to give a crude product (2.5 g), which was purified by HPLC to give the target product (1.56 g, yield: 6.4%). LC-MS[M+H]⁺:413.1.

¹H NMR (400 MHz, DMSO-*d*₆) δ 8.26 (d, *J* = 2.9 Hz, 1H), 7.40 (d, *J* = 8.9 Hz, 2H), 7.29 (dd, *J* = 8.6, 2.9 Hz, 1H), 7.24 (d, *J =* 8.6 Hz, 1H), 5.49 (s, 1H), 5.38-5.26 (m, 2H), 4.90-4.75 (m, 1H), 4.40 (dd, *J* = 16.9, 9.1 Hz, 1H), 4.07-3.95 (m, 2H), 3.66 (td, *J* = 9.5, 3.7 Hz, 1H), 2.88-2.72 (m, 1H), 2.50-2.47 (m, 1H), 2.44 (s, 3H).

### Example 36-S

### (S)-3-((3,5-Difluoro-4-((6-methylpyridin-3-yl)oxy)benzyl)oxy-6,7,7a,8-tetrahydro-1H-azeto[1',2':3,4]imidazo[1,2-c]pyrimidin-1-one

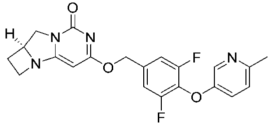

### Synthetic route:

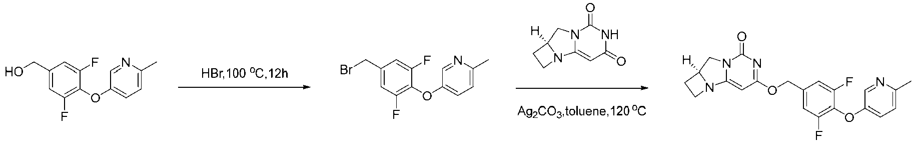

For the synthetic route, reference was made to Example 36-R, and (R)-6,7,7a,8-tetrahydro-1H-azeto[1',2':3,4]imidazo[1,2-c]pyrimidine-1,3(2H)-dione in step 2 was replaced by (S)-6,7,7a,8-tetrahydro-1H-azeto[1',2':3,4]imidazo[1,2-c]pyrimidine-1,3(2H)-dione.

LC-MS[M+H]⁺:413.1.

¹H NMR (400 MHz, DMSO-*d*₆): δ 8.26 (d, *J* = 2.8 Hz, 1H), 7.41-7.38 (m, 2H), 7.30-7.23 (m, 2H), 5.49 (s, 1H), 5.34-5.33 (m, 2H), 4.86-4.83 (m, 1H), 4.41-4.39 (m, 1H), 4.03-4.01 (m, 2H), 3.69-3.64 (m, 1H), 2.83-2.79 (m, 1H), 2.51 (s, 1H), 2.44 (s, 3H).

### Example 37

### 3-((4-(3,4-Difluorophenoxy)-3,5-difluorobenzyl)oxy)-6,7,7a,8-tetrahydro-1H-azeto[1',2':3,4]imidazo[1,2-c]pyrimidin-1-one

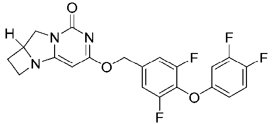

### Synthetic route:

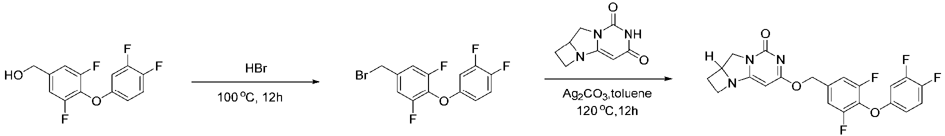

LC-MS[M+H]⁺:434.1.

### Example 38

### 3-((6-(4-Chloro-3-(trifluoromethyl)phenoxy)-5-fluoropyridin-3-yl)methoxy)-6,7,7a,8-tetrahydro-1H-azeto[1',2':3,4]imidazo[1,2-c]pyrimidin-1-one

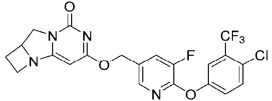

### Synthetic route:

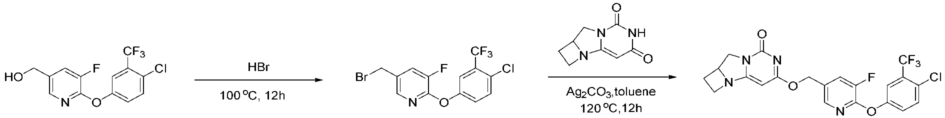

LC-MS[M+H]⁺:483.1.

¹H NMR (400 MHz, DMSO-*d*₆) δ 8.05 (s, 1H), 8.03-7.97 (m, 1H), 7.80 (dd, *J* = 5.7, 2.8 Hz, 2H), 7.61 (dd, *J* = 8.8, 2.7 Hz, 1H), 5.42 (s, 1H), 5.36-5.24 (m, 2H), 4.87-4.75 (m, 1H), 4.38 (dd, *J* = 16.9, 9.1 Hz, 1H), 4.05-3.92 (m, 2H), 3.63 (td, *J=* 9.5, 3.7 Hz, 1H), 2.91-2.79 (m, 1H), 2.50-2.46 (m, 1H).

### Example 39

### 2-(4-Chloro-3-(trifluoromethyl)phenoxy)-5-(((1-oxo-6,7,7a,8-tetrahydro-1H-azeto[1',2':3,4]imidazo[1,2-c]pyrimidin-3-yl)oxy)methyl)benzonitrile

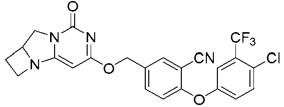

### Synthetic route:

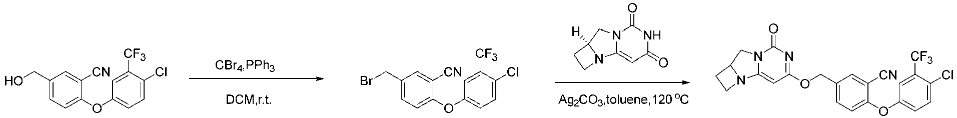

LC-MS[M+H]⁺:489.1.

¹H NMR (400 MHz, DMSO-*d*₆) δ 8.00 (s, 1H), 7.81 (d, *J* = 8.7 Hz, 1H), 7.75 (d, *J* = 9.7 Hz, 2H), 7.51 (d, *J* = 8.4 Hz, 1H), 7.17 (d, *J* = 8.7 Hz, 1H), 5.45 (s, 1H), 5.37-5.24 (m, 2H), 4.90-4.74 (m, 1H), 4.39 (q, *J =* 8.9 Hz, 1H), 4.01 (dd, *J* = 11.6, 8.0 Hz, 2H), 3.64 (d, *J* = 9.2 Hz, 1H), 2.86-2.73 (m, 1H), 2.49-2.44 (m, 1H).

### Example 40

### 3-((3,5-Difluoro-4-(3-(trifluoromethyl)phenoxy)benzyl)oxy)-6,7,7a,8-tetrahydro-1H-azeto[1',2':3,4]imidazo[1,2-c]pyrimidin-1-one

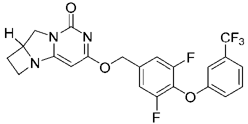

### Synthetic route:

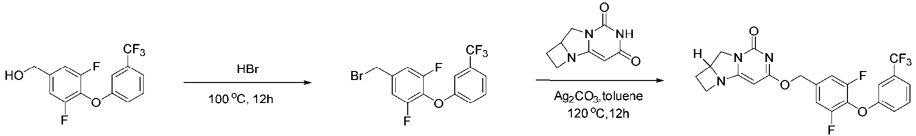

LC-MS[M+H]⁺:466.1.

### Example 41

### 3-((6-(4-Fluoro-3-(trifluoromethyl)phenoxy)pyridin-3-yl)methoxy)-6,7,7a,8-tetrahydro-1H-azeto[1',2':3,4]imidazo[1,2-c]pyrimidin-1-one

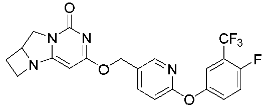

### Synthetic route:

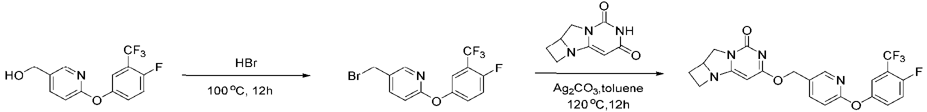

LC-MS[M+H]⁺:449.1.

¹H NMR (400 MHz, DMSO-*d*₆) δ 8.22 (d, *J* = 2.1 Hz, 1H), 7.97 (dd, *J* = 8.4, 2.3 Hz, 1H), 7.62 (d, *J* = 5.1 Hz, 1H), 7.59-7.55 (m, 2H), 7.15 (d, *J* = 8.5 Hz, 1H), 5.40 (s, 1H), 5.28 (q, *J* = 12.3 Hz, 2H), 4.87-4.75 (m, 1H), 4.37 (dd, *J* = 16.9, 9.1 Hz, 1H), 4.05-3.93 (m, 2H), 3.63 (td, *J* = 9.5, 3.8 Hz, 1H), 2.79 (dt, *J =* 19.1, 9.6 Hz, 1H), 2.50-2.46 (m, 1H).

### Example 42

### 2-(3,5-Difluorophenoxy)-5-(((1-oxo-6,7,7a,8-tetrahydro-1H-azeto[1',2':3,4]imidazo[1,2-c]pyrimidin-3-yl)oxy)methyl)benzonitrile

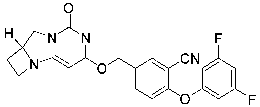

### Synthetic route:

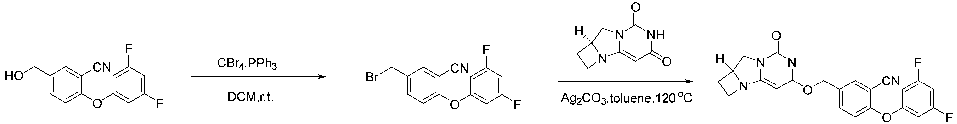

LC-MS[M+H]⁺:423.1.

### Example 43

### 3-((3,5-Difluoro-4-((2-(trifluoromethyl)pyrimidin-5-yl)oxy)benzyl)oxy-6,7,7a,8-tetrahydro-1H-azeto[1',2':3,4]imidazo[1,2-c]pyrimidin-1-one

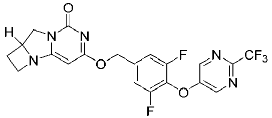

### Synthetic route:

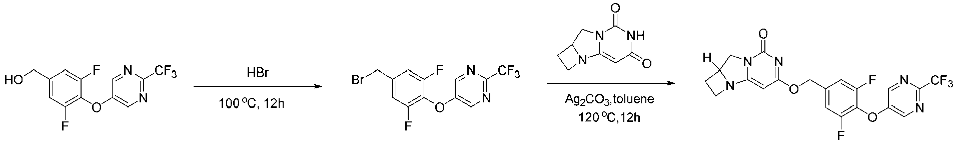

LC-MS[M+H]⁺:468.1.

### Example 44

### 3-((3,5-Difluoro-4-((2-methylpyrimidin-5-yl)oxy)benzyl)oxy-6,7,7a,8-tetrahydro-1H-azeto[1',2':3,4]imidazo[1,2-c]pyrimidin-1-one

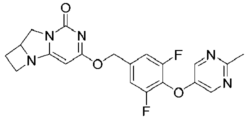

### Synthetic route:

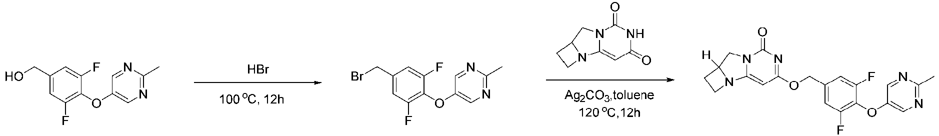

LC-MS[M+H]⁺:414.1.

¹H NMR (400 MHz, DMSO-*d*₆) δ 8.55 (s, 2H), 7.42 (d, *J* = 9.0 Hz, 2H), 5.49 (s, 1H), 5.41-5.26 (m, 2H), 4.91-4.77 (m, 1H), 4.40 (dd, *J* = 16.9, 9.1 Hz, 1H), 4.06-3.94 (m, 2H), 3.66 (td, *J =* 9.5, 3.8 Hz, 1H), 2.81 (dt, *J =* 19.1, 9.5 Hz, 1H), 2.60 (s, 3H), 2.49-2.44 (m, 1H).

### Example 45

### 3-((3,5-Difluoro-4-((1-methyl-1H-pyrazol-4-yl)oxy)benzyl)oxy-6,7,7a,8-tetrahydro-1H-azeto[1',2':3,4]imidazo[1,2-c]pyrimidin-1-one

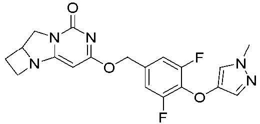

### Synthetic route:

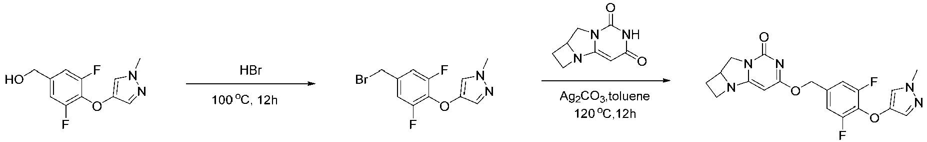

LC-MS[M+H]⁺:402.1.

¹H NMR (400 MHz, DMSO-*d*₆) δ 7.60 (s, 1H), 7.32 (d, *J* = 8.9 Hz, 2H), 7.27 (s, 1H), 5.47 (s, 1H), 5.36-5.22 (m, 2H), 4.84 (tt, *J* = 12.3, 6.0 Hz, 1H), 4.39 (dd, *J* = 16.9, 9.1 Hz, 1H), 4.08-3.94 (m, 2H), 3.73 (s, 3H), 3.65 (td, *J* = 9.5, 3.7 Hz, 1H), 2.86-2.74 (m, 1H), 2.47-2.41 (m, 1H).

### Example 46

### 3-((4-((1-Ethyl-1H-pyrazol-4-yl)oxy)-3,5-difluorobenzyl)oxy-6,7,7a,8-tetrahydro-1H-azeto[1',2':3,4]imidazo[1,2-c]pyrimidin-1-one

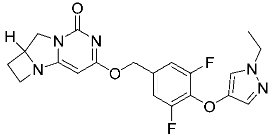

### Synthetic route:

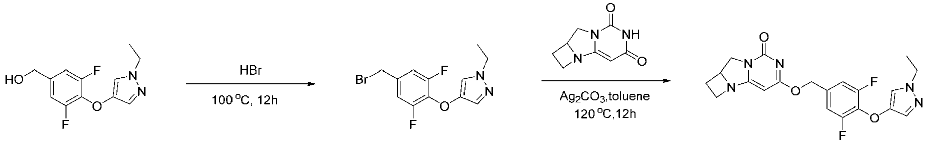

LC-MS[M+H]⁺:416.2.

### Example 47

### 3-((4-((1-Isopropyl-1H-pyrazol-4-yl)oxy)-3,5-difluorobenzyl)oxy-6,7,7a,8-tetrahydro-1H-azeto[1',2':3,4]imidazo[1,2-c]pyrimidin-1-one

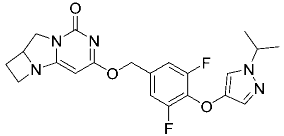

### Synthetic route:

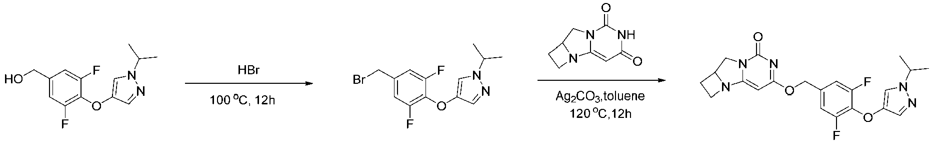

LC-MS[M+H]⁺:430.2.

¹H NMR (400 MHz, DMSO-*d*₆) δ 7.68 (s, 1H), 7.32 (d, *J =* 8.8 Hz, 2H), 7.26 (s, 1H), 5.47 (s, 1H), 5.36-5.21 (m, 2H), 4.90-4.75 (m, 1H), 4.46-4.27 (m, 2H), 4.08-3.92 (m, 2H), 3.65 (td, *J* = 9.5, 3.7 Hz, 1H), 2.80 (dt, *J* = 19.0, 9.5 Hz, 1H), 2.50-2.47 (m, 1H), 1.35 (d, *J =* 6.7 Hz, 6H).

### Example 48

### 3-((4-((1-(Trifluoromethyl)-1H-pyrazol-4-yl)oxy)-3,5-difluorobenzyl)oxy-6,7,7a,8-tetrahydro-1H-azeto[1',2':3,4]imidazo[1,2-c]pyrimidin-1-one

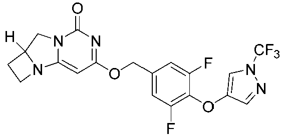

### Synthetic route:

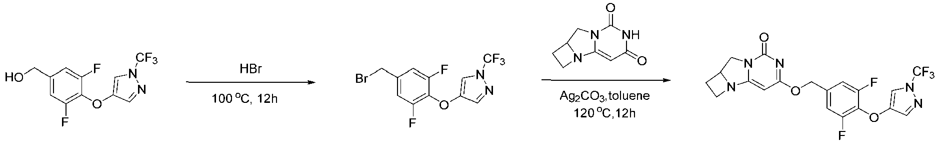

LC-MS[M+H]⁺:456.1.

### Example 49

### 3-((3-Fluoro-4-((1-methyl-1H-pyrazol-4-yl)oxy)benzyl)oxy-6,7,7a,8-tetrahydro-1H-azeto[1',2':3,4]imidazo[1,2-c]pyrimidin-1-one

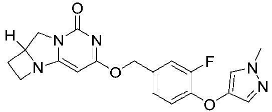

### Synthetic route:

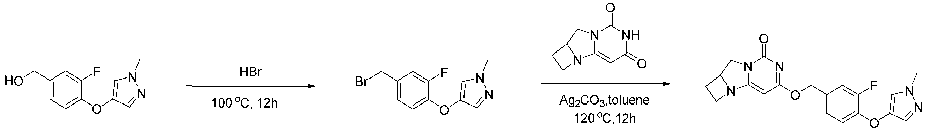

LC-MS[M+H]⁺:384.1.

### Example 50

### 3-((4-(4-Chloro-3-(trifluoromethyl)phenoxy)benzyl)oxy)-6,7,7a,8-tetrahydro-1H-azeto[1',2':3,4]imidazo[1,2-c]pyrimidin-1-one

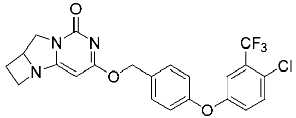

### Synthetic route:

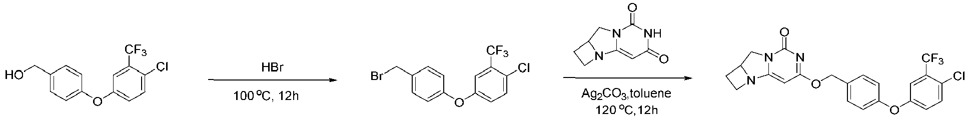

LC-MS[M+H]⁺:464.1.

¹H NMR (400 MHz, DMSO-*d*₆) δ 7.72 (d, *J* = 8.8 Hz, 1H), 7.50 (d, *J* = 8.5 Hz, 2H), 7.46 (d,*J* = 2.8 Hz, 1H), 7.29 (dd, *J* = 8.9, 2.8 Hz, 1H), 7.14 (d, *J* = 8.5 Hz, 2H), 5.43 (s, 1H), 5.30 (q, *J* = 12.4 Hz, 2H), 4.83 (tt, *J* = 11.8, 5.9 Hz, 1H), 4.38 (dd, *J* = 17.0, 9.1 Hz, 1H), 4.09-3.93 (m, 2H), 3.63 (td, *J* = 9.6, 3.7 Hz, 1H), 2.89-2.72 (m, 1H), 2.46-2.37 (m, 1H).

### Example 51

### 3-((3-Fluoro-4-((2-(trifluoromethyl)pyrimidin-5-yl)oxy)benzyl)oxy-6,7,7a,8-tetrahydro-1H-azeto[1',2':3,4]imidazo[1,2-c]pyrimidin-1-one

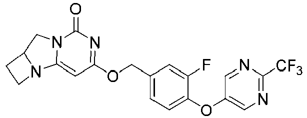

### Synthetic route:

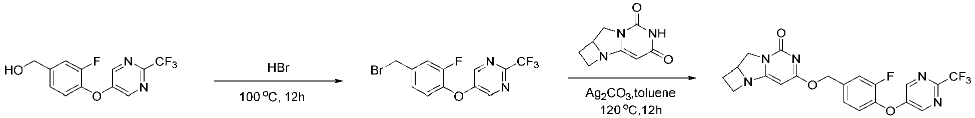

LC-MS[M+H]⁺:450.1.

¹H NMR (400 MHz, DMSO-*d*₆) δ 8.88 (s, 2H), 7.56 (dd, *J* = 11.6, 1.5 Hz, 1H), 7.49 (t, *J* = 8.4 Hz, 1H), 7.36 (d, *J* = 8.1 Hz, 1H), 5.47 (s, 1H), 5.40-5.28 (m, 2H), 4.89-4.78 (m, 1H), 4.39 (dd, *J* = 16.9, 9.1 Hz, 1H), 4.07-3.93 (m, 2H), 3.65 (td, *J* = 9.6, 3.8 Hz, 1H), 2.80 (dt, *J* = 19.1, 9.5 Hz, 1H), 2.50-2.48 (m, 1H).

### Example 52

### 3-((3,5-Difluoro-4-((6-(trifluoromethyl)pyridin-3-yl)oxy)benzyl)oxy-6,7,7a,8-tetrahydro-1H-azeto[1',2':3,4]imidazo[1,2-c]pyrimidin-1-one

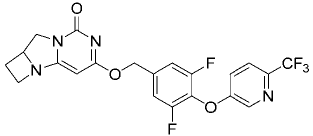

### Synthetic route:

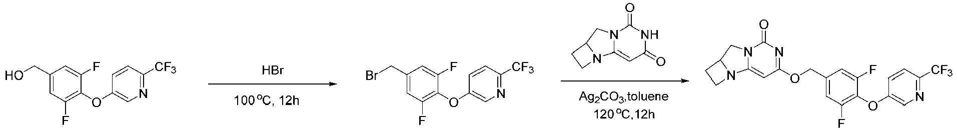

LC-MS[M+H]⁺:467.1.

¹H NMR (400 MHz, DMSO-*d*₆) δ 8.68 (d, *J* = 2.8 Hz, 1H), 7.91 (d, *J* = 8.8 Hz, 1H), 7.63 (dd, *J* = 8.6, 2.7 Hz, 1H), 7.46 (d, *J* = 8.9 Hz, 2H), 5.50 (s, 1H), 5.40-5.27 (m, 2H), 4.91-4.75 (m, 1H), 4.40 (dd, *J* = 16.9, 9.1 Hz, 1H), 4.08-3.93 (m, 2H), 3.66 (td, *J* = 9.5, 3.7 Hz, 1H), 2.91-2.72 (m, 1H), 2.48-2.36 (m, 1H).

### Example 53

### 2-Chloro-5-(2-fluoro-4-(((1-oxo-6,7,7a,8-tetrahydro-1H-azeto[1',2':3,4]imidazo[1,2-c]pyrimidin-3-yl)oxy)methyl)phenoxy)benzonitrile

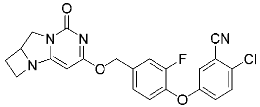

### Synthetic route:

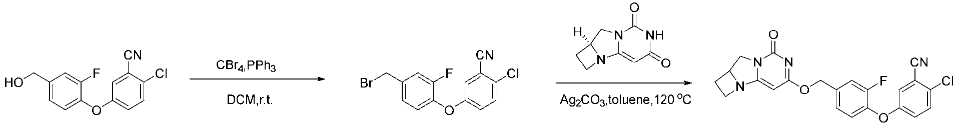

LC-MS[M+H]⁺:439.1.

¹H NMR (400 MHz, DMSO-*d*₆) δ 7.75-7.69 (m, 2H), 7.51 (d, *J* = 12.2 Hz, 1H), 7.38-7.27 (m, 3H), 5.46 (s, 1H), 5.38-5.25 (m, 2H), 4.83 (qd, *J* = 7.5, 4.9 Hz, 1H), 4.39 (dd, *J* = 16.9, 9.1 Hz, 1H), 4.06-3.93 (m, 2H), 3.64 (td, *J* = 9.5, 3.8 Hz, 1H), 2.90-2.73 (m, 1H), 2.48-2.42 (m, 1H).

### Example 54

### 3-((3,4-Difluorobenzyl)oxy)-6,7,7a,8-tetrahydro-1H-azeto[1',2':3,4]imidazo[1,2-c]pyrimidin-1-one

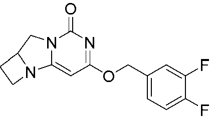

### Synthetic route:

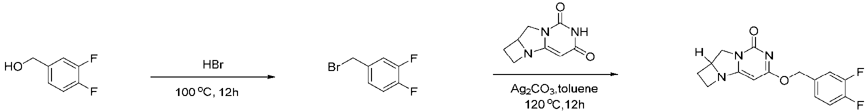

LC-MS[M+H]⁺:306.1.

¹H NMR (400 MHz, DMSO-*d*₆) δ 7.55-7.40 (m, 2H), 7.33-7.25 (m, 1H), 5.45 (s, 1H), 5.33-5.21 (m, 2H), 4.83 (qd, *J =* 7.4, 4.6 Hz, 1H), 4.38 (dt, *J =* 9.2, 7.7 Hz, 1H), 4.06-3.94 (m, 2H), 3.64 (td, *J =* 9.5, 3.8 Hz, 1H), 2.87-2.72 (m, 1H), 2.48-2.41 (m, 1H).

**The synthetic routes for the enantiomers of Examples 1-54 referred to the preparation of racemates thereof, with specific details shown in the table below:**

| Example No. | Compound name | Compound structure | LC-MS [M+H]⁺ |
|---|---|---|---|
| 1-R | (R)-3-((3-Fluoro-4-((6-(trifluoromethyl)pyridin-3-yl)oxy)benzyl)oxy-6,7,7a,8-tetrahydro-1H-azeto[1',2':3,4]imidazo[1,2-c]pyrimidin-1-one | 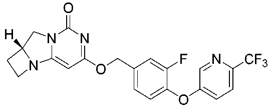 | 449.1 |
| 1-S | (S)-3-((3-Fluoro-4-((6-(trifluoromethyl)pyridin-3-yl)oxy)benzyl)oxy-6,7,7a,8-tetrahydro-1H-azeto[1',2':3,4]imidazo[1,2-c]pyrimidin-1-one | 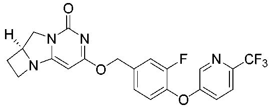 | 449.1 |
| 2-R | (R)-3-((3,4,5-Trifluorobenzyl)oxy)-6,7,7a,8-tetrahydro-1H-azeto[1',2':3,4]imidazo[1,2-c]pyrimidin-1-one | 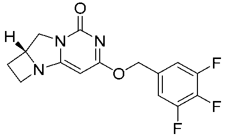 | 324.1 |
| 2-S | (S)-3-((3,4,5-Trifluorobenzyl)oxy)-6,7,7a,8-tetrahydro-1H-azeto[1',2':3,4]imidazo[1,2-c]pyrimidin-1-one | 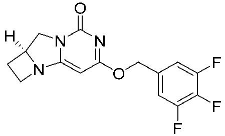 | 324.1 |
| 3-R | (R)-3-((3-Chloro-5-fluorobenzyl)oxy)-6,7,7a,8-tetrahydro-1H-azeto[1',2':3,4]imidazo[1,2-c]pyrimidin-1-one | 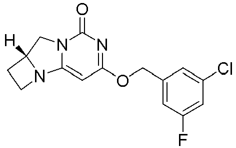 | 322.1 |
| 3-S | (S)-3-((3-Chloro-5-fluorobenzyl)oxy)-6,7,7a,8-tetrahydro-1H-azeto[1',2':3,4]imidazo[1,2-c]pyrimidin-1-one | 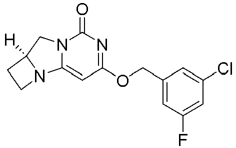 | 322.1 |
| 4-R | (R)-3-((3,5-Difluorobenzyl)oxy)-6,7,7a,8-tetrahydro-1H-azeto[1',2':3,4]imidazo[1,2-c]pyrimidin-1-one | 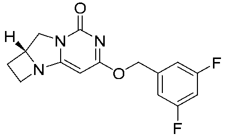 | 306.1 |
| 4-S | (S)-3-((3,5-Difluorobenzyl)oxy)-6, 7, 7a,8-tetrahydro-1H-azeto[1',2':3,4]imidazo[1,2-c]pyrimidin-1-one | 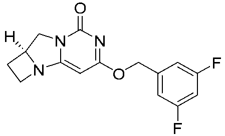 | 306.1 |
| 5-R | (R)-3-((3-Chloro-4-(trifluoromethyl)benzyl)oxy)-6,7,7a,8-tetrahydro-1H-azeto[1',2':3,4]imidazo[1,2-c]pyrimidin-1-one | 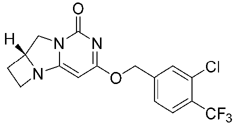 | 372.1 |
| 5-S | (S)-3-((3-Chloro-4-(trifluoromethyl)benzyl)oxy)-6,7,7a,8-tetrahydro-1H-azeto[1',2':3,4]imidazo[1,2-c]pyrimidin-1-one | 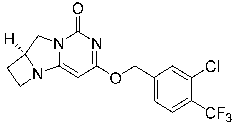 | 372.1 |
| 6-R | (R)-3-((2,4-Difluorobenzyl)oxy)-6,7,7a,8-tetrahydro-1H-azeto[1',2':3,4]imidazo[1,2-c]pyrimidin-1-one | 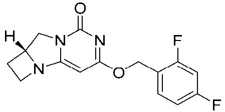 | 306.1 |
| 6-S | (S)-3-((2,4-Difluorobenzyl)oxy)-6,7,7a,8-tetrahydro-1H-azeto[1',2':3,4]imidazo[1,2-c]pyrimidin-1-one | 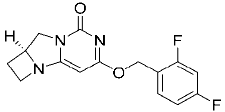 | 306.1 |
| 7-R | (R)-3-((4-Fluorobenzyl)oxy)-6,7,7a,8-tetrahydro-1H-azeto[1',2':3,4]imidazo[1,2-c]pyrimidin-1-one | 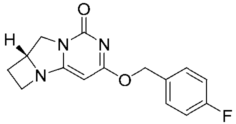 | 288.1 |
| 7-S | (S)-3-((4-Fluorobenzyl)oxy)-6,7,7a,8-tetrahydro-1H-azeto[1',2':3,4]imidazo[1,2-c]pyrimidin-1-one | 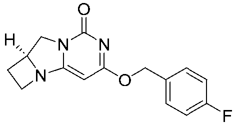 | 288.1 |
| 8-R | (R)-3-((4-Methylbenzyl)oxy)-6,7,7a,8-tetrahydro-1H-azeto[1',2':3,4]imidazo[1,2-c]pyrimidin-1-one | 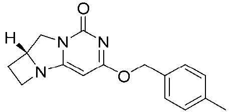 | 284.1 |
| 8-S | (S)-3-((4-Methylbenzyl)oxy)-6,7,7a,8-tetrahydro-1H-azeto[1',2':3,4]imidazo[1,2-c]pyrimidin-1-one | 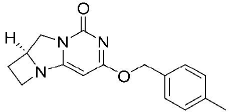 | 284.1 |
| 9-R | (R)-3-((3,4-Difluoro-5-methylbenzyl)oxy)-6,7,7a,8-tetrahydro-1H-azeto[1',2':3,4]imidazo[1,2-c]pyrimidin-1-one | 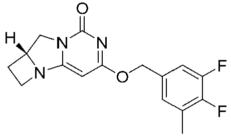 | 320.1 |
| 9-S | (S)-3-((3,4-Difluoro-5-methylbenzyl)oxy)-6,7,7a,8-tetrahydro-1H-azeto[1',2':3,4]imidazo[1,2-c]pyrimidin-1-one | 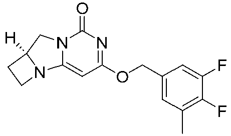 | 320.1 |
| 10-R | (R)-3-((3-Cyano-5-fluorobenzyl)oxy)-6,7,7a,8-tetrahydro-1H-azeto[1',2':3,4]imidazo[1,2-c]pyrimidin-1-one | 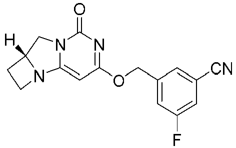 | 313.1 |
| 10-S | (S)-3-((3-Cyano-5-fluorobenzyl)oxy)-6,7,7a,8-tetrahydro-1H-azeto[1',2':3,4]imidazo[1,2-c]pyrimidin-1-one | 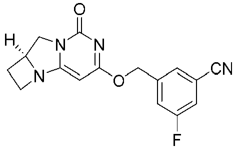 | 313.1 |
| 11-R | (R)-3-((3,4-Difluoro-5-chlorobenzyl)oxy)-6,7,7a,8-tetrahydro-1H-azeto[1',2':3,4]imidazo[1,2-c]pyrimidin-1-one | 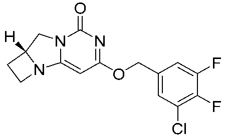 | 340.1 |
| 11-S | (S)-3-((3,4-Difluoro-5-chlorobenzyl)oxy)-6,7,7a,8-tetrahydro-1H-azeto[1',2':3,4]imidazo[1,2-c]pyrimidin-1-one | 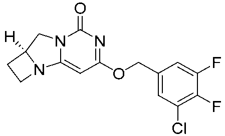 | 340.1 |
| 12-R | (R)-3-((4-Phenoxybenzyl)oxy)-6,7,7a,8-tetrahydro-1H-azeto[1',2':3,4]imidazo[1,2-c]pyrimidin-1-one | 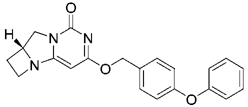 | 362.1 |
| 12-S | (S)-3-((4-Phenoxybenzyl)oxy)-6,7,7a,8-tetrahydro-1H-azeto[1',2':3,4]imidazo[1,2-c]pyrimidin-1-one | 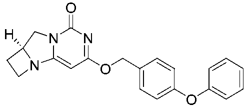 | 362.1 |
| 13-R | (R)-3-((4-(Pyridin-4-yloxy)benzyl)oxy)-6,7,7a,8-tetrahydro-1H-azeto[1',2':3,4]imidazo[1,2-c]pyrimidin-1-one | 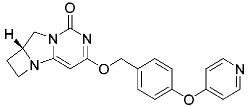 | 363.1 |
| 13-S | (S)-3-((4-(Pyridin-4-yloxy)benzyl)oxy)-6,7,7a,8-tetrahydro-1H-azeto[1',2':3,4]imidazo[1,2-c]pyrimidin-1-one | 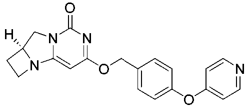 | 363.1 |
| 14-R | (R)-3-((4-(Pyridin-2-yloxy)benzyl)oxy)-6,7,7a,8-tetrahydro-1H-azeto[1',2':3,4]imidazo[1,2-c]pyrimidin-1-one | 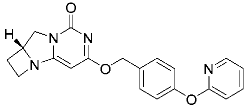 | 363.1 |
| 14-S | (S)-3-((4-(Pyridin-2-yloxy)benzyl)oxy)-6,7,7a,8-tetrahydro-1H-azeto[1',2':3,4]imidazo[1,2-c]pyrimidin-1-one | 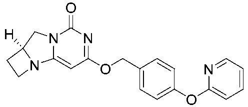 | 363.1 |
| 15-R | (R)-3-((4-(Pyrimidin-4-yloxy)benzyl)oxy)-6,7,7a,8-tetrahydro-1H-azeto[1',2':3,4]imidazo[1,2-c]pyrimidin-1-one | 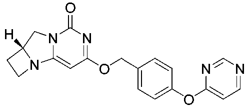 | 364.1 |
| 15-S | (S)-3-((4-(Pyrimidin-4-yloxy)benzyl)oxy)-6,7,7a,8-tetrahydro-1H-azeto[1',2':3,4]imidazo[1,2-c]pyrimidin-1-one | 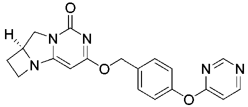 | 364.1 |
| 16-R | (R)-3-((6-Phenoxypyridin-3-yl)methoxy)-6,7,7a,8-tetrahydro-1H-azeto[1',2':3,4]imidazo[1,2-c]pyrimidin-1-one | 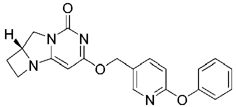 | 363.1 |
| 16-S | (S)-3-((6-Phenoxypyridin-3-yl)methoxy)-6,7,7a,8-tetrahydro-1H-azeto[1',2':3,4]imidazo[1,2-c]pyrimidin-1-one | 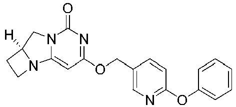 | 363.1 |
| 17-R | (R)-3-((5-Phenoxypyridin-2-yl)methoxy)-6,7,7a,8-tetrahydro-1H-azeto[1',2':3,4]imidazo[1,2-c]pyrimidin-1-one | 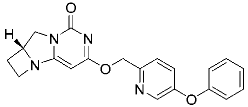 | 363.1 |
| 17-S | (S)-3-((5-Phenoxypyridin-2-yl)methoxy)-6,7,7a,8-tetrahydro-1H-azeto[1',2':3,4]imidazo[1,2-c]pyrimidin-1-one | 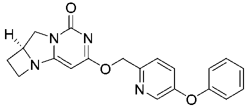 | 363.1 |
| 18-R | (R)-3-((5-Phenoxypyrimidin-2-yl)methoxy)-6,7,7a,8-tetrahydro-1H-azeto[1',2':3,4]imidazo[1,2-c]pyrimidin-1-one | 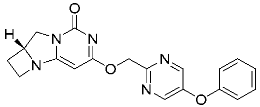 | 364.1 |
| 18-S | (S)-3-((5-Phenoxypyrimidin-2-yl)methoxy)-6,7,7a,8-tetrahydro-1H-azeto[1',2':3,4]imidazo[1,2-c]pyrimidin-1-one | 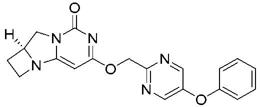 | 364.1 |
| 19-R | (R)-3-((6-(Pyrimidin-4-yloxy)pyridin-3-yl)methoxy)-6,7,7a,8-tetrahydro-1H-azeto[1',2':3,4]imidazo[1,2-c]pyrimidin-1-one | 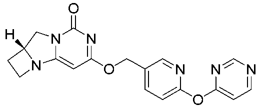 | 365.1 |
| 19-S | (S)-3-((6-(Pyrimidin-4-yloxy)pyridin-3-yl)methoxy)-6,7,7a,8-tetrahydro-1H-azeto[1',2':3,4]imidazo[1,2-c]pyrimidin-1-one | 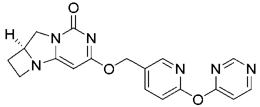 | 365.1 |
| 20-R | (R)-5-(((1-Oxo-6,7,7a,8-tetrahydro-1H-azeto[1',2':3,4]imidazo[1,2-c]pyrimidin-3-yl)oxy)methyl)-2-((5-(trifluoromethyl)pyridin-3-yl)oxy)benzonitrile | 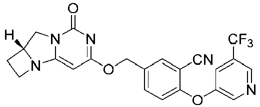 | 456.1 |
| 20-S | (S)-5-(((1-Oxo-6,7,7a,8-tetrahydro-1H-azeto[1',2':3,4]imidazo[1,2-c]pyrimidin-3-yl)oxy)methyl)-2-((5-(trifluoromethyl)pyridin-3-yl)oxy)benzonitrile | 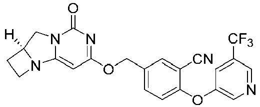 | 456.1 |
| 21-R | (R)-5-(((1-Oxo-6,7,7a,8-tetrahydro-1H-azeto[1',2':3,4]imidazo[1,2-c]pyrimidin-3-yl)oxy)methyl)-2-((5-(trifluoromethyl)pyridin-2-yl)oxy)benzonitrile | 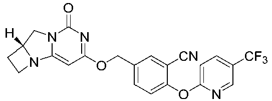 | 456.1 |
| 21-S | (S)-5-(((1-Oxo-6,7,7a,8-tetrahydro-1H-azeto[1',2':3,4]imidazo[1,2-c]pyrimidin-3-yl)oxy)methyl)-2-((5-(trifluoromethyl)pyridin-2-yl)oxy)benzonitrile | 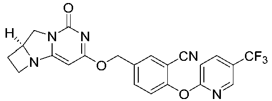 | 456.1 |
| 22-R | (R)-5-(((1-Oxo-6,7,7a,8-tetrahydro-1H-azeto[1',2':3,4]imidazo[1,2-c]pyrimidin-3-yl)oxy)methyl)-2-(3-(trifluoromethyl)phenoxy)benzonitrile | 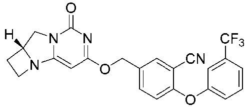 | 455.1 |
| 22-S | (S)-5-(((1-Oxo-6,7,7a,8-tetrahydro-1H-azeto[1',2':3,4]imidazo[1,2-c]pyrimidin-3-yl)oxy)methyl)-2-(3-(trifluoromethyl)phenoxy)benzonitrile | 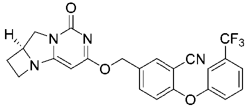 | 455.1 |
| 23-R | (R)-3-((3-Fluoro-4-((2-(trifluoromethyl)pyridin-4-yl)oxy)benzyl)oxy-6,7,7a,8-tetrahydro-1H-azeto[1',2':3,4]imidazo[1,2-c]pyrimidin-1-one | 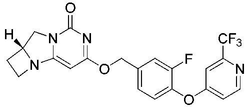 | 449.1 |
| 23-S | (S)-3-((3-Fluoro-4-((2-(trifluoromethyl)pyridin-4-yl)oxy)benzyl)oxy-6,7,7a,8-tetrahydro-1H-azeto[1',2':3,4]imidazo[1,2-c]pyrimidin-1-one | 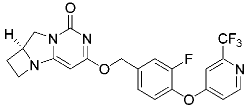 | 449.1 |
| 24-R | (R)-3-((4-(3,4-Difluorophenoxy)-3-fluorobenzyl)oxy)-6,7,7a,8-tetrahydro-1H-azeto[1',2':3,4]imidazo[1,2-c]pyrimidin-1-one | 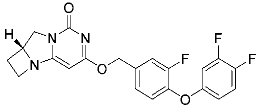 | 416.1 |
| 24-S | (S)-3-((4-(3,4-Difluorophenoxy)-3-fluorobenzyl)oxy)-6,7,7a,8-tetrahydro-1H-azeto[1',2':3,4]imidazo[1,2-c]pyrimidin-1-one | 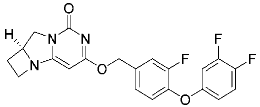 | 416.1 |
| 25-R | (R)-2-(3,4-Difluorophenoxy)-5-(((1-oxo-6,7,7a,8-tetrahydro-1H-azeto[1',2':3,4]imidazo[1,2-c]pyrimidin-3-yl)oxy)methyl)benzonitrile | 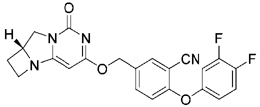 | 423.1 |
| 25-S | (S)-2-(3,4-Difluorophenoxy)-5-(((1-oxo-6,7,7a,8-tetrahydro-1H-azeto[1',2':3,4]imidazo[1,2-c]pyrimidin-3-yl)oxy)methyl)benzonitrile | 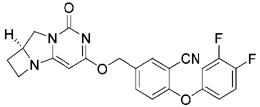 | 423.1 |
| 26-R | (R)-2-Chloro-4-(2-cyano-4-(((1-oxo-6,7,7a,8-tetrahydro-1H-azeto[1',2':3,4]imidazo[1,2-c]pyrimidin-3-yl)oxy)methyl)phenoxy)benzonitrile | 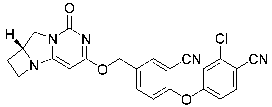 | 446.1 |
| 26-S | (S)-2-Chloro-4-(2-cyano-4-(((1-oxo-6,7,7a,8-tetrahydro-1H-azeto[1',2':3,4]imidazo[1,2-c]pyrimidin-3-yl)oxy)methyl)phenoxy)benzonitrile | 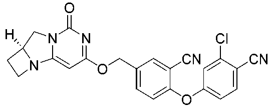 | 446.1 |
| 27-R | (R)-3-((3,5-Difluoro-4-((5-(trifluoromethyl)pyridin-3-yl)oxy)benzyl)oxy-6,7,7a,8-tetrahydro-1H-azeto[1',2':3,4]imidazo[1,2-c]pyrimidin-1-one | 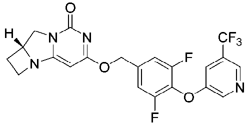 | 467.1 |
| 27-S | (S)-3-((3,5-Difluoro-4-((5-(trifluoromethyl)pyridin-3-yl)oxy)benzyl)oxy-6,7,7a,8-tetrahydro-1H-azeto[1',2':3,4]imidazo[1,2-c]pyrimidin-1-one | 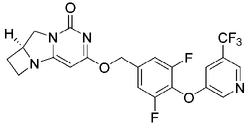 | 467.1 |
| 28-R | (R)-3-((4-Cyanobenzyl)oxy)-6,7,7a,8-tetrahydro-1H-azeto[1',2':3,4]imidazo[1,2-c]pyrimidin-1-one | 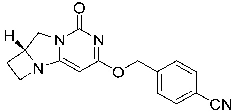 | 295.1 |
| 28-S | (S)-3-((4-Cyanobenzyl)oxy)-6,7,7a,8-tetrahydro-1H-azeto[1',2':3,4]imidazo[1,2-c]pyrimidin-1-one | 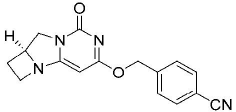 | 295.1 |
| 29-R | (R)-5-(((1-Oxo-6,7,7a,8-tetrahydro-1H-azeto[1',2':3,4]imidazo[1,2-c]pyrimidin-3-yl)oxy)methyl)-2-(4-(trifluoromethyl)phenoxy)benzonitrile | 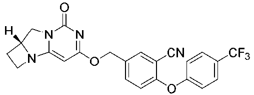 | 455.1 |
| 29-S | (S)-5-(((1-Oxo-6,7,7a,8-tetrahydro-1H-azeto[1',2':3,4]imidazo[1,2-c]pyrimidin-3-yl)oxy)methyl)-2-(4-(trifluoromethyl)phenoxy)benzonitrile | 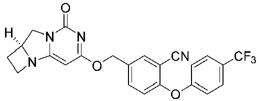 | 455.1 |
| 30-R | (R)-2-(4-Chloro-3-fluorophenoxy)-5-(((1-oxo-6,7,7a,8-tetrahydro-1H-azeto[1',2':3,4]imidazo[1,2-c]pyrimidin-3-yl)oxy)methyl)benzonitrile | 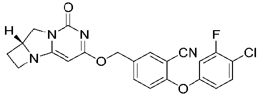 | 439.1 |
| 30-S | (S)-2-(4-Chloro-3-fluorophenoxy)-5-(((1-oxo-6,7,7a,8-tetrahydro-1H-azeto[1',2':3,4]imidazo[1,2-c]pyrimidin-3-yl)oxy)methyl)benzonitrile | 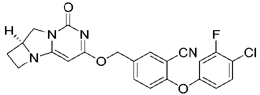 | 439.1 |
| 31-R | (R)-3-((3,5-Difluoro-4-((5-(trifluoromethyl)pyridin-2-yl)oxy)benzyl)oxy-6,7,7a,8-tetrahydro-1H-azeto[1',2':3,4]imidazo[1,2-c]pyrimidin-1-one | 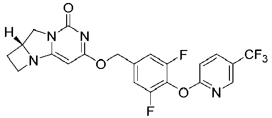 | 467.1 |
| 31-S | (S)-3-((3,5-Difluoro-4-((5-(trifluoromethyl)pyridin-2-yl)oxy)benzyl)oxy-6,7,7a,8-tetrahydro-1H-azeto[1',2':3,4]imidazo[1,2-c]pyrimidin-1-one | 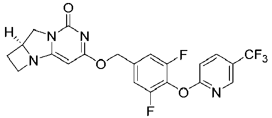 | 467.1 |
| 32-R | (R)-3-((4-((2-Chloropyridin-4-yl)oxy)-3,5-difluorobenzyl)oxy-6,7,7a,8-tetrahydro-1H-azeto[1',2':3,4]imidazo[1,2-c]pyrimidin-1-one | 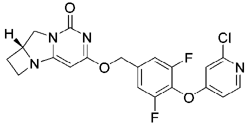 | 433.1 |
| 32-S | (S)-3-((4-((2-Chloropyridin-4-yl)oxy)-3,5-difluorobenzyl)oxy-6,7,7a,8-tetrahydro-1H-azeto[1',2':3,4]imidazo[1,2-c]pyrimidin-1-one | 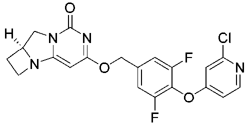 | 433.1 |
| 33-R | (R)-3-(2-Fluoro-4-(((1-oxo-6,7,7a,8-tetrahydro-1H-azeto[1',2':3,4]imidazo[1,2-c]pyrimidin-3-yl)oxy)methyl)phenoxy)-5-(trifluoromethyl)benzonitrile | 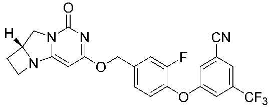 | 473.1 |
| 33-S | (S)-3-(2-Fluoro-4-(((1-oxo-6,7,7a,8-tetrahydro-1H-azeto[1',2':3,4]imidazo[1,2-c]pyrimidin-3-yl)oxy)methyl)phenoxy)-5-(trifluoromethyl)benzonitrile | 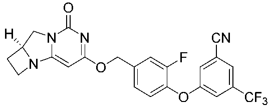 | 473.1 |
| 35-R | (R)-3-((3-Fluoro-4-((5-(trifluoromethyl)pyridin-3-yl)oxy)benzyl)oxy-6,7,7a,8-tetrahydro-1H-azeto[1',2':3,4]imidazo[1,2-c]pyrimidin-1-one | 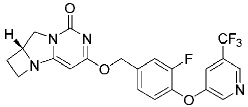 | 449.1 |
| 35-S | (S)-3-((3-Fluoro-4-((5-(trifluoromethyl)pyridin-3-yl)oxy)benzyl)oxy-6,7,7a,8-tetrahydro-1H-azeto[1',2':3,4]imidazo[1,2-c]pyrimidin-1-one | 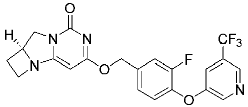 | 449.1 |
| 37-R | (R)-3-((4-(3,4-Difluorophenoxy)-3,5-difluorobenzyl)oxy)-6,7,7a,8-tetrahydro-1H-azeto[1',2':3,4]imidazo[1,2-c]pyrimidin-1-one | 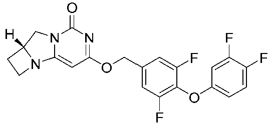 | 434.1 |
| 37-S | (S)-3-((4-(3,4-Difluorophenoxy)-3,5-difluorobenzyl)oxy)-6,7,7a,8-tetrahydro-1H-azeto[1',2':3,4]imidazo[1,2-c]pyrimidin-1-one | 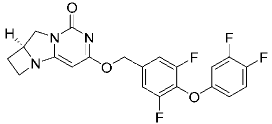 | 434.1 |
| 38-R | (R)-3-((6-(4-Chloro-3-(trifluoromethyl)phenoxy)-5-fluoropyridin-3-yl)methoxy)-6,7,7a,8-tetrahydro-1H-azeto[1',2':3,4]imidazo[1,2-c]pyrimidin-1-one | 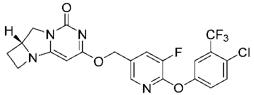 | 483.1 |
| 38-S | (S)-3-((6-(4-Chloro-3-(trifluoromethyl)phenoxy)-5-fluoropyridin-3-yl)methoxy)-6,7,7a,8-tetrahydro-1H-azeto[1',2':3,4]imidazo[1,2-c]pyrimidin-1-one | 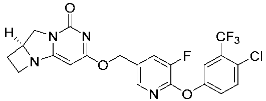 | 483.1 |
| 39-R | (R)-2-(4-Chloro-3-(trifluoromethyl)phenoxy)-5-(((1-oxo-6,7,7a,8-tetrahydro-1H-azeto[1',2':3,4]imidazo[1,2-c]pyrimidin-3-yl)oxy)methyl)benzonitrile | 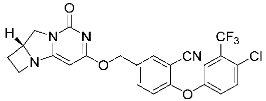 | 489.1 |
| 39-S | (S)-2-(4-Chloro-3-(trifluoromethyl)phenoxy)-5-(((1-oxo-6,7,7a,8-tetrahydro-1H-azeto[1',2':3,4]imidazo[1,2-c]pyrimidin-3-yl)oxy)methyl)benzonitrile | 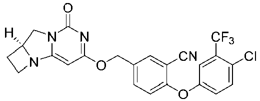 | 489.1 |
| 40-R | (R)-3-((3,5-Difluoro-4-(3-(trifluoromethyl)phenoxy)benzyl)oxy)-6,7,7a,8-tetrahydro-1H-azeto[1',2':3,4]imidazo[1,2-c]pyrimidin-1-one | 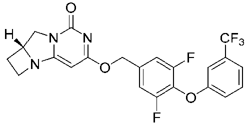 | 466.1 |
| 40-S | (S)-3-((3,5-Difluoro-4-(3-(trifluoromethyl)phenoxy)benzyl)oxy)-6,7,7a,8-tetrahydro-1H-azeto[1',2':3,4]imidazo[1,2-c]pyrimidin-1-one | 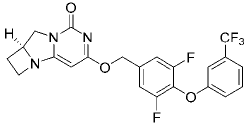 | 466.1 |
| 41-R | (R)-3-((6-(4-Fluoro-3-(trifluoromethyl)phenoxy)pyridin-3-yl)methoxy)-6,7,7a,8-tetrahydro-1H-azeto[1',2':3,4]imidazo[1,2-c]pyrimidin-1-one | 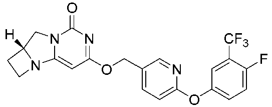 | 449.1 |
| 41-S | (S)-3-((6-(4-Fluoro-3-(trifluoromethyl)phenoxy)pyridin-3-yl)methoxy)-6,7,7a,8-tetrahydro-1H-azeto[1',2':3,4]imidazo[1,2-c]pyrimidin-1-one | 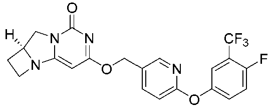 | 449.1 |
| 42-R | (R)-2-(3,5-Difluorophenoxy)-5-(((1-oxo-6,7,7a,8-tetrahydro-1H-azeto[1',2':3,4]imidazo[1,2-c]pyrimidin-3-yl)oxy)methyl)benzonitrile | 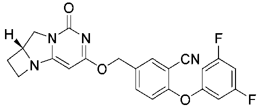 | 423.1 |
| 42-S | (S)-2-(3,5-Difluorophenoxy)-5-(((1-oxo-6,7,7a,8-tetrahydro-1H-azeto[1',2':3,4]imidazo[1,2-c]pyrimidin-3-yl)oxy)methyl)benzonitrile | 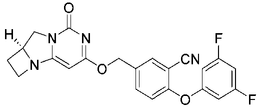 | 423.1 |
| 43-R | (R)-3-((3,5-Difluoro-4-((2-(trifluoromethyl)pyrimidin-5-yl)oxy)benzyl)oxy-6,7,7a,8-tetrahydro-1H-azeto[1',2':3,4]imidazo[1,2-c]pyrimidin-1-one | 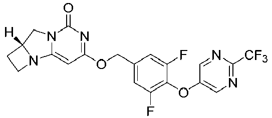 | 468.1 |
| 43-S | (S)-3-((3,5-Difluoro-4-((2-(trifluoromethyl)pyrimidin-5-yl)oxy)benzyl)oxy-6,7,7a,8-tetrahydro-1H-azeto[1',2':3,4]imidazo[1,2-c]pyrimidin-1-one | 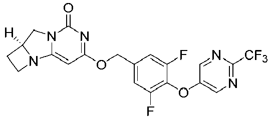 | 468.1 |
| 44-R | (R)-3-((3,5-Difluoro-4-((2-methylpyrimidin-5-yl)oxy)benzyl)oxy-6,7,7a,8-tetrahydro-1H-azeto[1',2':3,4]imidazo[1,2-c]pyrimidin-1-one | 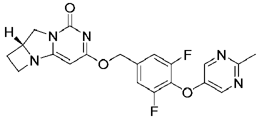 | 414.1 |
| 44-S | (S)-3-((3,5-Difluoro-4-((2-methylpyrimidin-5-yl)oxy)benzyl)oxy-6,7,7a,8-tetrahydro-1H-azeto[1',2':3,4]imidazo[1,2-c]pyrimidin-1-one | 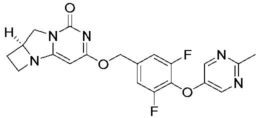 | 414.1 |
| 45-R | (R)-3-((3,5-Difluoro-4-((1-methyl-1H-pyrazol-4-yl)oxy)benzyl)oxy-6,7,7a,8-tetrahydro-1H-azeto[1',2':3,4]imidazo[1,2-c]pyrimidin-1-one | 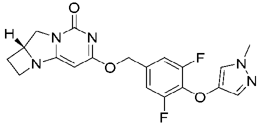 | 402.1 |
| 45-S | (S)-3-((3,5-Difluoro-4-((1-methyl-1H-pyrazol-4-yl)oxy)benzyl)oxy-6,7,7a,8-tetrahydro-1H-azeto[1',2':3,4]imidazo[1,2-c]pyrimidin-1-one | 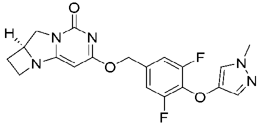 | 402.1 |
| 46-R | (R)-3-((4-((1-Ethyl-1H-pyrazol-4-yl)oxy)-3,5-difluorobenzyl)oxy-6,7,7a,8-tetrahydro-1H-azeto[1',2':3,4]imidazo[1,2-c]pyrimidin-1-one | 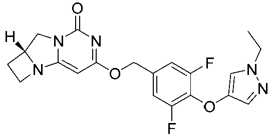 | 416.2 |
| 46-S | (S)-3-((4-((1-Ethyl-1H-pyrazol-4-yl)oxy)-3,5-difluorobenzyl)oxy-6,7,7a,8-tetrahydro-1H-azeto[1',2':3,4]imidazo[1,2-c]pyrimidin-1-one | 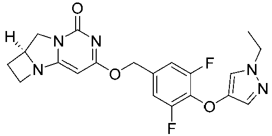 | 416.2 |
| 47-R | (R)-3-((4-((1-Isopropyl-1H-pyrazol-4-yl)oxy)-3,5-difluorobenzyl)oxy-6, 7, 7a,8-tetrahydro-1H-azeto[1',2':3,4]imidazo[1,2-c]pyrimidin-1-one | 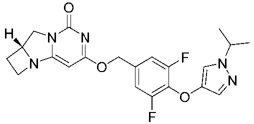 | 430.2 |
| 47-S | (S)-3-((4-((1-Isopropyl-1H-pyrazol-4-yl)oxy)-3,5-difluorobenzyl)oxy-6,7,7a,8-tetrahydro-1H-azeto[1',2':3,4]imidazo[1,2-c]pyrimidin-1-one | 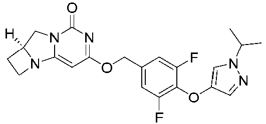 | 430.2 |
| 48-R | (R)-3-((4-((1-(Trifluoromethyl)-1H-pyrazol-4-yl)oxy)-3,5-difluorobenzyl)oxy-6,7,7a,8-tetrahydro-1H-azeto[1',2':3,4]imidazo[1,2-c]pyrimidin-1-one | 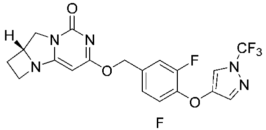 | 456.1 |
| 48-S | (S)-3-((4-((1-(Trifluoromethyl)-1H-pyrazol-4-yl)oxy)-3,5-difluorobenzyl)oxy-6,7,7a,8-tetrahydro-1H-azeto[1',2':3,4]imidazo[1,2-c]pyrimidin-1-one | 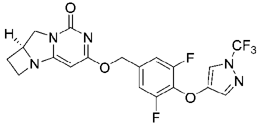 | 456.1 |
| 49-R | (R)-3-((3-Fluoro-4-((1-methyl-1H-pyrazol-4-yl)oxy)benzyl)oxy-6,7,7a,8-tetrahydro-1H-azeto[1',2':3,4]imidazo[1,2-c]pyrimidin-1-one | 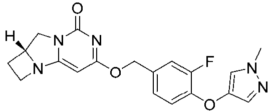 | 384.1 |
| 49-S | (S)-3-((3-Fluoro-4-((1-methyl-1H-pyrazol-4-yl)oxy)benzyl)oxy-6,7,7a,8-tetrahydro-1H-azeto[1',2':3,4]imidazo[1,2-c]pyrimidin-1-one | 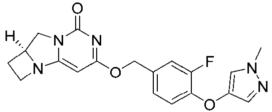 | 384.1 |
| 50-R | (R)-3-((4-(4-Chloro-3-(trifluoromethyl)phenoxy)benzyl)oxy)-6,7,7a,8-tetrahydro-1H-azeto[1',2':3,4]imidazo[1,2-c]pyrimidin-1-one | 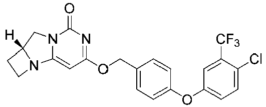 | 464.1 |
| 50-S | (S)-3-((4-(4-Chloro-3-(trifluoromethyl)phenoxy)benzyl)oxy)-6,7,7a,8-tetrahydro-1H-azeto[1',2':3,4]imidazo[1,2-c]pyrimidin-1-one | 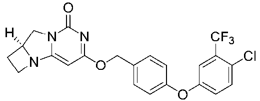 | 464.1 |
| 51-R | (R)-3-((3-Fluoro-4-((2-(trifluoromethyl)pyrimidin-5-yl)oxy)benzyl)oxy-6,7,7a,8-tetrahydro-1H-azeto[1',2':3,4]imidazo[1,2-c]pyrimidin-1-one | 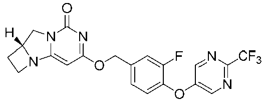 | 450.1 |
| 51-S | (S)-3-((3-Fluoro-4-((2-(trifluoromethyl)pyrimidin-5-yl)oxy)benzyl)oxy-6,7,7a,8-tetrahydro-1H-azeto[1',2':3,4]imidazo[1,2-c]pyrimidin-1-one | 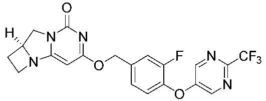 | 450.1 |
| 52-R | (R)-3-((3,5-Difluoro-4-((6-(trifluoromethyl)pyridin-3-yl)oxy)benzyl)oxy-6,7,7a,8-tetrahydro-1H-azeto[1',2':3,4]imidazo[1,2-c]pyrimidin-1-one | 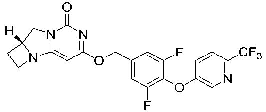 | 467.1 |
| 52-S | (S)-3-((3,5-Difluoro-4-((6-(trifluoromethyl)pyridin-3-yl)oxy)benzyl)oxy-6,7,7a,8-tetrahydro-1H-azeto[1',2':3,4]imidazo[1,2-c]pyrimidin-1-one | 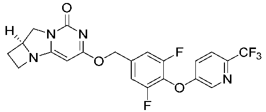 | 467.1 |
| 53-R | (R)-2-Chloro-5-(2-fluoro-4-(((1-oxo-6,7,7a,8-tetrahydro-1H-azeto[1',2':3,4]imidazo[1,2-c]pyrimidin-3-yl)oxy)methyl)phenoxy)benzonitrile | 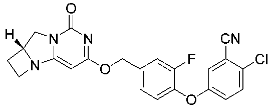 | 439.1 |
| 53-S | (S)-2-Chloro-5-(2-fluoro-4-(((1-oxo-6,7,7a,8-tetrahydro-1H-azeto[1',2':3,4]imidazo[1,2-c]pyrimidin-3-yl)oxy)methyl)phenoxy)benzonitrile | 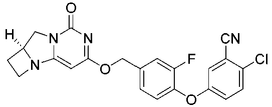 | 439.1 |
| 54-R | (R)-3-((3,4-Difluorobenzyl)oxy)-6,7,7a,8-tetrahydro-1H-azeto[1',2':3,4]imidazo[1,2-c]pyrimidin-1-one | 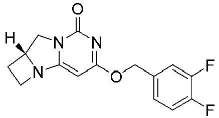 | 306.1 |
| 54-S | (S)-3-((3,4-Difluorobenzyl)oxy)-6,7,7a,8-tetrahydro-1H-azeto[1',2':3,4]imidazo[1,2-c]pyrimidin-1-one | 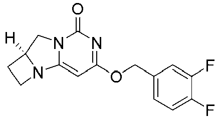 | 306.1 |

### Biological Assay Evaluations

The present disclosure is further described below using test examples, but these examples are not intended to limit the scope of the present disclosure. The compounds of the present disclosure are Lp-PLA2 inhibitors and are therefore useful in the treatment of diseases or conditions associated with Lp-PLA2 activity. The biological activity of the compounds of the present disclosure can be determined by using any suitable assays, as well as tissues and *in vivo* models, including the assays described herein, to determine the activity of the compounds as Lp-PLA2 inhibitors. Biological activity data for each tested example compound are reported either in at least one experiment or in multiple experiments. The data described herein may have reasonable variations depending on the particular conditions and procedures used by those who conducted the experiments.

The chemical structure of Comparative Example 1 below of the present disclosure is shown below, and was prepared by referring to the method in Example E67 of international patent application WO2016011931A1,

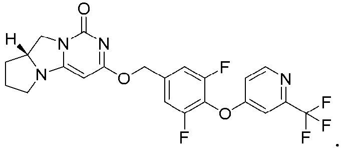

### Test Example 1. Determination of Inhibitory Activity of Compounds of the Present Disclosure Against Lp-PLA2 in Human Plasma

The literature reports that 2-Thio-PAF, commercially available from Cayman Chemical, is a substrate for PAF hydrolase (PAF-AH). After its cleavage by PAF-AH, the free thiol released at the sn-2 position can react with 7-diethylamino-3-(4'-maleimidophenyl)-4-methylcoumarin (CPM), resulting in increased fluorescence. Thus, Lp-PLA2 inhibitors can prevent this cleavage, and no increase in fluorescence is observed. In this experiment, the human plasma assay employed the same thioester analog of PAF as described in the recombinant human Lp-PLA2 assay described above.

### 1.1 Experimental reagents, consumables, and instruments:

| Reagent/consumable/instrument name | Brand | Cat. No. |
|---|---|---|
| HBSS | Gibco | 14175103 |
| HEPES | Gibco | 15630080 |
| CHAPS | sigma | 220201 |
| NaCl | General-ReaGent | 7647-14-5 |
| NaOH | General-ReaGent | 1310-73-2 |
| 2-thio-PAF | Cayman | 60945 |
| NEM | thermo | 23030 |
| CPM | Invitrogen | D346 |
| Trifluoroacetic acid (TFA) | adamas | 01162227 |
| DMSO | Sigma | D8418 |
| 96-well cell culture plate | Beyotime | FCP966-80pcs |
| 96-well plate | Beyotime | FPT019 |
| Envision | PerkinElmer | 2105 |
| Shaker | ThermoScientific | 88882006 |

### 1.2 Experimental method:

(1) The Thio-PAF assay was performed as a quenched 75 µL assay. A compound source plate was prepared by preparing 1:4 (v/v) serial diluents of the compounds in pure DMSO in a 96-well microplate.
(2) 1 µL of each test compound from the compound source plate was transferred into a 96-well microplate containing 40 µL of pre-thawed mixed human plasma by a Rainin multichannel pipette, and the plasma was thawed at room temperature and centrifuged at 2000 rpm for 10 min. The plate was shaken on a microplate shaker for 2 min for uniform mixing.
(3) After 30 min of pre-incubation at room temperature, 10 µL of a substrate solution was added sequentially to the 96-well microplate described above by a Rainin multichannel pipette. The substrate solution contained 2.5 mM 2-thio-PAF (from an ethanol mother liquor), 32 µM CPM (from a DMSO mother liquor), and 3.2 mM N-ethylmaleimide (freshly prepared in DMSO for each experiment) in assay buffer (pH 7.4) consisting of 50 mM HEPES, 150 mM NaCl, and 1 mM CHAPS. After the addition was completed, the plate was immediately shaken for 2 min, and the reaction time for the substrate was 4 min in total. After the reaction was completed, the reaction was quenched with 25 µL of an aqueous solution of 5% trifluoroacetic acid. The plate was centrifuged at 1000 rpm for 1 min.
(4) The FLINT signal (FLINT380/480) was read using a microplate reader.
(5) IC₅₀ data, curve fitting, and QC analyses were performed using GraphPad Prism 8.0 and Excel.

### 1.3. Experimental results:

The specific results of the IC₅₀ for the inhibitory activity of the compounds of the present disclosure against Lp-PLA2 in human plasma were determined through the above schemes, as shown in Table 1.

**Table 1. Results of the inhibitory activity of the compounds of the present disclosure against Lp-PLA2 in human plasma**

| Example No. | IC₅₀ (nM) |
|---|---|
| 1 | 3.9 |
| 7 | 68.7 |
| 8 | 2426 |
| 12 | 2086 |
| 16 | 2744 |
| 21 | 200 |
| 22 | 4.6 |
| 23 | 4.3 |
| 24 | 12.7 |
| 28 | 892.1 |
| 34 | 5.2 |
| 34-S | 52.3 |
| 34-R | 3.3 |
| 36 | 35.8 |
| 36-S | 96.4 |
| 36-R | 15.9 |
| 38 | 56.3 |
| 39 | 3.9 |
| 41 | 291.8 |
| 44 | 25.6 |
| 45 | 202.8 |
| 50 | 12.6 |
| 51 | 21.2 |
| 52 | 4.3 |
| 53 | 107 |
| 54 | 30.3 |
| Comparative Example 1 | 16.4 |

### 1.4. Experimental conclusion:

The results of the *in vitro* experiment indicate that the IC₅₀ values for the inhibitory activity of the compounds of the present disclosure against Lp-PLA2 in human plasma are all less than 300 nM; preferably, the IC₅₀ values for the inhibitory activity of Examples 1, 22, 23, 24, 34, 34-R, 36, 36-R, 39, 44, 50, 51, 52, 53, and 54 against Lp-PLA2 in human plasma are all less than 50 nM; more preferably, the IC₅₀ values for the inhibitory activity of Examples 1, 22, 23, 34, 34-R, 39, and 52 against Lp-PLA2 in human plasma are all less than 10 nM. This suggests that the compounds of the present disclosure exhibit good inhibitory activity against Lp-PLA2.

### Test Example 2. Liver Microsome Stability Experiment for Compounds of the Present Disclosure

### 2.1 Experimental objective

This experiment aimed to evaluate the phase I metabolic stability of the compounds of the present disclosure in liver microsomes of SD rats, beagle dogs, cynomolgus monkeys, and humans.

### 2.2 Experimental materials

### 2.2.1 Main experimental reagents

| Reagent | Supplier | Model |
|---|---|---|
| SD rat liver microsome | RILD | JYYJ |
| Beagle dog | RILD | DMXD |
| Cynomolgus monkey | RILD | LSRL |
| Human liver microsome | Corning | 38285 |
| β-NADP | Sigma | SLBZ0419 |
| G6P | Shanghai Yuanye | Y10A11C111059 |
| G6PDH | Shanghai Yuanye | L29A11Y112239 |
| MgCl₂ | Shanghai Titan | P1712091 |
| KH₂PO₄ | Shanghai Titan | P1722836 |
| K₂HPO₄ 3H₂O | Shanghai Titan | P1722886 |
| Testosterone | Shanghai Titan | G157843 |

### 2.2.2 Experimental instruments

| Name | Supplier | Model |
|---|---|---|
| Liquid chromatograph | SCIEX | Exion LC^{tm} |
| Mass spectrometer | SCIEX | Qtrap 5500+ |
| Centrifuge | Thermo Fisher | 75009915 |
| Shaker | Thermo Scientific | 88882006 |

### 2.3 Experimental procedures

(1) Buffer preparation: 73.21 g of dipotassium hydrogen phosphate trihydrate and 10.78 g of potassium dihydrogen phosphate were dissolved in 4000 mL of ultrapure water. The pH of the solution was adjusted to within 7.40 ± 0.10 using 10% phosphoric acid or 1 M potassium hydroxide, resulting in a final concentration of 100 mM.
(2) Solution preparation and dilution: Stock solutions of the test compounds at a concentration of 10 mM were prepared in dimethyl sulfoxide (DMSO) and stored at 4 °C, and they were diluted into 100 µM working solutions with pure acetonitrile when used. A stock solution of the control compound testosterone at a concentration of 10 mM was prepared in DMSO and stored at -20 °C, and it was diluted into a 400 µM working solution with pure acetonitrile when used.
(3) Termination solution preparation: The termination solution was an acetonitrile solution containing an internal standard (terfenadine). The prepared termination solution was stored in a refrigerator at 2-8 °C.
(4) Liver microsome solution preparation: Liver microsomes from various species (SD rat, beagle dog, cynomolgus monkey, and human) were diluted into 20× working solutions using 100 mM potassium phosphate buffer. The final concentration of the liver microsomes in the reaction system was 0.5 mg/mL.
(5) Reduced nicotinamide adenine dinucleotide phosphate (NADPH) regeneration system preparation: Appropriate amounts of nicotinamide adenine dinucleotide phosphate (NADP), glucose-6-phosphate (G6P), magnesium chloride (MgCl₂), and glucose-6-phosphate dehydrogenase (G6PDH) were weighed to prepare stock solutions at concentrations of 65.33 mM, 330 mM, 300 mM, and 250 Units/mL, respectively. The four stock solutions described above were each added to an appropriate amount of buffer, and the mixture was mixed well by gently inverting up and down. The final concentrations of NADP, G6P, MgCl₂, and G6PDH in the NADPH regeneration system were 2.65 mM, 10.2 mM, 6.12 mM, and 2.45 Units/mL, respectively.
(6) Incubation process: The incubation was completed in 96-well plates. Several incubation plates were prepared and named T0, T5, T10, T20, T40, T60, and NCF60, respectively. The first 6 plates corresponded to reaction time points of 0 min, 5 min, 10 min, 20 min, 40 min, and 60 min, respectively. The NCF60 plate was incubated for 60 min with potassium phosphate buffer replacing the NADPH regeneration system solution. Samples under all conditions were prepared in triplicate.

2 µL of the test compound or control compound and 100 µL of microsome working solution (containing 1 mg/mL liver microsome protein) were added to each of the T0, T5, T10, T20, T40, T60, and NCF60 plates, and only the microsome working solution was added to the Blank 60 plate. The plates described above were then pre-incubated in a 37 °C water bath for about 10 min.

After the pre-incubation was completed, 98 µL of the NADPH regeneration system working solution was added to each sample well except for the NCF60 plate and the T0 plate to initiate the reaction, and 98 µL of potassium phosphate buffer was added to each well of the NCF60 plate. Therefore, for the sample wells containing the test sample or control substance working solution, the final concentrations of TC and testosterone in the reaction were 1 µM and 4 µM, respectively, the concentration of liver microsomes was 0.5 mg/mL, and the final concentrations of DMSO and acetonitrile in the reaction system were 0.01% (v/v) and 0.99% (v/v), respectively.

After incubation for an appropriate time (e.g., 5 min, 10 min, 20 min, 40 min, and 60 min), 600 µL of a termination solution (an acetonitrile solution containing 100 ng/mL tolbutamide) was added to each test compound sample well and control compound sample well to terminate the reaction.

For the samples in the T0 plate, the termination solution was added before the addition of the NADPH regeneration system working solution. All sample plates were shaken uniformly and centrifuged at 4000 rpm for 20 min, and then 50 µL of supernatant was taken from each test compound sample well and control compound sample well and diluted with 150 µL of pure water. The mixture was shaken uniformly and then subjected to LC-MS/MS analysis. Data were processed using the software Analyst 7.1 (Sciex, Framingham, Massachusetts, USA).

2.4 Experimental results: The results of the *in vitro* liver microsome stability test for the compounds of the present disclosure are shown in Table 2.

**Table 2. Results of in vitro liver microsome stability test**

| Sample No. | Liver microsome species | T_{1/2} (min) | CLint(liver) (mL/min/Kg) | CL(liver) (mL/min/Kg) | Clearance rate classification |
|---|---|---|---|---|---|
| Example 34-R | Human | >186.38 | <6.69 | <5.06 | Low |
| | Cynomolgus monkey | >186.38 | <10.0 | <8.2 | Low |
| | Beagle dog | >186.38 | <10.7 | <8.0 | Low |
| | SD rat | > 186.4 | <13.4 | <10.8 | Low |
| Comparative Example 1 | Human | 120.70 | 10.3 | 6.9 | Medium |
| | Cynomolgus monkey | 23.10 | 81.0 | 28.3 | Medium |
| | Beagle dog | 126.01 | 15.8 | 10.5 | Medium |
| | SD rat | 86.96 | 28.7 | 18.9 | Medium |
| Rilapladib | Human | 20.71 | 60.3 | 15.4 | High |
| | Cynomolgus monkey | 4.06 | 461.2 | 39.8 | High |
| | Beagle dog | 161.50 | 12.4 | 8.8 | Low |
| | SD rat | > 186.4 | <13.4 | <10.8 | Low |

| | | | | | |
|---|---|---|---|---|---|
| Note: The clearance rate classification criteria are shown in the following table: | | | | | |

| Liver | Clearance rate classification criteria CLᵢₙₜ₍ₗᵢᵥₑᵣ₎ (mL/min/kg) | | |
|---|---|---|---|
| microsome species | Low | Medium | High |
| Human | ≤8.9 | 8.9<CLᵢₙₜ₍ₗᵢᵥₑᵣ₎≤48.3 | >48.3 |
| Cynomolgus monkey | ≤18.7 | 18.7<CLᵢₙₜ₍ₗᵢᵥₑᵣ₎≤101.7 | >101.7 |
| Beagle dog | ≤13.2 | 13.2<CLᵢₙₜ₍ₗᵢᵥₑᵣ₎≤72.1 | >72.1 |
| SD rat | ≤23.7 | 23.7<CLᵢₙₜ₍ₗᵢᵥₑᵣ₎≤128.8 | >128.8 |

### 2.5 Experimental conclusion

It can be seen from the results in Table 2 described above that the compounds of the present disclosure have lower clearance rates and longer half-lives in liver microsomes of SD rats, beagle dogs, cynomolgus monkeys, and humans. Based on the pharmacodynamic activity and pharmacological mechanism of the series of compounds, a slow metabolic rate is beneficial for the compounds to exert the pharmacodynamic activity, so the compounds of the present disclosure exhibit a more excellent effect in the aspect of metabolic stability.

## Claims

1. A compound represented by general formula (IA), a stereoisomer thereof, a pharmaceutically acceptable salt thereof, or a prodrug thereof: 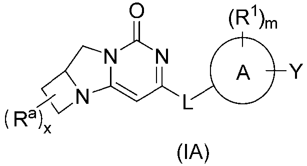 wherein:
L is -O(CH₂)ₙ₁-, -S(CH₂)ₙ₁-, -(CH₂)ₙ₁-, or -NRₐₐ(CH₂)ₙ₁-; the (CH₂)ₙ₁ end is linked to ring A;
Rₐₐ is hydrogen, deuterium, C₁₋₆ alkyl, C₁₋₆ deuterated alkyl, or C₃₋₆ cycloalkyl;
n1 is an integer of 1-4;
Y is H or 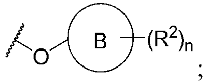
ring A and ring B are each independently C₆₋₁₄ aryl or 5- to 14-membered heteroaryl containing 1-4 heteroatoms selected from N, O, and S; when Y is not H, ring A is a group that is at least divalent;
R¹ and R² are each independently hydrogen, deuterium, halogen, hydroxyl, cyano, amino, oxo, C₁₋₆ alkyl, C₁₋₆ deuterated alkyl, C₁₋₆ haloalkyl, C₁₋₆ alkoxy, C₁₋₆ alkylthio, C₁₋₆ alkylamino, C₂₋₆ alkenyl, C₂₋₆ alkynyl, -S(O)-C₁₋₆ alkyl, -S(O)₂-C₁₋₆ alkyl, C₃₋₆ cycloalkyl, or -S(O)₂-C₃₋₆ cycloalkyl;
m and n are each independently an integer of 0-6;
R^{a} is hydrogen, deuterium, halogen, hydroxyl, cyano, amino, oxo, C₁₋₆ alkyl, C₁₋₆ deuterated alkyl, C₁₋₆ haloalkyl, C₁₋₆ alkoxy, C₁₋₆ alkylthio, C₁₋₆ alkylamino, or C₃₋₆ cycloalkyl;
x is an integer of 0-5.

2. The compound represented by general formula (IA), the stereoisomer thereof, the pharmaceutically acceptable salt thereof, or the prodrug thereof according to claim 1, wherein general formula (IA) is further represented by general formula (I): 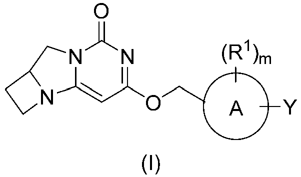 wherein:
Y is H or 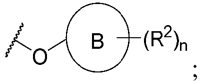
ring A and ring B are each independently C₆₋₁₄ aryl or 5- to 14-membered heteroaryl containing 1-4 heteroatoms selected from N, O, and S;
R¹ and R² are each independently hydrogen, deuterium, halogen, hydroxyl, cyano, amino, oxo, C₁₋₆ alkyl, C₁₋₆ deuterated alkyl, C₁₋₆ haloalkyl, C₁₋₆ alkoxy, C₁₋₆ alkylthio, or C₁₋₆ alkylamino;
m and n are each independently 0, 1, 2, 3, or 4.

3. The compound represented by general formula (IA), the stereoisomer thereof, the pharmaceutically acceptable salt thereof, or the prodrug thereof according to claim 1 or 2, wherein one or more of the following conditions are met:
(1) ring A and ring B are each independently C₆₋₁₀ aryl or 5- to 6-membered heteroaryl containing 1-3 heteroatoms selected from N, O, and S;
(2) R¹ and R² are each independently hydrogen, deuterium, halogen, cyano, C₁₋₆ alkyl, C₁₋₆ deuterated alkyl, or C₁₋₆ haloalkyl;
(3) m and n are each independently 0, 1, 2, or 3;
(4) a compound represented by general formula (I) is: 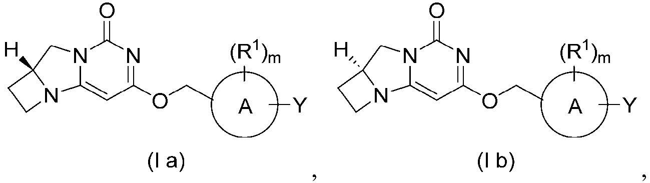 or a mixture thereof, wherein each group is as defined in claim 1 or 2.

4. The compound represented by general formula (IA), the stereoisomer thereof, the pharmaceutically acceptable salt thereof, or the prodrug thereof according to any one of claims 1-3, wherein one or more of the following conditions are met:
(1) ring A and ring B are each independently phenyl, naphthyl, pyridinyl, pyrimidinyl, or pyrazolyl;
(2) R₁ and R₂ are each independently hydrogen, deuterium, halogen, cyano, C₁₋₃ alkyl, C₁₋₃ deuterated alkyl, or C₁₋₃ haloalkyl.

5. The compound represented by general formula (IA), the stereoisomer thereof, the pharmaceutically acceptable salt thereof, or the prodrug thereof according to any one of claims 1-4, wherein one or more of the following conditions are met:
(1) ring A and ring B are each independently 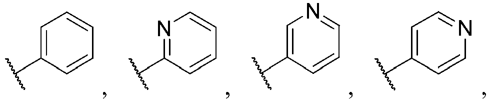 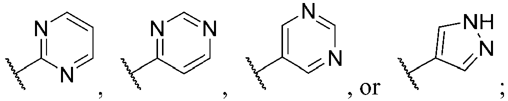
(2) R₁ and R₂ are each independently hydrogen, deuterium, fluorine, chlorine, bromine, cyano, methyl, ethyl, n-propyl, isopropyl, or trifluoromethyl.

6. The compound represented by general formula (IA), the stereoisomer thereof, the pharmaceutically acceptable salt thereof, or the prodrug thereof according to any one of claims 1-5, wherein one or more of the following conditions are met:
(1) 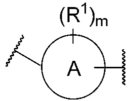 is 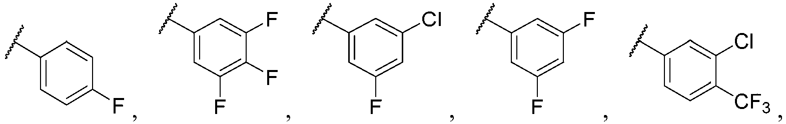 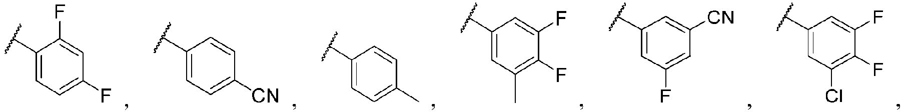 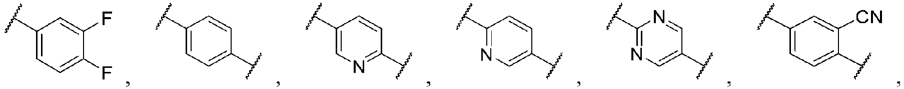 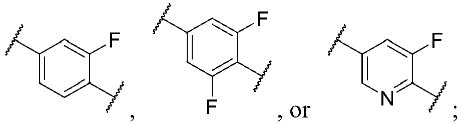
(2) 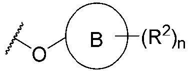 is

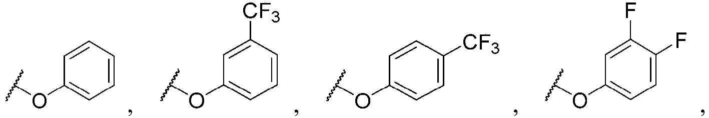

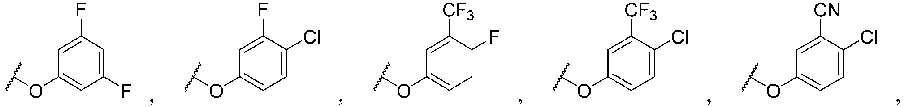

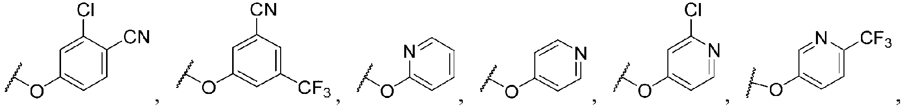

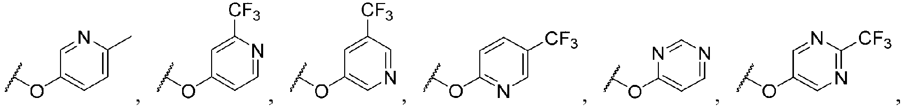

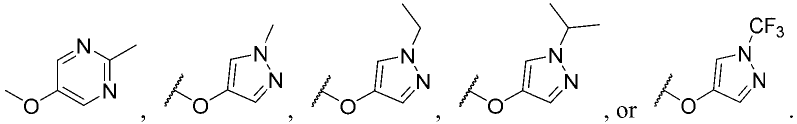

7. The compound represented by general formula (IA), the stereoisomer thereof, the pharmaceutically acceptable salt thereof, or the prodrug thereof according to any one of claims 1-6, wherein

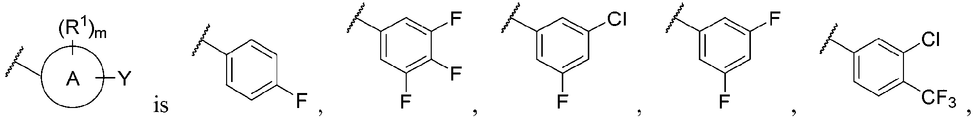

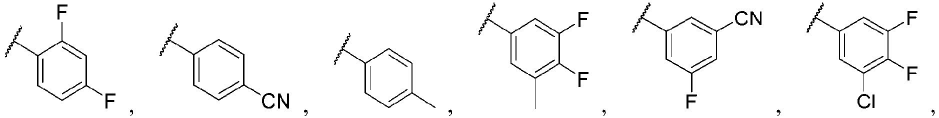

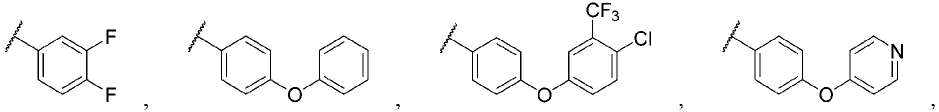

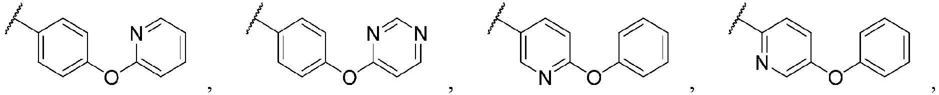

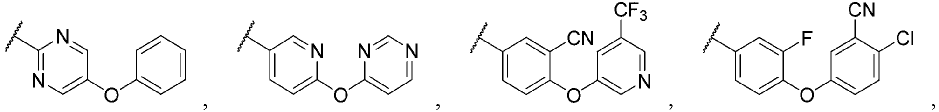

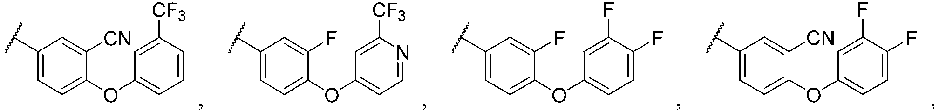

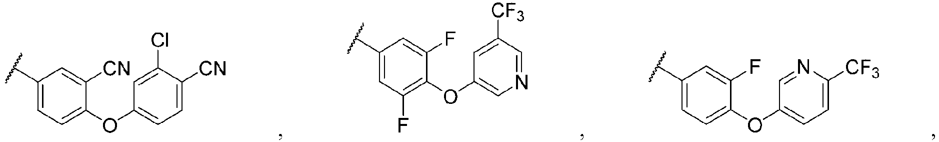

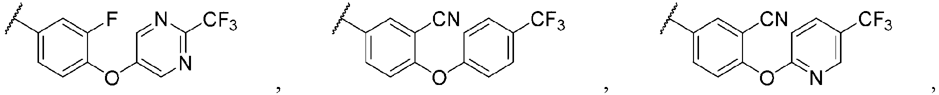

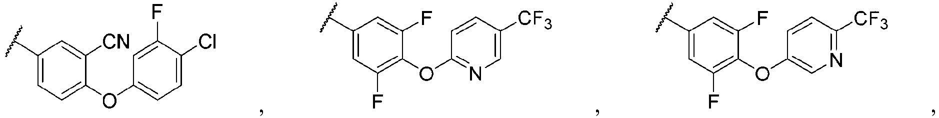

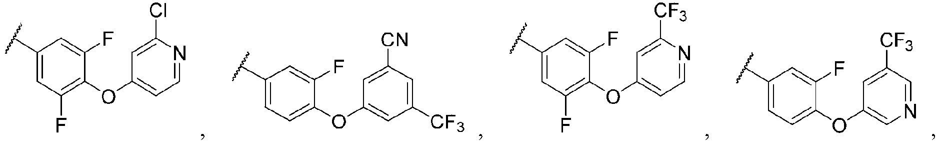

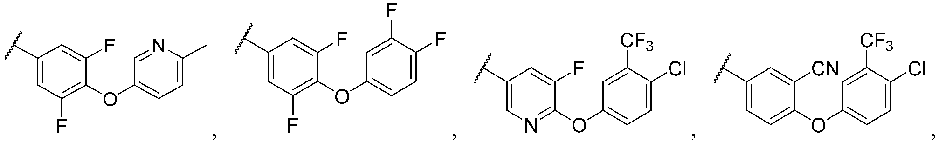

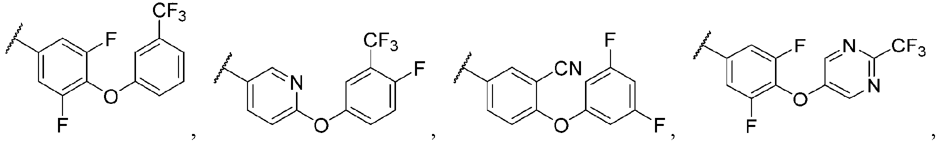

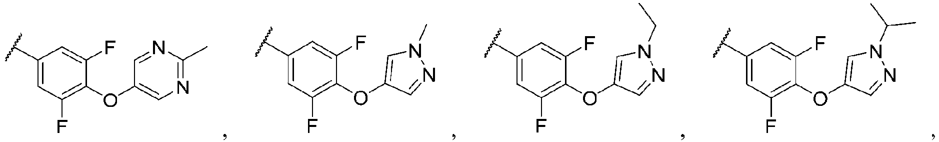

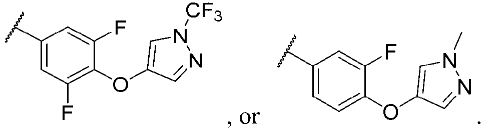

8. The compound represented by general formula (IA), the stereoisomer thereof, the pharmaceutically acceptable salt thereof, or the prodrug thereof according to claim 1, wherein general formula (IA) is further represented by general formula (II): 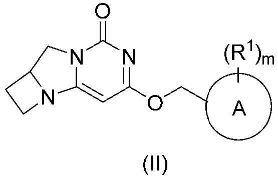 wherein ring A, R¹, and m are as defined in claim 1.

9. The compound represented by general formula (IA), the stereoisomer thereof, the pharmaceutically acceptable salt thereof, or the prodrug thereof according to claim 1, wherein general formula (IA) is further represented by general formula (III): 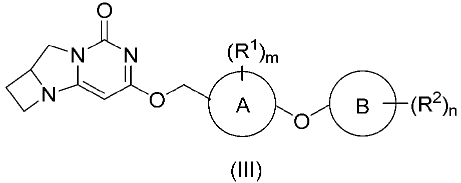 wherein ring A, ring B, R¹, R², m, and n are as defined in claim 1.

10. The compound represented by general formula (IA), the stereoisomer thereof, the pharmaceutically acceptable salt thereof, or the prodrug thereof according to any one of claims 1-9, wherein the compound represented by general formula (IA), the pharmaceutically acceptable salt thereof, or the prodrug thereof is selected from the following compounds:

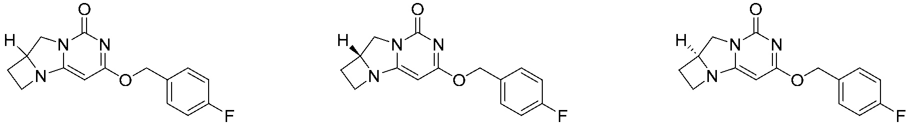

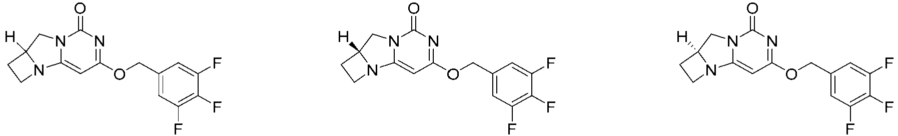

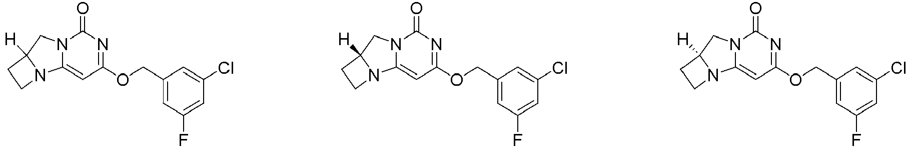

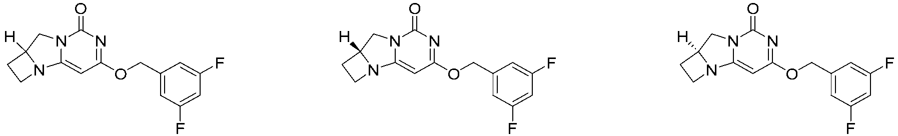

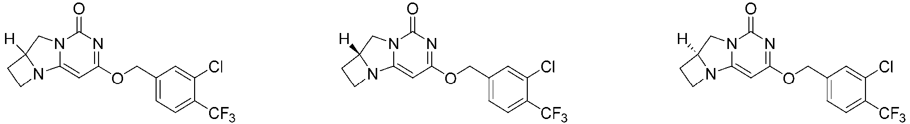

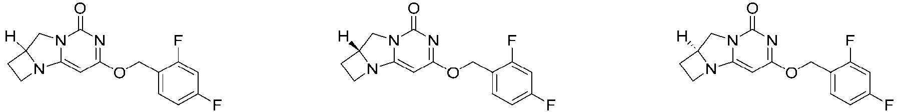

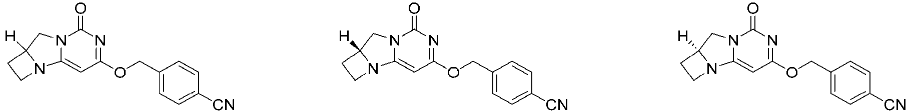

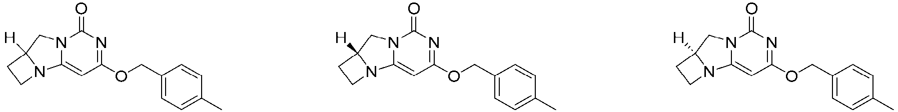

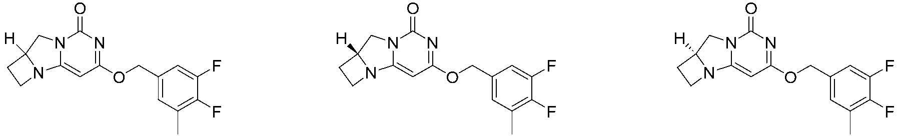

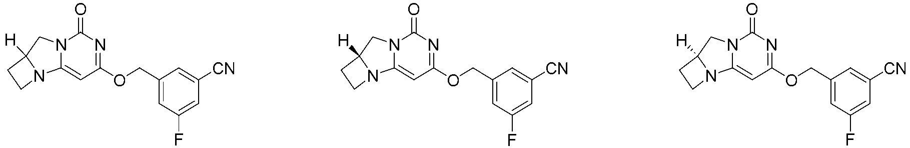

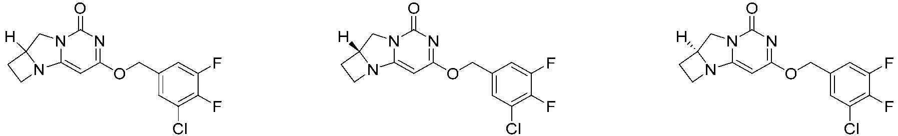

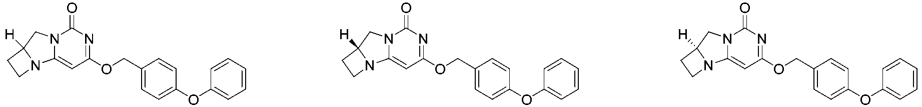

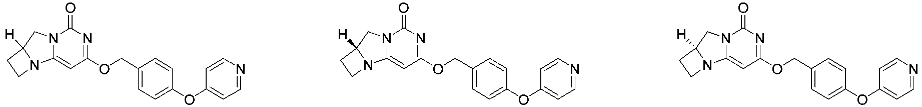

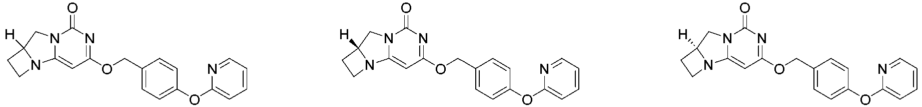

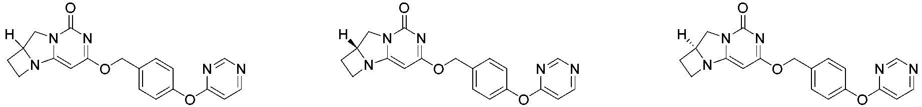

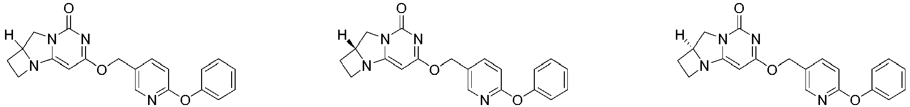

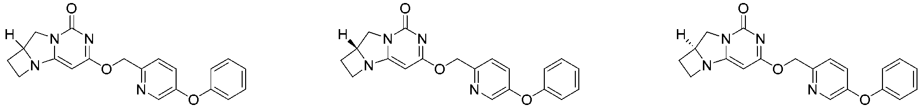

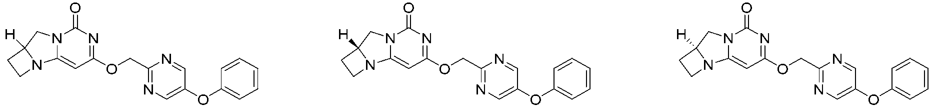

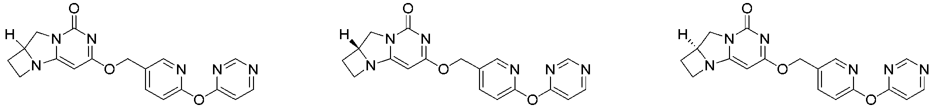

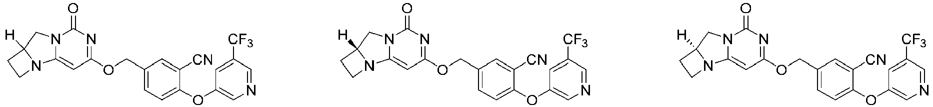

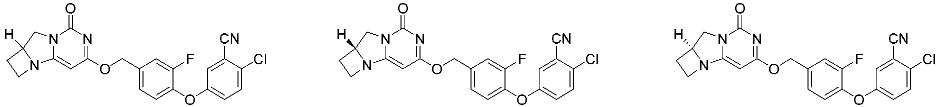

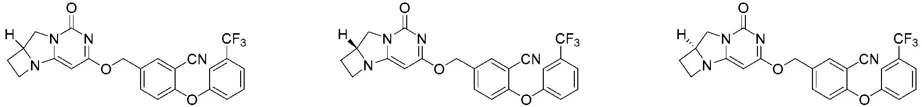

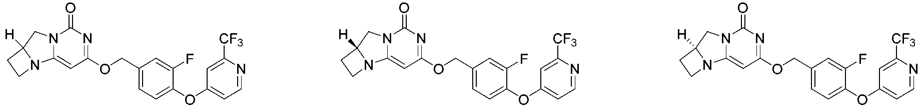

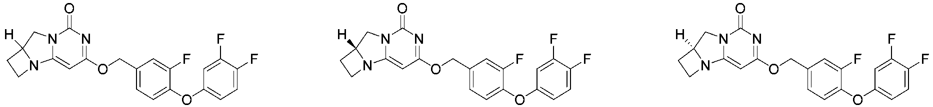

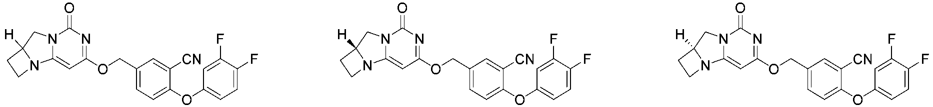

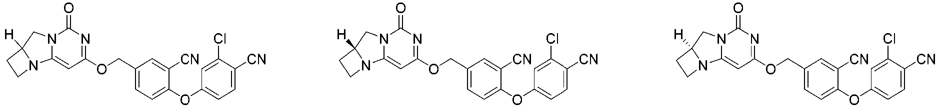

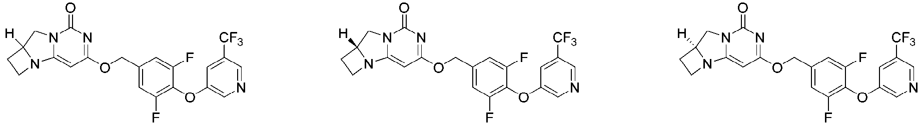

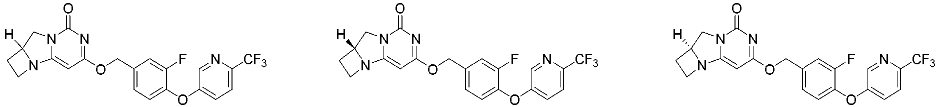

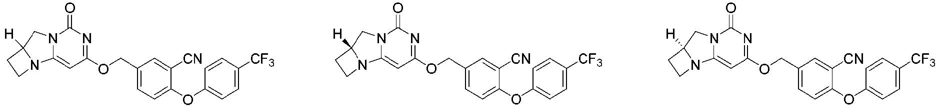

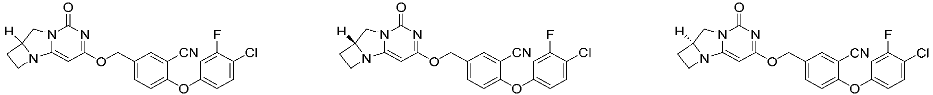

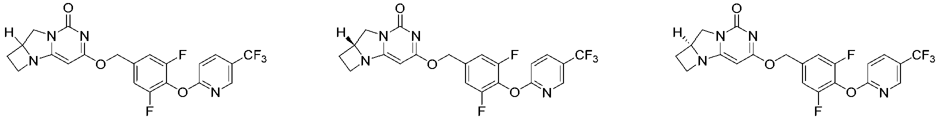

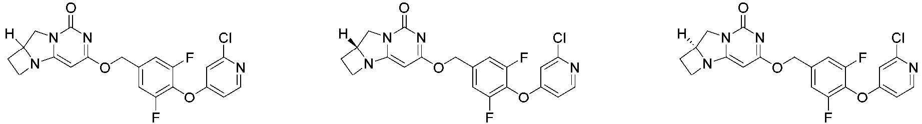

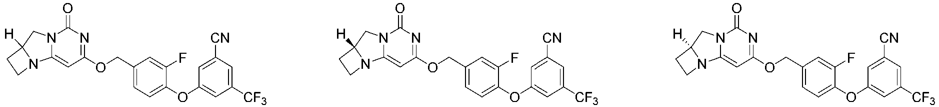

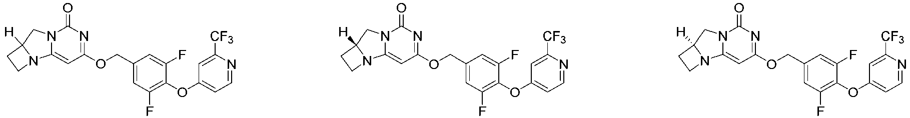

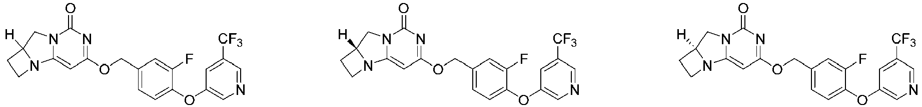

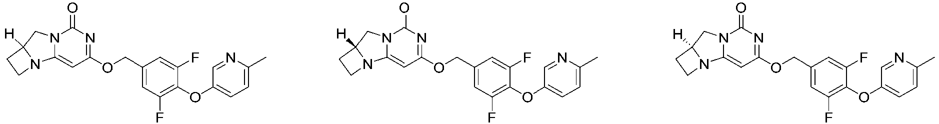

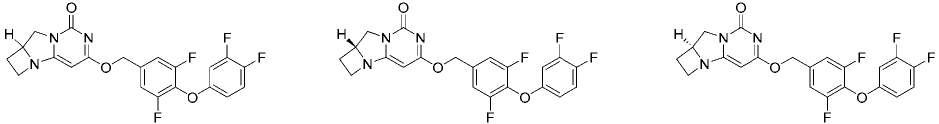

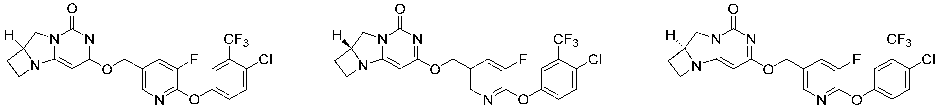

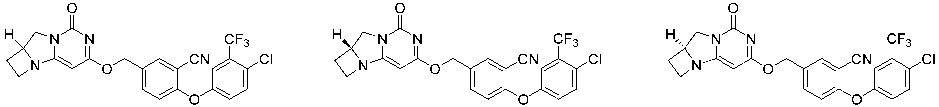

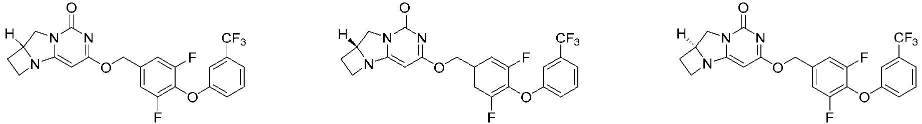

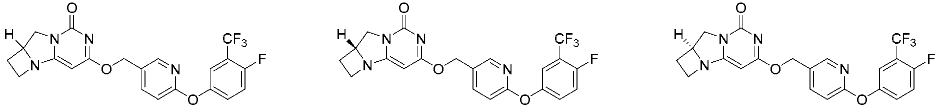

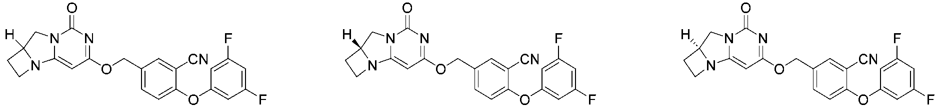

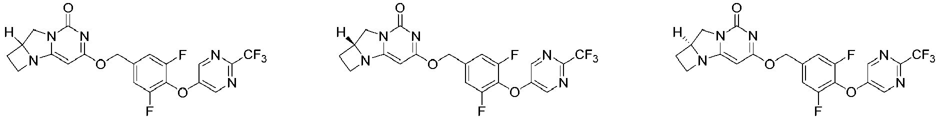

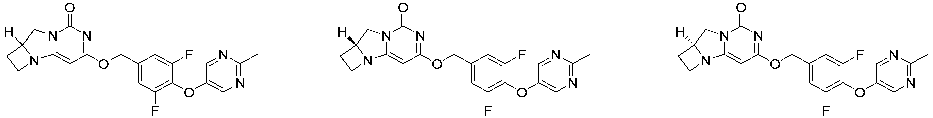

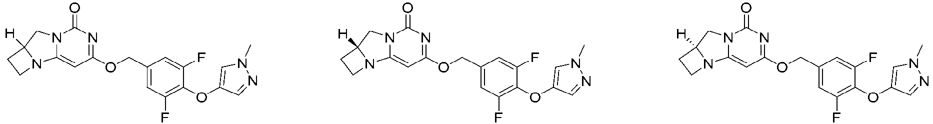

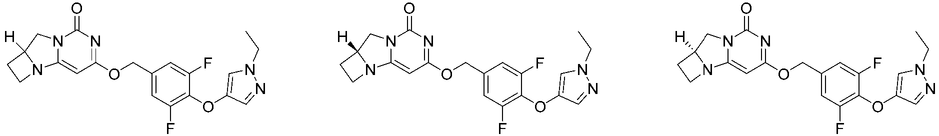

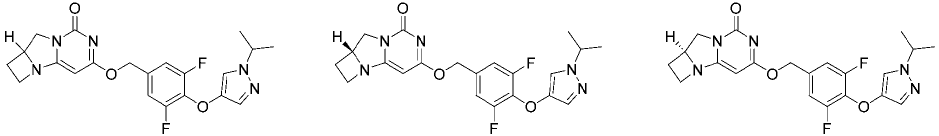

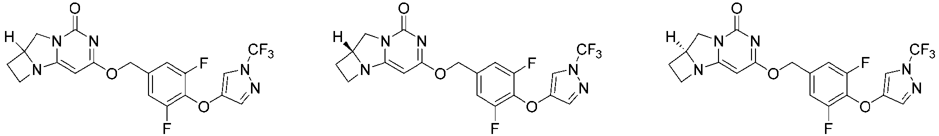

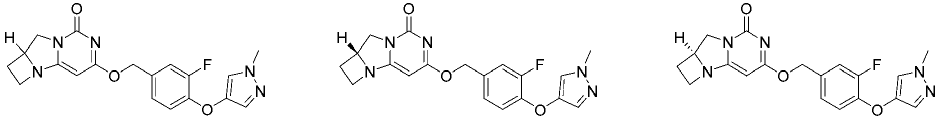

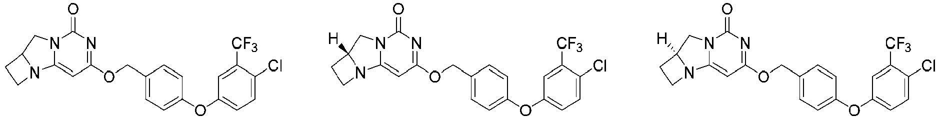

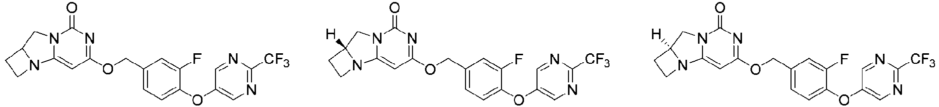

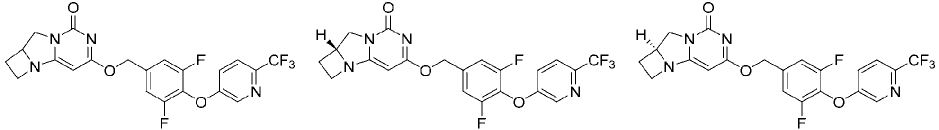

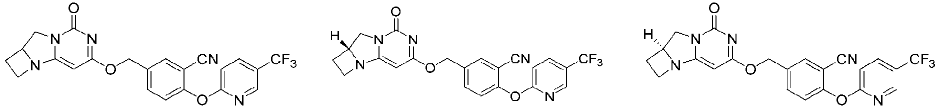

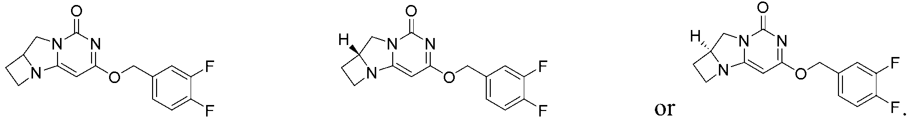

11. A compound represented by general formula (IA-1) or (IA-2), a stereoisomer thereof, or a salt thereof: 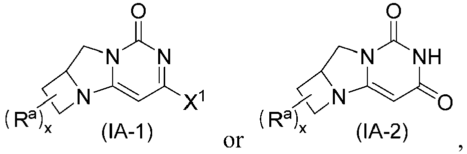
wherein X¹ is halogen, and R^{a} and x are as described in claim 1;
preferably, general formula (IA-1) or (IA-2) further has a structure represented by general formula (I-1) or (I-2): 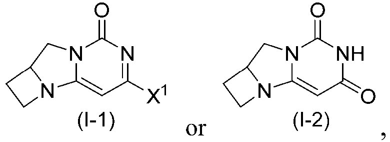
wherein X¹ is halogen.

12. A method for preparing a compound represented by general formula (IA), a stereoisomer thereof, or a pharmaceutically acceptable salt thereof, comprising the following step:
in a solvent, reacting a compound represented by general formula (IA-1) with a compound represented by general formula (IA-3) to give a compound represented by general formula (IA); 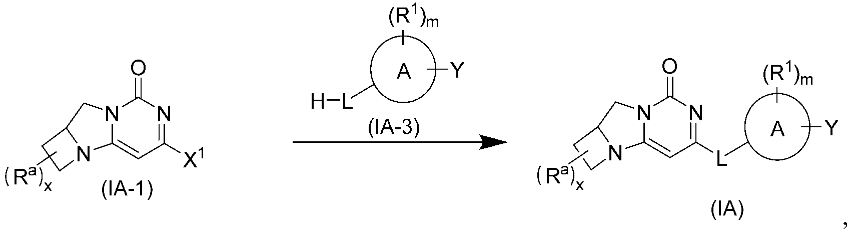
wherein:
X¹ is halogen;
L, R^{a}, x, ring A, R¹, Y, and m are as described in any one of claims 1-10.

13. A method for preparing a compound represented by general formula (I), a stereoisomer thereof, or a pharmaceutically acceptable salt thereof, comprising the following step:
in a solvent, reacting a compound represented by general formula (I-1) with a compound represented by general formula (I-3) to give a compound represented by general formula (I); 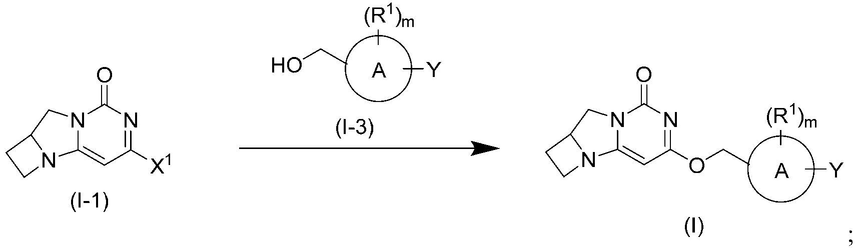
or in a solvent, reacting a compound represented by general formula (I-2) with a compound represented by general formula (I-4) to give a compound represented by general formula (I); 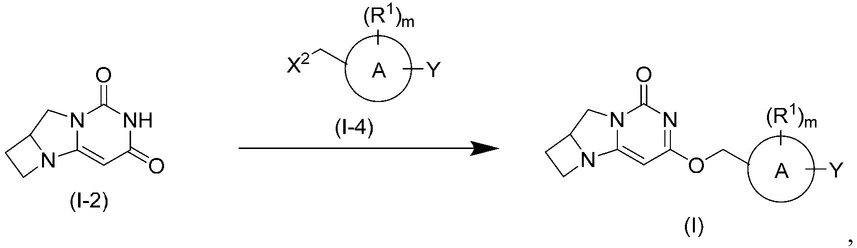
wherein:
X¹ and X² are each independently halogen;
ring A, R¹, Y, and m are as described in any one of claims 1-10.

14. A pharmaceutical composition, comprising a therapeutically effective dose of the compound, the stereoisomer thereof, the pharmaceutically acceptable salt thereof, or the prodrug thereof according to any one of claims 1-10, and one or more pharmaceutically acceptable carriers.

15. Use of the compound, the stereoisomer thereof, the pharmaceutically acceptable salt thereof, or the prodrug thereof according to any one of claims 1-10, or the pharmaceutical composition according to claim 14 in the preparation of an Lp-PLA2 inhibitor medicament.

16. Use of the compound, the stereoisomer thereof, the pharmaceutically acceptable salt thereof, or the prodrug thereof according to any one of claims 1-10, or the pharmaceutical composition according to claim 14 in the preparation of a medicament for preventing and/or treating a related disease mediated by Lp-PLA2.

17. The use according to claim 16, wherein the disease is one or more of a neurodegenerative disease, a cardiovascular and cerebrovascular disease, and a diabetic complication; the neurodegenerative disease is dementia, amyotrophic lateral sclerosis, or Parkinson's disease; the cardiovascular and cerebrovascular disease is coronary heart disease, cerebral stroke, or atherosclerosis; the diabetic complication is diabetic retinopathy, diabetic macular edema, diabetic nephropathy, diabetic neuropathy, diabetic peripheral neuropathic pain, or diabetic foot; the dementia is Alzheimer's disease, vascular dementia, or multi-infarct dementia.
